# EUROPEAN PATENT SPECIFICATION

(11) **EP 2 797 641 B1**
(45) Date of publication and mention of the grant of the patent: **10.07.2019**
(21) Application number: 12862472.3
(22) Date of filing: 31.12.2012
(51) Int. Cl.: A61L 27/22, A61L 27/26, A61L 27/40, A61L 27/56, A61L 27/50

(54) **FUNCTIONALIZATION OF BIOMATERIALS TO CONTROL REGENERATION AND INFLAMMATION RESPONSES**
FUNKTIONALISIERUNG VON BIOMATERIALIEN ZUR STEUERUNG VON REGENERATIONS- UND ENTZÜNDUNGSREAKTIONEN
FONCTIONNALISATION DE BIOMATÉRIAUX POUR COMMANDER LA RÉGÉNÉRATION ET DES RÉPONSES À UNE INFLAMMATION

(30) Priority: 29.12.2011 US 201161581364 P
(43) Date of publication of application: 05.11.2014
(73) Proprietor: Trustees Of Tufts College, Medford, MA 02155 (US)
(72) Inventor: LLAMAS, Jabier Gallego, Somerville Massachusetts 02143 (US); KAPLAN, David L., Concord Massachusetts 01742 (US); PANILAITIS, Bruce, Tewksbury Massachusetts 01876 (US)
(74) Representative: EIP
(86) International application number: PCT/US2012/072275
(87) International publication number: WO 2013/102193

(56) References cited:
- WO-A1-96/13275
- WO-A2-2004/062697
- WO-A2-2004/062697
- US-A1- 2007 149 477
- US-A1- 2008 254 045
- US-A1- 2011 218 143
- MATTHIAS BARTNECK ET AL: "Induction of specific macrophage subtypes by defined micro-patterned structures", ACTA BIOMATERIALIA, vol. 6, no. 10, 1 October 2010 (2010-10-01), pages 3864-3872, XP055196624, ISSN: 1742-7061, DOI: 10.1016/j.actbio.2010.04.025
- Ryan M Boehler ET AL: "Tissue engineering tools for modulation of the immune response", BioTechniques, 1 October 2011 (2011-10-01), page 239, XP055363413, England DOI: 10.2144/000113754 Retrieved from the Internet: URL:https://www.ncbi.nlm.nih.gov/pmc/artic les/PMC3526814/pdf/nihms-426889.pdf

## Description

### TECHNICAL FIELD OF THE DISCLOSURE

The inventions provided herein generally relate to compositions and methods for controlling response of immune cells to at least one stimulus or condition (*e.g.,* but not limited to, tissue damage, an implantable device and/or a cytokine) *in vitro* or *in vivo.* In some embodiments, the compositions and methods described herein can be used to control and/or skew response of macrophages to at least one stimulus or condition (*e.g.,* but not limited to, tissue damage, an implantable device and/or a cytokine) *in vitro* or *in vivo.*

### BACKGROUND

Optimization of a host response to a biomaterial, *e.g.,* used as a tissue engineering scaffold, is important for promoting tissue regeneration and/or wound healing. Many factors, such as the biodegradability, surface characteristics, size, and/or chemical composition of the biomaterial, can affect the level of the inflammatory response induced.

For example, the size and/or shape of a biomaterial can contribute to the inflammatory reaction. By way of example only, silk fibroin particles of different sizes can induce different degrees of inflammatory response when they are seeded on macrophages (20). Previous reports on effect of poly(lactic-co-glycolic) acid (PLGA) particles used as adjuvant systems for immunization showed that microparticles (∼5 µm - ∼7 µm) cannot be phagocytosed by murine macrophages with the same avidity demonstrated for nanoparticles (∼389 nm), but instead attach to cell membrane and constitute more potent inflammatory stimulus (39). Additionally, PLGA (poly(lactic-co-glycolic acid)) nanoparticles (∼265 nm) have been shown to be phagocytosed in rat synovium by macrophages and then delivered to the deep underlying tissues almost without localized inflammatory responses, but PLGA microspheres (∼26.5 µm) were not phagocytosed (40). Another previous report analyzed the effect of titanium dioxide nanoparticle size on gene expression of proinflammatory cytokines, and reported that sub-micro large titania particles (∼596 nm) showed a larger effect on cell viability and gene expression (IL-6, IL-8, TNF-α) when compared with the small particles (166 nm) *in vitro* on THP1 (Human monocytic leukemia cell line) macrophages. (33).

Biomaterial scaffolds can be used to promote the constructive remodeling of injured tissues through mechanisms that include, *e.g.,* angiogenesis, the recruitment of multipotential progenitor cells to the site of tissue reconstruction, the release of antimicrobial peptides, and/or activation of the alternative pathway of immunity. However, host-mediated degradation of the biomaterial scaffolds can generally occur within 8-12 weeks and thus the full beneficial effects of the scaffolds cannot be realized. While a biomaterial can be chemically crosslinked to increase resistance to host-mediated degradation, it is desirable to degrade the scaffold material upon tissue integration. Accordingly, there is a need for an improved biomaterial that can selectively control immune response, *e.g.,* to optimize tissue repair and/or regeneration.

WO 2004/062697 discloses porous silk fibroin scaffold material and its use for tissue engineering.

### SUMMARY

Described herein are compositions and methods directed to the functionalization of biomaterials with at least one active agent (*e.g.,* at least one immune cell-modulating agent and/or macrophage-skewing agent) that can modulate or skew the response of at least one type of immune cells (*e.g.,* but not limited to macrophages and/or dendritic cells) to a target stimulus (*e.g.,* but not limited to tissue damage, an implant, and/or a cytokine) and/or to the biomaterial itself. In some embodiments, the compositions comprising biomaterials functionalized with at least one immune cell-modulating agent (including, *e.g.,* macrophage-skewing agents) can promote or reduce inflammation mediated by immune cells such as macrophages, thereby modulating the immune response (*e.g.,* inducing inflammation or regeneration) to a target stimulus (*e.g.,* but not limited to tissue damage, an implant, and/or a cytokine) and/or to the biomaterial itself.

Embodiments of various aspects described herein are in part based on the inventors' recognition that properties of a biomaterial *(e.g.,* shape and/or size of a biomaterial) can be related to the cellular uptake of immune cells involved in the acute and chronic immune responses, such as macrophages (Mcps) and/or dendritic cells (DCs). For example, the cellular uptake pathway of the biomaterial particles (*e.g.,* silk fibroin particles) can depend on particle size and/or surface properties. These physical characteristics can affect the way proteins (*e.g.,* but not limited to, integrin, and clathrin) present on the plasma membrane of Mϕs and DCs bind to these particles, and this, in turn, can influence the capacity of these cells to eliminate them and the degree of the inflammatory response induced.

Accordingly, the inventors have discovered that biomaterials can be modified, for example, chemically and/or via inclusion of appropriate active agents, particularly immune cell-modulating agents and/or macrophage-skewing agents, during processing or post processing, to regulate immune cells (*e.g.,* macrophages) and/or other cellular responses *in vitro* or *in vivo.* For example, introduction of a selective compound that can promote inflammation or reduce inflammation, to a cell population can influence degradation of the biomaterial, *e.g.,* degrading the biomaterial faster, slower, or in a more selective way, *e.g.,* to control inflammatory responses and/or regeneration responses for biomaterials and/or tissues. The control of the immune responses can be mediated by the nature of the cell populations (*e.g.,* macrophages) activated or promoted in the presence of the selective immune cell-modulating agents and/or macrophage-skewing agents, *e.g.,* but not limited to, small molecules, peptides, antimicrobial peptides, and/or lipopolysaccharides. Accordingly, embodiments of various aspects described herein generally relate to compositions and methods for controlling immune response, *e.g.,* macrophage response, *in vitro* or *in vivo.*

In one aspect, provided herein is a composition comprising a functionalized silk fibroin biomaterial as defined in the claims appended hereto. The at least one immune cell-modulating agent is present in the biomaterial in an effective amount sufficient to selectively control or alter activation state of at least one type of immune cells upon contact with the at least one immune cell-modulating agent. Examples of immune cell types can include, but are not limited to, monocytes, macrophages, dendritic cells, megakaryocytes, granulocytes, T cells, B cells, natural killer (NK) cells, and any combinations thereof.

In some embodiments, the immune cell-modulating agent can be selected to control or alter activation state of at least dendritic cells. In some embodiments, the immune cell-modulating agent, *e.g.,* macrophage-skewing agent, can be selected to control or alter activation state of at least macrophages.

In one embodiment, the biomaterial can comprise a silk fibroin-based matrix.

Accordingly, described herein is a composition comprising a silk fibroin-based matrix comprising or functionalized with at least one immune cell-modulating agent (*e.g.,* macrophage-skewing agents) is also provided herein. The at least one immune cell-modulating agent can be present in the silk fibroin-based matrix in an effective amount sufficient to selectively control or alter activation state of immune cells upon contact with said at least one immune cell-modulating agent.

As described herein, the biomaterial or the silk fibroin-based matrix can be adapted for a sustained release of said at least one immune cell-modulating agent (*e.g.,* at least one macrophage-skewing agent) for a period of time, *e.g.,* at least about 1 week, at least about 2 weeks, at least about 3 weeks, at least about 4 weeks, at least about 5 weeks, at least about 6 weeks, at least about 7 weeks, at least about 8 weeks, at least about 12 weeks, or longer.

As described herein, the biomaterial or the silk fibroin-based matrix can comprise at least two (including, *e.g.,* at least three, at least four or more) immune cell-modulating agents (*e.g.,* at least two macrophage-skewing agents). In these embodiments, the biomaterial or the silk fibroin-based matrix can be adapted to release a first immune cell-modulating agent (*e.g.,* a first macrophage-skewing agent) at a first time point and a second immune cell-modulating agent (*e.g.,* a second macrophage-skewing agent) at a second time point. In some embodiments, the first time point and the second time point are the same. In other embodiments, the first time point and the second time point are different.

Distribution of immune cell-modulating agents (*e.g.,* macrophage-skewing agents) in the biomaterial or silk fibroin-based matrix can vary, depending on a number of factors, *e.g.,* but not limited to, number and/or types of immune cell-modulating agents (*e.g.,* macrophage-skewing agents), form of the matrix (*e.g.,* a film vs. particles), desired release profiles of the immune cell-modulating agent(s) (*e.g.,* macrophage-skewing agent(s)), applications of the compositions described herein, and any combinations thereof. Immune cell-modulating agents (*e.g.,* macrophage-skewing agents) can be dispersed homogenously or heterogeneously in the biomaterial or silk fibroin-based matrix. As described herein, at least one immune cell-modulating agent (*e.g.,* at least one macrophage-skewing agent) can be encapsulated into the biomaterial. At least one immune cell-modulating agent (*e.g.,* at least one macrophage-skewing agent) can be present on a surface of the biomaterial. At least one immune cell-modulating agent (*e.g.,* at least one macrophage-skewing agent) can be encapsulated into the silk fibroin-based matrix. At least one immune cell-modulating agent (*e.g.,* at least one macrophage-skewing agent) can be present on a surface of the silk fibroin-based matrix.

As described herein, different immune cell-modulating agents (*e.g.,* macrophage-skewing agents) can be dispersed in separate layers of the biomaterial or the silk fibroin-based matrix. For example, a multi-layered biomaterial or silk fibroin-based matrix can comprise a first immune cell-modulating agent (*e.g.,* a first macrophage-skewing agent) in its first layer and a second immune cell-modulating agent (*e.g.,* a second macrophage-skewing agent) in its second layer.

Without wishing to be limiting, the immune cell-modulating agent(s) (including, e.g., macrophage-skewing agent(s)) can also be distributed as described herein.

Selection of immune cell-modulating agent(s) (*e.g.,* macrophage-skewing agent(s)) can be determined, *e.g.,* depending on types of immune cells to be targeted, and/or applications of the compositions described herein. At least one immune cell-modulating agent (*e.g.,* at least one macrophage-skewing agent) can be selected to control response of immune cells (*e.g.,* macrophages) to a target stimulus *in vitro* or *in vivo.* At least one immune cell-modulating agent (*e.g.,* at least one macrophage-skewing agent) can be selected to control response of immune cells (*e.g.,* macrophages) to a target stimulus in a subject.

A target stimulus can comprise any *in vitro* or *in vivo* condition, object, and/or matter that can cause or induce immune cells (*e.g.,* macrophages) to respond or react, *e.g.,* inducing inflammatory responses and/or regenerative responses. A target stimulus can comprise a macrophage-associated condition. Exemplary macrophage-associated conditions can include, but are not limited to, bacterial infection, tissue regeneration, tissue damage, tissue reconstruction, including, e.g., tissue repair and/or augmentation (e.g., soft tissue repair and/or augmentation), arthritis, obesity, diabetes, arteriosclerosis, allograft transplantation, Langerhans cell histiocytosis (LCH), osteoporosis, glomerulonephritis, cancer, and any combinations thereof. The target stimulus can comprise an implantable structure (*e.g.,* but not limited to, scaffolds, allograft tissues, medical devices). The target stimulus can comprise a cytokine (*e.g.,* including a chemokine).

At least one immune cell-modulating agent can be selected to target specific types of immune cells described herein. Where macrophages are to be targeted, the immune cell-modulating agent(s) can comprise at least one or more macrophage-skewing agents. Accordingly, a composition described herein can comprise a biomaterial (*e.g.,* a silk fibroin-based matrix) comprising or functionalized with at least one macrophage-skewing agent. The at least one macrophage-skewing agent can be present in the silk fibroin-based matrix in an effective amount sufficient to selectively control or alter activation state of macrophages upon contact with the at least one macrophage-skewing agent.

At least one macrophage-skewing agent can comprise an agent selected to switch at least a population of the macrophages to M1 phenotypic state. At least one macrophage-skewing agent can comprise an agent selected to switch at least a population of the macrophages to M2 phenotypic state (*e.g.,* M2a, M2b, and M2c). Non-limiting examples of a macrophage-skewing agent can include glucocorticoid (*e.g.,* dexamethasone); nicotine; statins (*e.g.,* simvastatin); an antimicrobial peptide (*e.g.,* LL-37 peptide, apolipoprotein E); LPS; INF-γ; TNF-α; prolactin; Notch activators (*e.g.,* delta or jagged ligands); IL-4; IL-13; IL-10; insulin sensitizer and PPAR-γ inducer (*e.g.,* rosiglitazone or other thiazolidinediones); HDAC inhibitors (*e.g.,* VPA); Notch signaling inhibitors (*e.g.,* GSI or DAPT); JAK inhibitors (*e.g.,* AG490); inhibitors of 3-hydroxy-3-methylglutaryl-coenzyme A (HMG-CoA) reductase; LiCl; thymosin; PLA2, and any combinations thereof.

The compositions and/or the biomaterials (*e.g.,* silk fibroin-based matrices) described herein can be adapted for various applications. For example, the composition can be formulated for use in treatment of the macrophage-associated condition described herein. The composition can be adapted for use as a wound dressing. The composition can be adapted for use as a coating, *e.g.,* forming a coating of an implantable structure. Accordingly, depending on the applications and/or application formats, the biomaterial (*e.g.,* silk fibroin-based matrix) can exist in any form. The biomaterial can be in a form of a film, a fiber, a collection of particles, a tube, a mat, a gel, a mesh, a fabric, or any combinations thereof. The biomaterial comprising a silk fibroin-based matrix can be in a form of a film, a fiber, a collection of particles, a tube, a mat, a gel, a mesh, a fabric, or any combinations thereof.

The compositions described herein can be used to control immune response, *e.g.,* macrophage response *in vitro* or *in vivo.* Accordingly, also described herein are methods, *e.g.,* for controlling response of immune cells to a target stimulus described herein. Exemplary immune cells can include, but are not limited to, monocytes, macrophages, dendritic cells, megakaryocytes, granulocytes, T cells, B cells, natural killer (NK) cells, and any combinations thereof.

The method can comprise: (a) placing in close proximity to or at a target site one embodiment of the compositions described herein, wherein the composition can comprise a biomaterial comprising at least one immune cell-modulating agent in an effective amount sufficient to selectively alter activation state of at least one type of immune cells; and (b) releasing the immune cell-modulating agent at a pre-determined rate from the biomaterial, upon the placement at the target site, to alter the ratio of activated immune cells to inactivated immune cells surrounding the biomaterial. When the ratio of activated immune cells to inactivated immune cells reaches above a threshold, the biomaterial described herein can induce an inflammatory response; and when the ratio of activated immune cells to inactivated immune cells reaches below a threshold, the biomaterial described herein can induce a regenerative or anti-inflammatory response.

The at least one immune cell-modulating agent can be present in an effective amount sufficient to selectively alter activation state of at least dendritic cells.

The at least one immune cell-modulating agent can be present in an effective amount sufficient to selectively alter activation state of at least macrophages. In these cases, the at least one immune cell-modulating agent can comprise at least one macrophage-skewing agent. Thus, methods, *e.g.,* for controlling response of at least macrophages, are also described herein. The method can comprise (a) placing in close proximity to or at a target site one embodiment of the compositions described herein, wherein the composition can comprise a biomaterial comprising at least one macrophage-skewing agent in an effective amount sufficient to selectively alter activation state of macrophages; and (b) releasing the macrophage-skewing agent at a pre-determined rate from the biomaterial, upon the placement at the target site, to alter the ratio of Ml macrophages to M2 macrophages surrounding the biomaterial. When the ratio of M1 macrophages to M2 macrophages is above a threshold, the biomaterial described herein can induce an inflammatory response; and when the ratio of M1 macrophages to M2 macrophages is below a threshold, the biomaterial described herein can induce a regenerative or anti-inflammatory response.

Where the ratio of M1 macrophages to M2 macrophages is above a threshold, the inflammatory response induced by the biomaterial can comprise inducing degradation of the biomaterial. In some embodiments, the inflammatory response induced by the biomaterial can comprise protecting tissue in close proximity and/or at the target site from infection (*e.g.,* bacterial infection).

Where the ratio of M1 macrophages to M2 macrophages is below a threshold, the regenerative or anti-inflammatory response can comprise facilitating tissue repair and/or regeneration at the target site. In some embodiments, the regenerative or anti-inflammatory response can comprise reducing rejection of the biomaterial by a host's immune system.

In some methods described herein, the target site to be treated can comprise a target stimulus described herein to which the response of immune cells (*e.g.,* macrophages) are to be controlled.

The target site to be treated can be present *in vitro* or *in vivo.* Described herein is a method for controlling macrophage response to a target stimulus in a subject. The method comprises placing in close proximity to or at the site of a target stimulus a composition comprising a biomaterial (*e.g.,* a biomaterial comprising a silk fibroin-based matrix) comprising at least one macrophage-skewing agent in an effective amount sufficient to (a) switch at least a population of macrophages to M1 phenotypic state, thereby inducing an inflammatory response to the target stimulus; (b) switch at least a population of macrophages to M2 phenotypic state, thereby inducing a regenerative response to the stimulus; or (c) both (a) and (b). In some embodiments, the biomaterial can comprise a silk fibroin-based matrix.

A macrophage-skewing agent to be employed in the compositions described herein can include any agent that can alter activation state of macrophages between M1 phenotype and M2 phenotype, or differentiate precursor cells (*e.g.,* monocytes) to M1 macrophages or M2 macrophages. In some embodiments of various methods described herein, the at least one macrophage-skewing agent can include, but are not limited to, glucocorticoid (*e.g.,* dexamethasone); nicotine; statins (*e.g.,* simvastatin); an antimicrobial peptide *(e.g.,* LL-37 peptide, apolipoprotein E); LPS; INF-γ; TNF-α; prolactin; Notch activators (*e.g.,* delta or jagged ligands); IL-4; IL-13; IL-10; insulin sensitizer and PPAR-γ inducer (*e.g.,* rosiglitazone or other thiazolidinediones); HDAC inhibitors (*e.g.,* VPA); Notch signaling inhibitors (*e.g.,* GSI or DAPT); JAK inhibitors (*e.g.,* AG490); inhibitors of 3-hydroxy-3-methylglutaryl-coenzyme A (HMG-CoA) reductase; LiCl; thymosin; PLA2, and any combinations thereof.

As described earlier, a target stimulus to be involved in the methods described herein can comprise any *in vitro* or *in vivo* condition, object, and/or matter that can cause or induce immune cells (*e.g.,* macrophages) to respond or react, *e.g.,* inducing inflammatory responses and/or regenerative responses. In various methods described herein, the target stimulus can comprise a macrophage-associated condition. Non-limiting examples of a macrophage-associated condition can include bacterial infection, tissue regeneration, tissue damage, tissue reconstruction, including, e.g., tissue repair and/or augmentation (e.g., soft tissue repair and/or augmentation), arthritis, obesity, diabetes, arteriosclerosis, allograft transplantation, Langerhans cell histiocytosis (LCH), osteoporosis, glomerulonephritis, cancer, and any combinations thereof. In some embodiments, the target stimulus can comprise an implantable structure (*e.g.,* but not limited to, scaffolds, allograft tissues, medical devices). In some embodiments, the target stimulus can comprise a cytokine (*e.g.,* including chemokine).

The macrophage-skewing agent(s) can be distributed homogenously or heterogeneously in the biomaterial or silk fibroin-based matrix. In some embodiments, the macrophage-skewing agent(s) can be distributed in the same layer and/or in separate layers, *e.g.,* in a multi-layered biomaterial or silk fibroin-based matrix. In some methods described herein, the macrophage-skewing agent(s) can be encapsulated into the biomaterial. The macrophage-skewing agent(s) can be present on a surface of the biomaterial. The macrophage-skewing agent(s) can be encapsulated into the silk fibroin-based matrix. The macrophage-skewing agent(s) can be present on a surface of the silk fibroin-based matrix. Without wishing to be limiting, the macrophage-skewing agent can also be distributed in some of the compositions described herein.

In some methods described herein, the composition can be formulated for use in treatment of the macrophage-associated condition. The composition can be adapted for use as a wound dressing. The composition can be adapted for use as a coating, *e.g.,* forming a coating of an implantable structure or device. The biomaterial can be in a form of a film, a fiber, a collection of particles, a gel, a fabric, a mesh, or any combinations thereof. The silk fibroin-based matrix can be in a form of a film, a fiber, a collection of particles, a tube, a mat, a gel, a fabric, a mesh, or any combinations thereof.

In various methods described herein, the composition comprising the biomaterial (*e.g.,* a biomaterial comprising a silk fibroin-based matrix) can be placed in close proximity or at the target site by any methods known in the art. The composition comprising the biomaterial (*e.g.,* silk fibroin-based matrix) can be placed by injection. The composition comprising the biomaterial (*e.g.,* silk fibroin-based matrix) can be placed by implantation.

In a further aspect, provided herein is a method for producing a biomedical material as defined in the claims appended hereto.

### BRIEF DESCRIPTION OF THE DRAWINGS

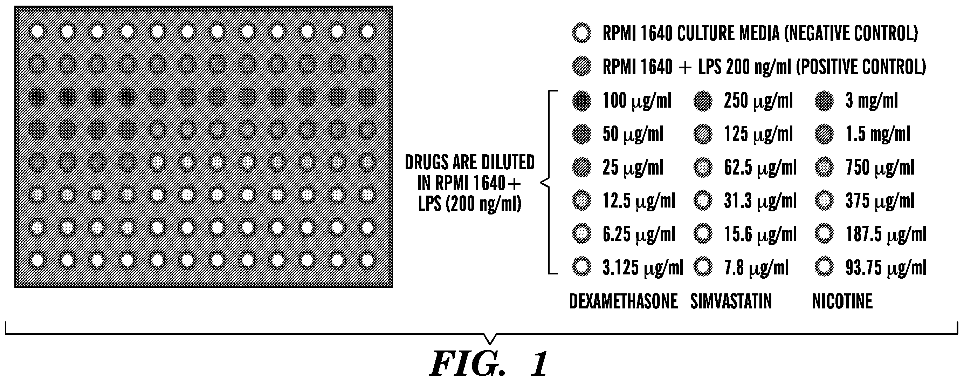

**Figure 1** depicts the schematic of a 96-well plate set-up used in Example 2 for evaluation of cytokine expression and secretion from differentiated THP1 monocytes seeded on ∼2 µm-sized silk fibroin particles (SP1) and modulation of the monocytes with antiinflammatory drugs (*e.g.,* but not limited to, dexamethasone, simvastatin, nicotine)

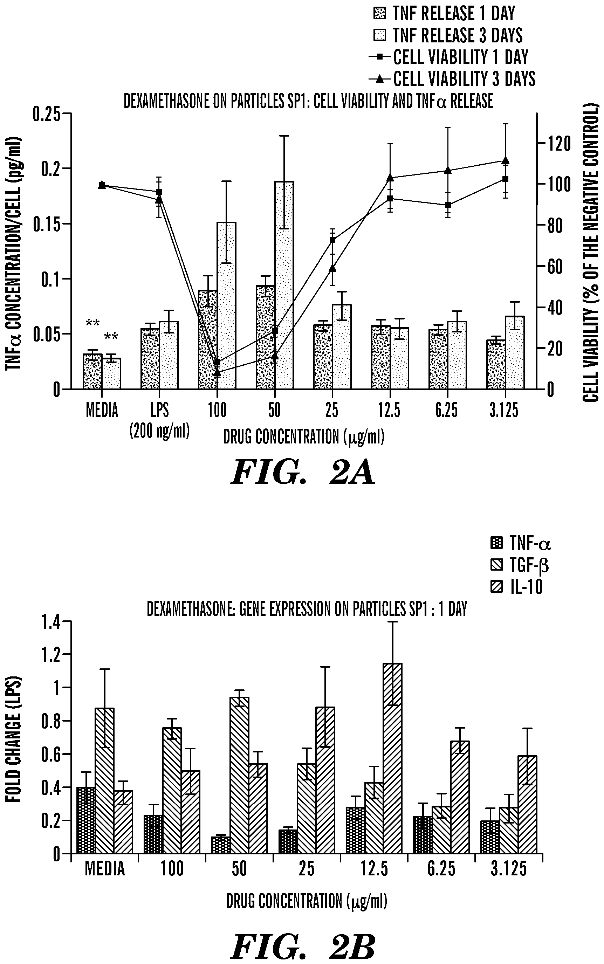

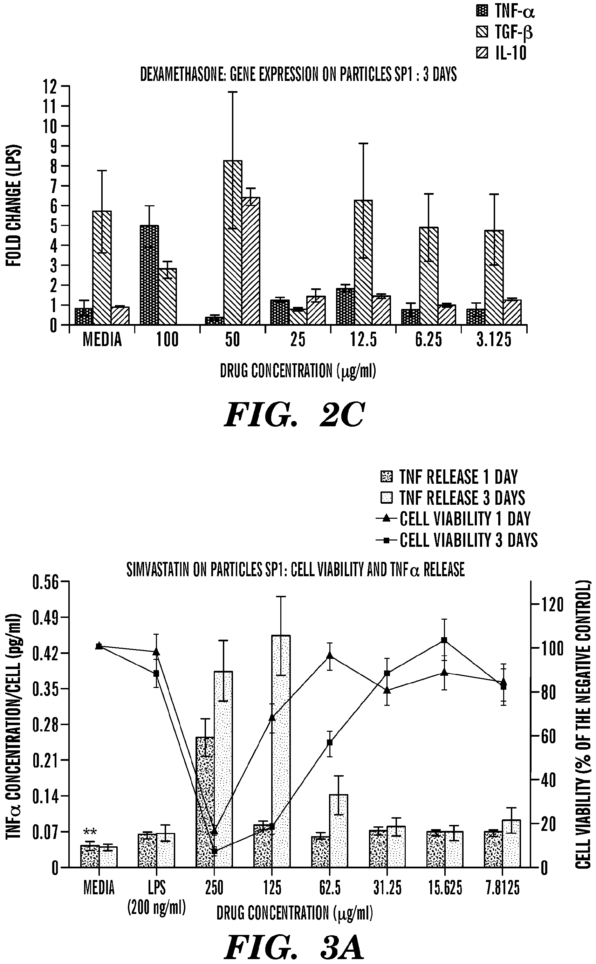

**Figures 2A-2C** are bar graphs showing responses of THP1 macrophages seeded on SP1 particles and stimulated with dexamethasone. **Figure 2A** is a graph of cell viability and TNF-α release at Day 1 and Day 3. **Figures 2B** and **2C** display TNF-α, TGF-β and IL-10 gene expression at Day 1 **(****Figure 2B****)** and Day 3 **(****Figure 2C****),** respectively.

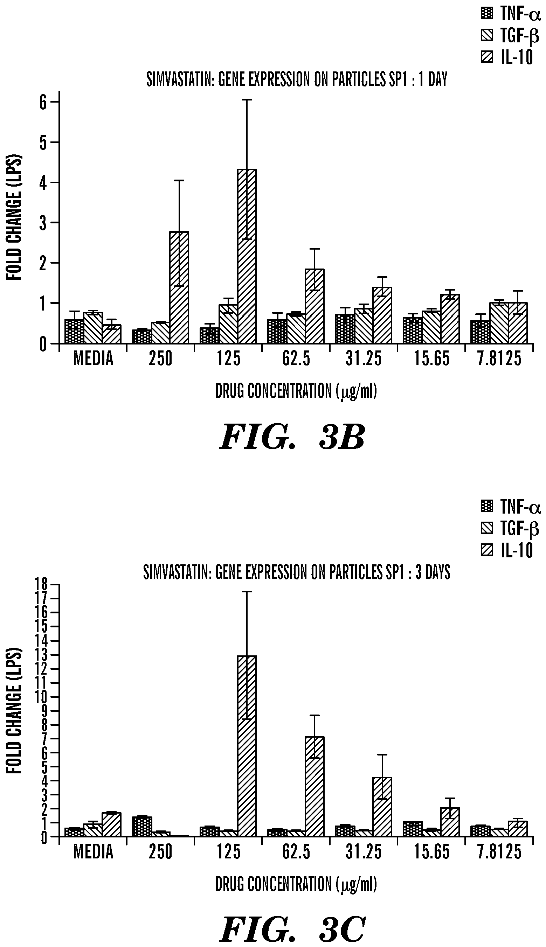

**Figures 3A-3C** are bar graphs showing responses of THP1 macrophages seeded on SP1 particles and stimulated with simvastatin. **Figure 3A** is a graph of cell viability and TNF-α release at Day 1 and Day 3. **Figures 3B** and **3C** display TNF-α, TGF-β and IL-10 gene expression at Day 1 **(****Figure 3B****)** and Day 3 **(****Figure 3C****).**

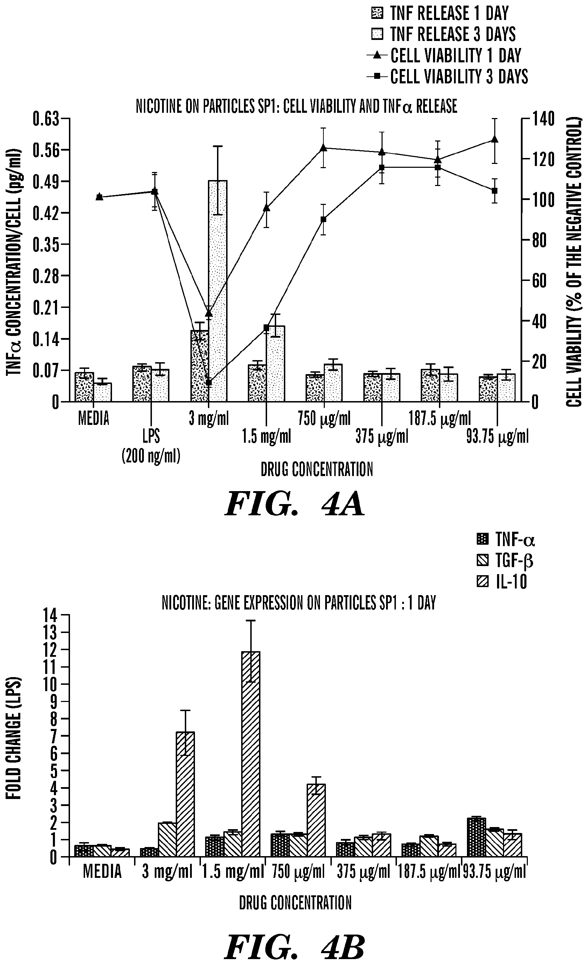

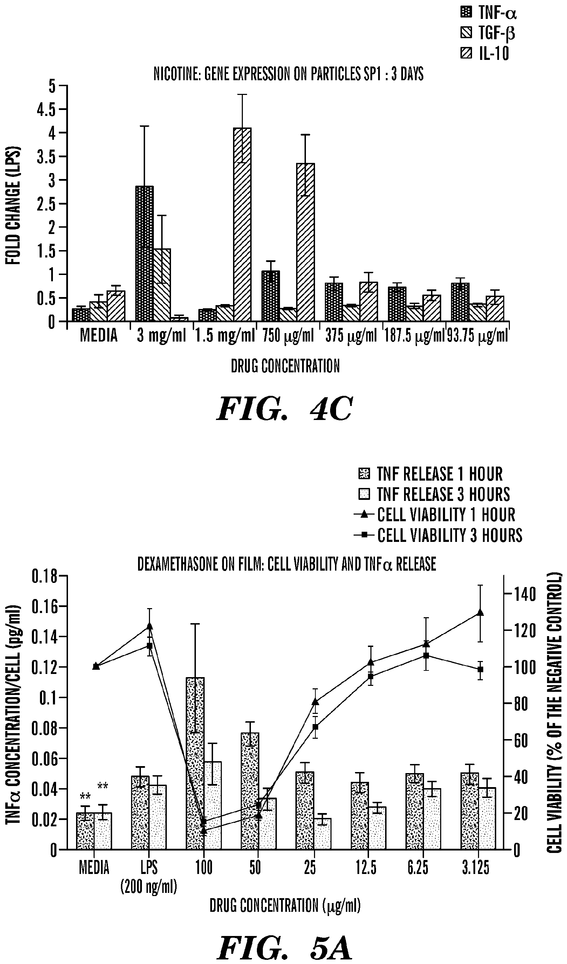

**Figures 4A-4C** are bar graphs showing responses of THP1 macrophages seeded on SP1 particles and stimulated with nicotine. **Figure 4A** is a graph of cell viability and TNF-α release at Day 1 and Day 3. **Figures 4B** and **4C** display TNF-α, TGF-β and IL-10 gene expression at Day 1 **(****Figure 4B****)** and Day 3 **(****Figure 4C****).**

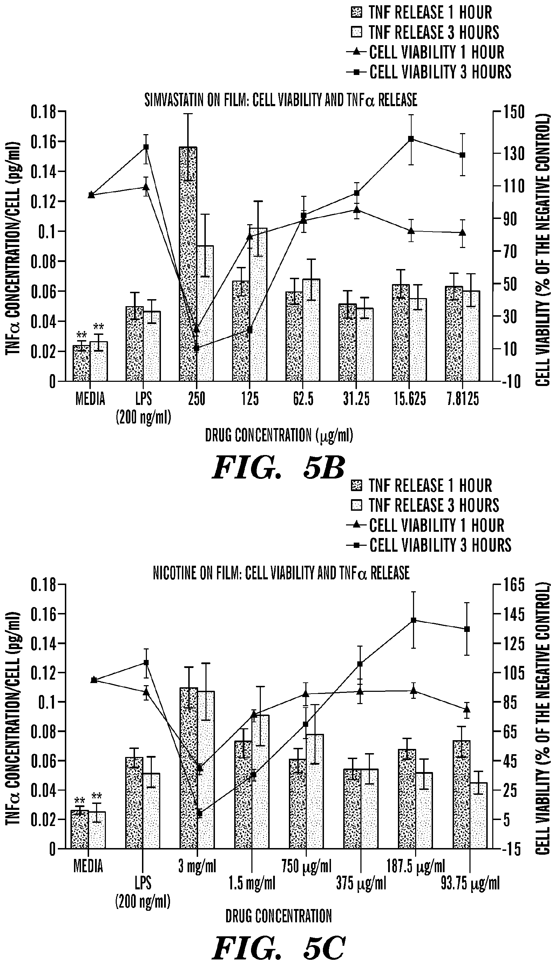

**Figures 5A-5C** are data graphs showing cell viability and TNF-α release of THP1 macrophages seeded on silk fibroin films and stimulated with various anti-inflammatory drugs (*e.g.,* dexamethasone, simvastatin, nicotine) under the same culture conditions as **Figures 2A-2C****,** **3A-3C****,** and **4A-4C**. Cells were stimulated with dexamethasone **(****Figure 5A****),** simvastatin **(****Figure 5B****),** or nicotine **(****Figure 5C****).** Results shown represent 5 experiments (20 samples).

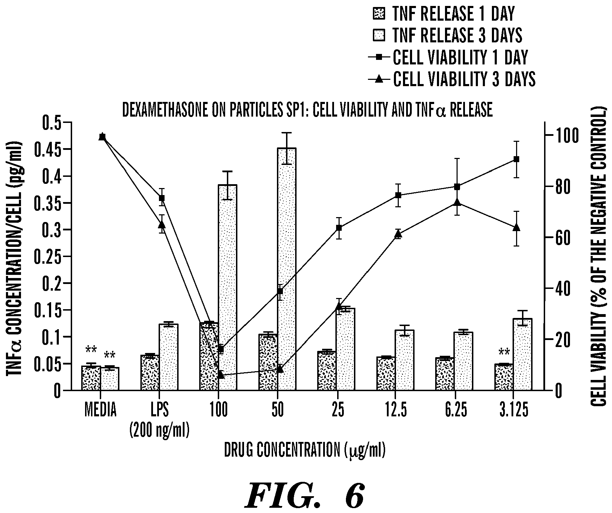

**Figure 6** depicts a graph of TNF-α release and the cell viability of THP1 macrophages stimulated by SP1 particles and modulated with different concentrations of dexamethasone after 1 day or 3 days.

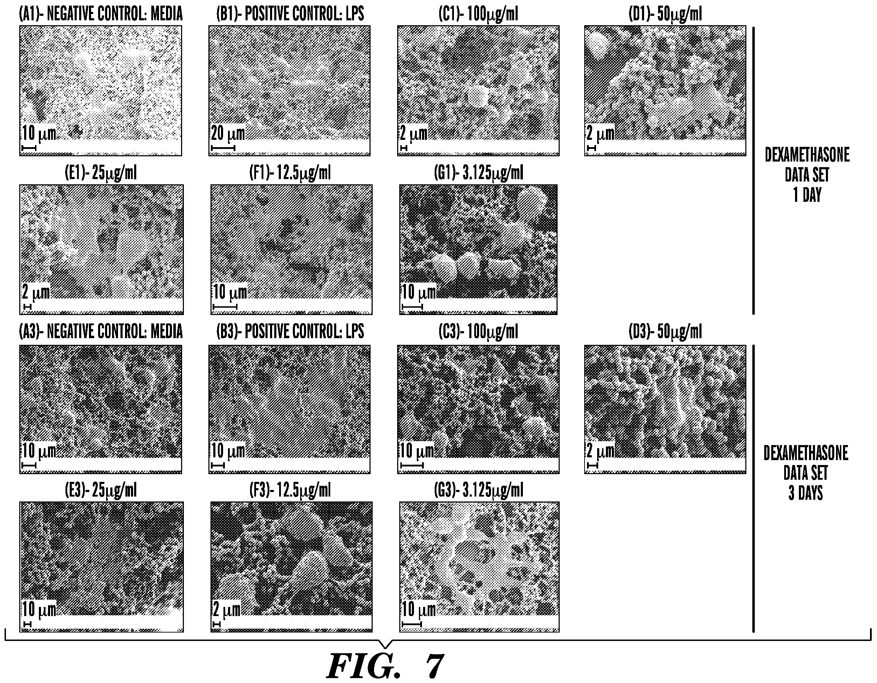

**Figure 7** depicts SEM images of THP1 macrophages seeded on SP1 particles and stimulated with various concentrations of dexamethasone after 1 day or 3 days. **Figure 7** also depicts SEM images of THP1 macrophages seeded on SP1 particles in culture medium alone (negative control) and in the presence of LPS (positive control).

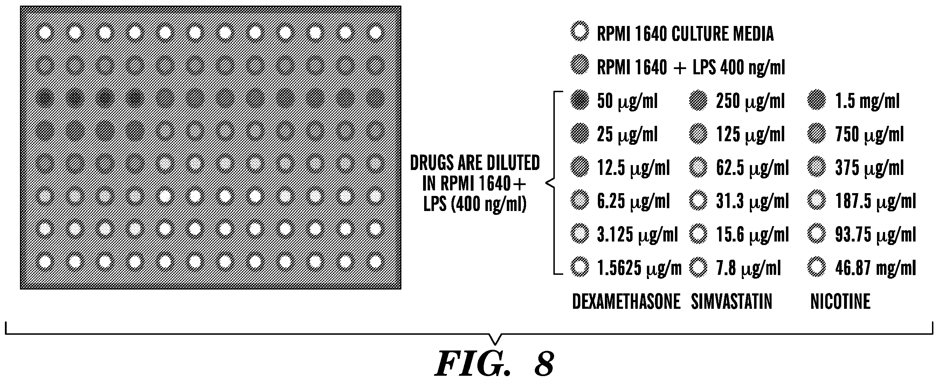

**Figure 8** depicts the schematic of a 96-well plate set-up used in Example 3 for evaluation of cytokine expression and secretion from differentiated THP1 monocytes seeded on ∼1 µm-sized silk fibroin particles (SP0.5) and modulation of the monocytes with antiinflammatory drugs (*e.g.,* but not limited to, dexamethasone, simvastatin, nicotine)

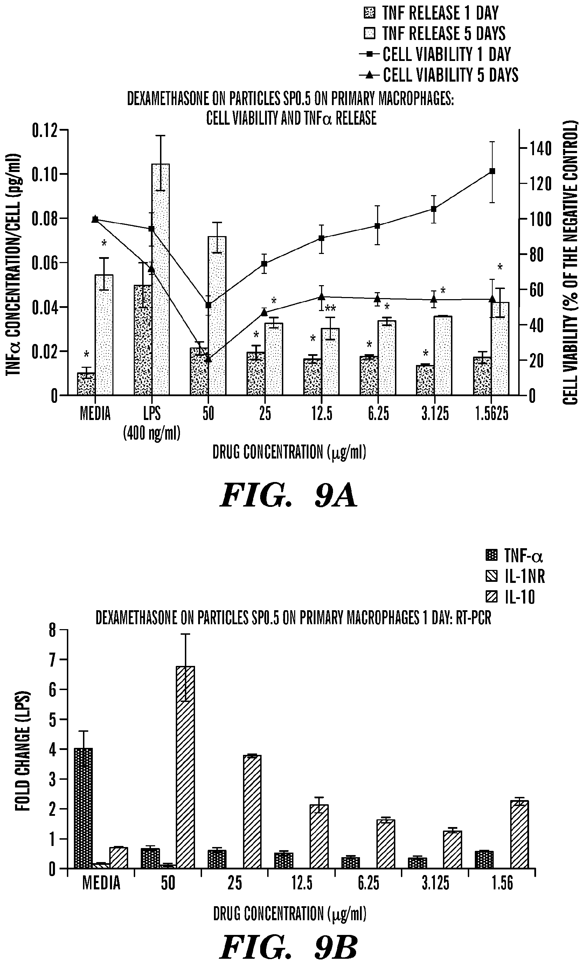

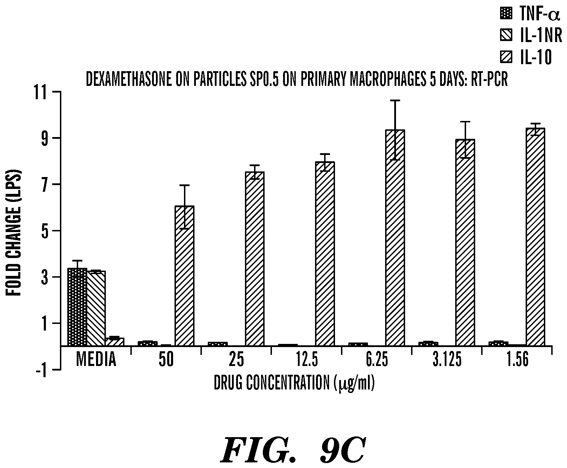

**Figures 9A-9C** are bar graphs showing responses of primary macrophages seeded on SP0.5 particles and stimulated with dexamethasone. **Figure 9A** is a graph of cell viability and TNF-α release at Day 1 and Day 5. **Figures 9B** and **9C** display TNF-α, IL-1RA and IL-10 gene expression at Day 1 **(****Figure 9B****)** and Day 5 **(****Figure 9C****).**

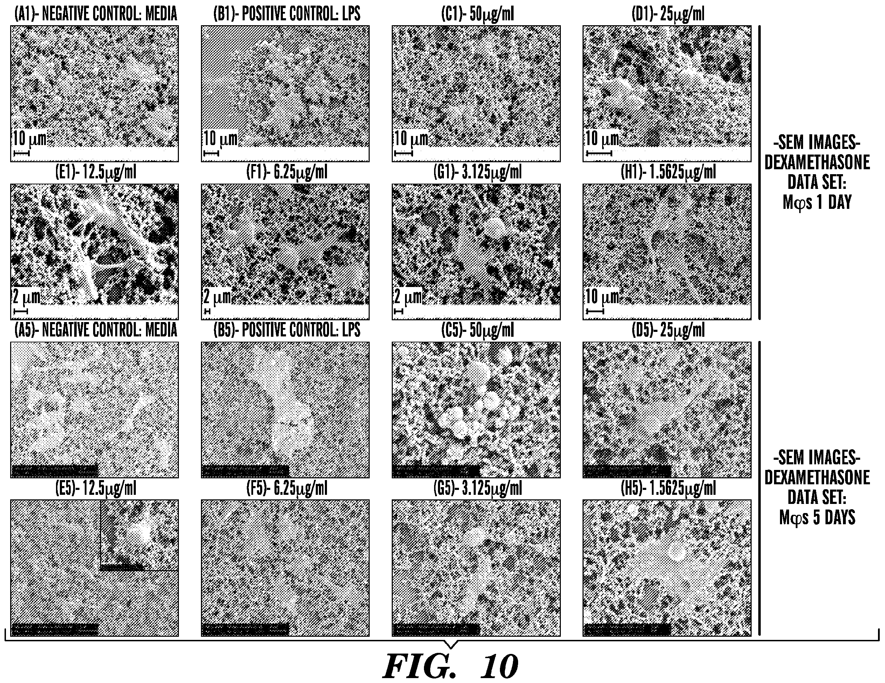

**Figure 10** depicts SEM images of primary macrophages seeded on SP0.5 particles and stimulated with dexamethasone. **Figure 10** also depicts SEM images of primary macrophages seeded on SP0.5 particles in culture medium alone (negative control) and in the presence of LPS (positive control).

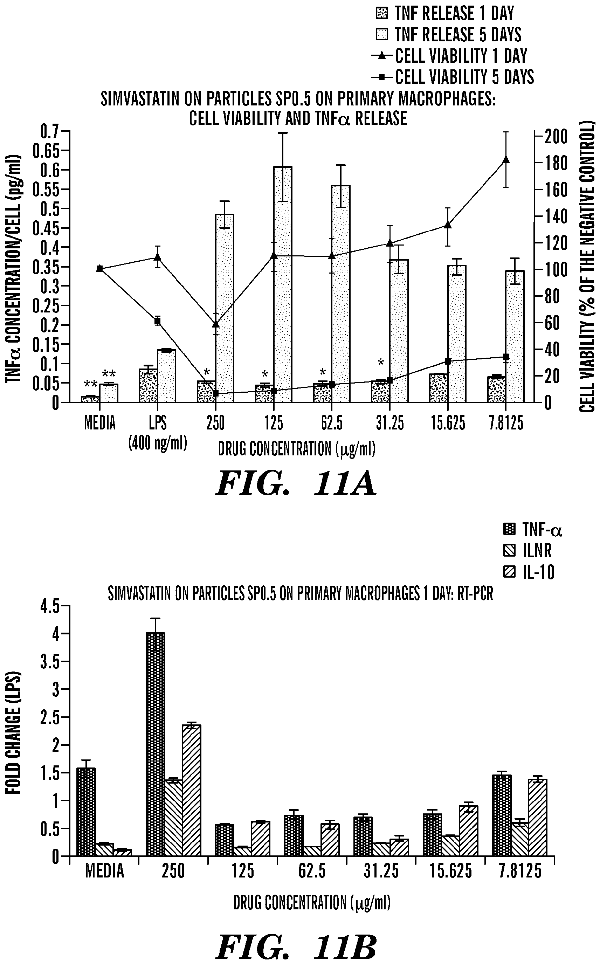

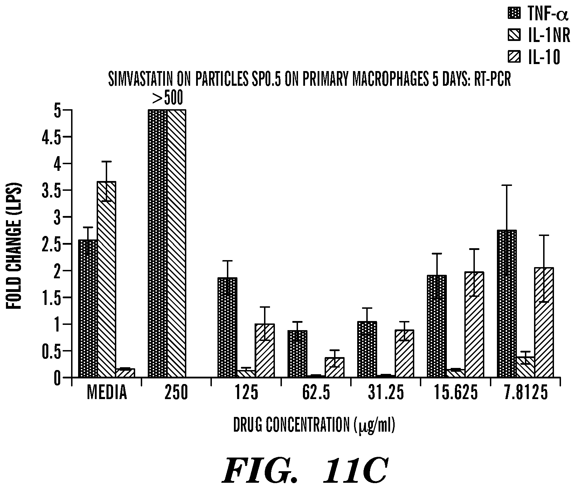

**Figures 11A-11C** are bar graphs showing responses of primary macrophages seeded on SP0.5 particles and stimulated with simvastatin. **Figure 11A** is a graph of cell viability and TNF-α release at Day 1 and Day 5. **Figures 11B** and **11C** display TNF-α, IL-1RA and IL-10 gene expression at Day 1 **(****Figure 11B****)** and Day 5 **(****Figure 11C****).**

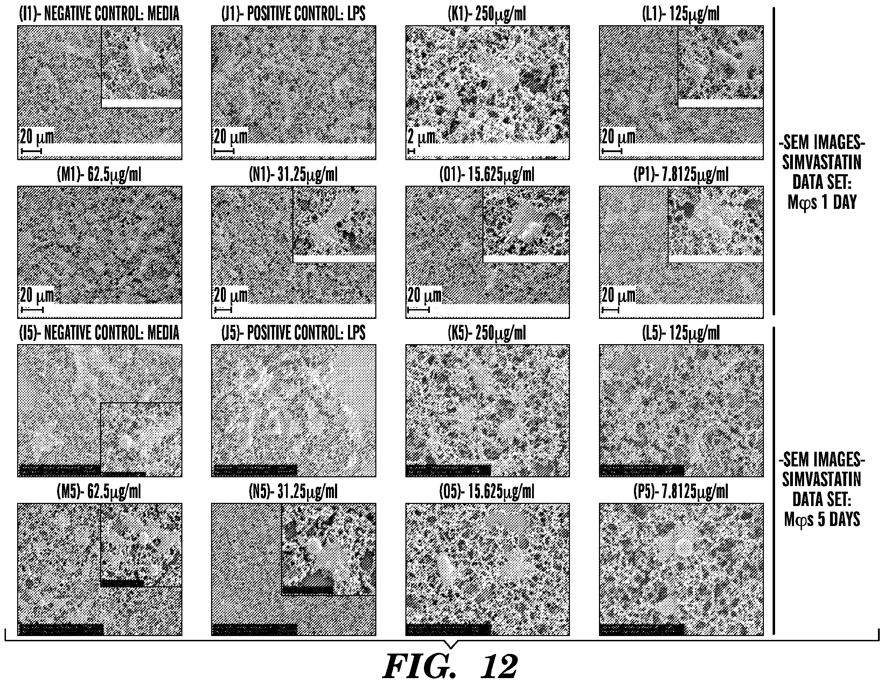

**Figure 12** depicts SEM images of primary macrophages seeded on SP0.5 particles and stimulated with simvastatin. **Figure 12** also depicts SEM images of primary macrophages seeded on SP0.5 particles in culture medium alone (negative control) and in the presence of LPS (positive control).

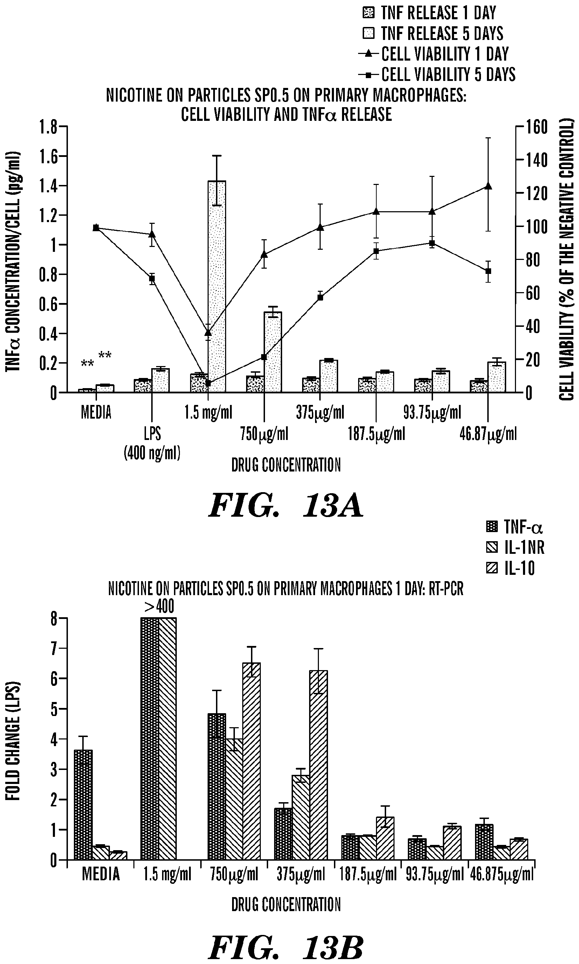

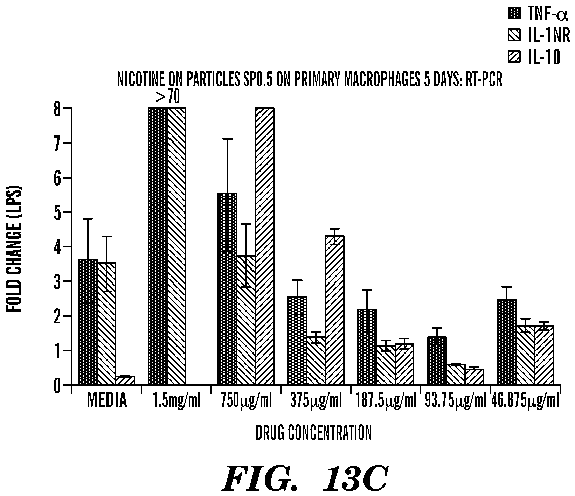

**Figures 13A-13C** are bar graphs showing responses of primary macrophages seeded on SP0.5 particles and stimulated with nicotine. **Figure 13A** is a graph of cell viability and TNF-α release at Day 1 and Day 5. **Figures 13B** and **13C** display TNF-α, IL-1RA and IL-10 gene expression at Day 1 **(****Figure 13B****)** and Day 5 **(****Figure 13C****).**

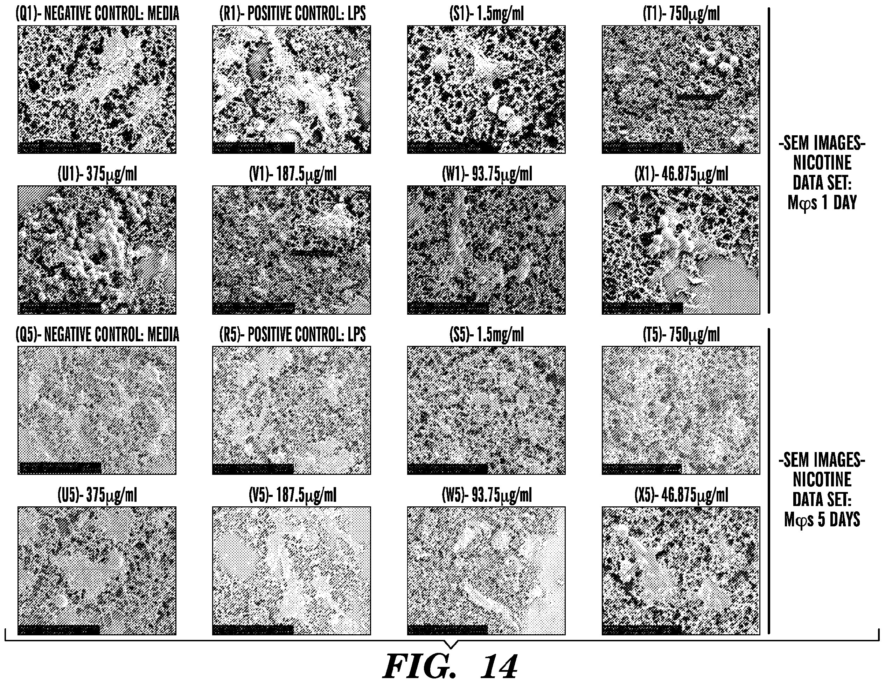

**Figure 14** depicts SEM images of primary macrophages seeded on SP0.5 particles and stimulated with nicotine. **Figure 14** also depicts SEM images of primary macrophages seeded on SP0.5 particles in culture medium alone (negative control) and in the presence of LPS (positive control).

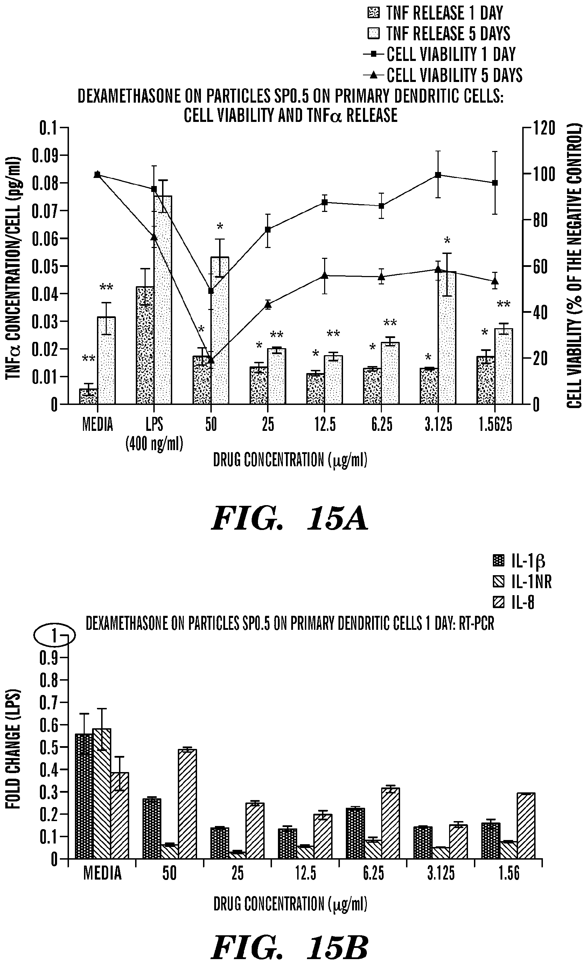

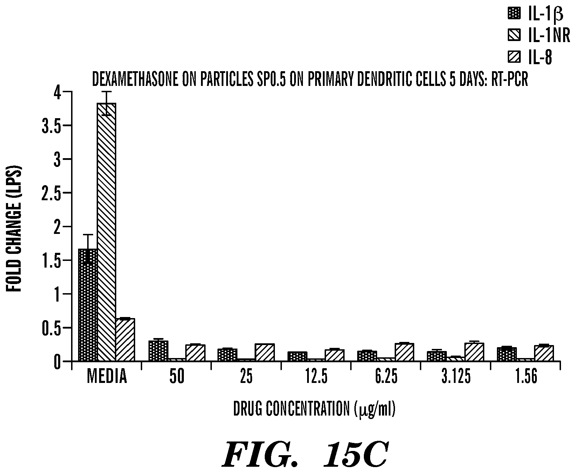

**Figures 15A-15C** are bar graphs showing responses of primary dendritic cells seeded on SP0.5 particles and stimulated with dexamethasone. **Figure 15A** is a graph of cell viability and TNF-α release at Day 1 and Day 5. **Figures 15B** and **15C** display IL-1β, IL-1RA and IL-8 gene expression at Day 1 **(****Figure 15B****)** and Day 5 **(****Figure 15C****).**

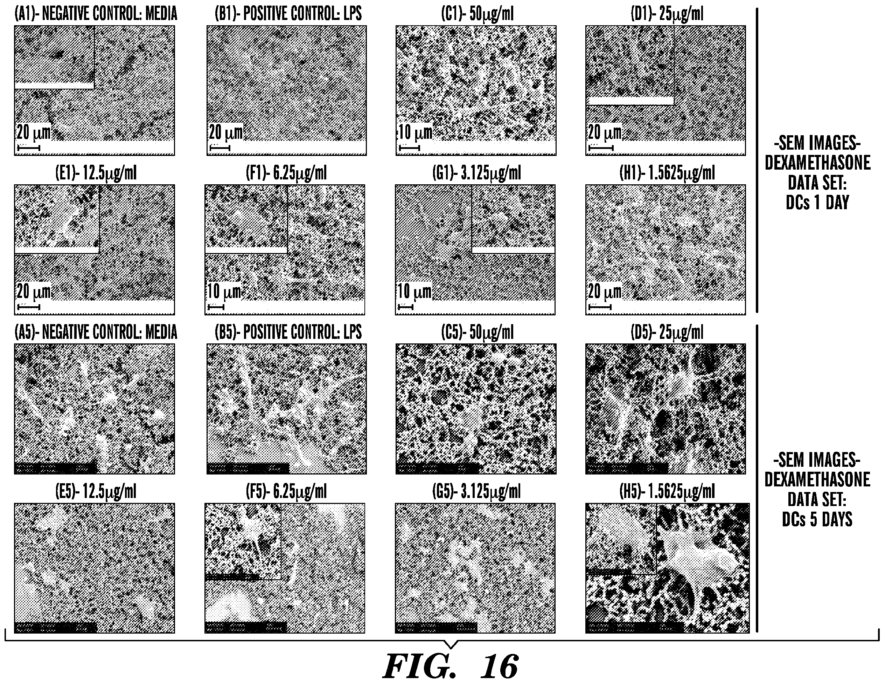

**Figure 16** depicts SEM images of primary dendritic cells seeded on SP0.5 particles and stimulated with dexamethasone. **Figure 16** also depicts SEM images of primary dendritic cells seeded on SP0.5 particles in culture medium alone (negative control) and in the presence of LPS (positive control).

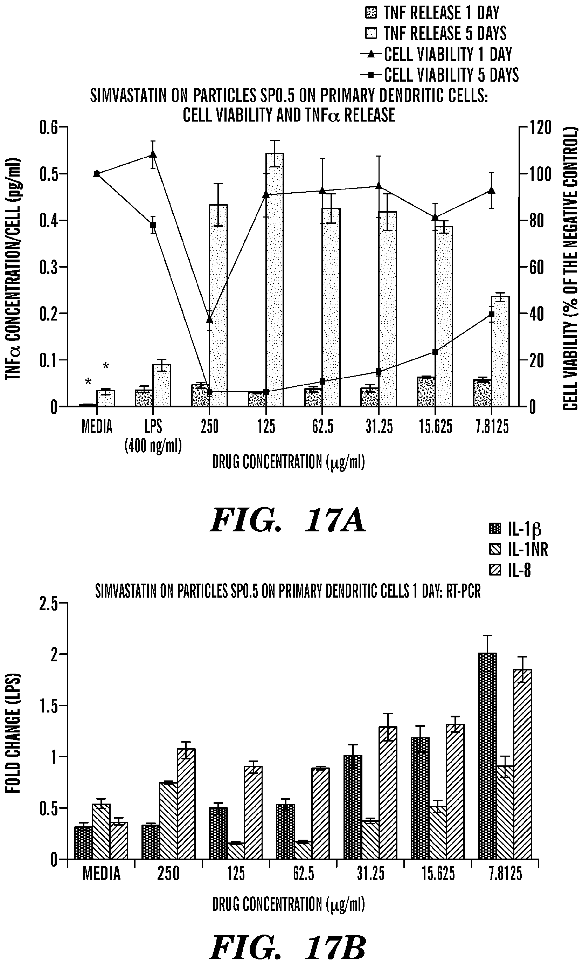

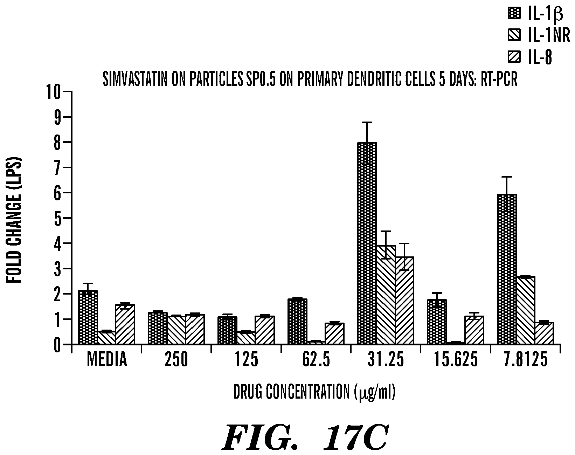

**Figures 17A-17C** are bar graphs showing responses of primary dendritic cells seeded on SP0.5 particles and stimulated with simvastatin. **Figure 17A** is a graph of cell viability and TNF-α release at Day 1 and Day 5. **Figures 17B** and **17C** display IL-1β, IL-1RA and IL-8 gene expression at Day 1 **(****Figure 17B****)** and Day 5 **(****Figure 17C****).**

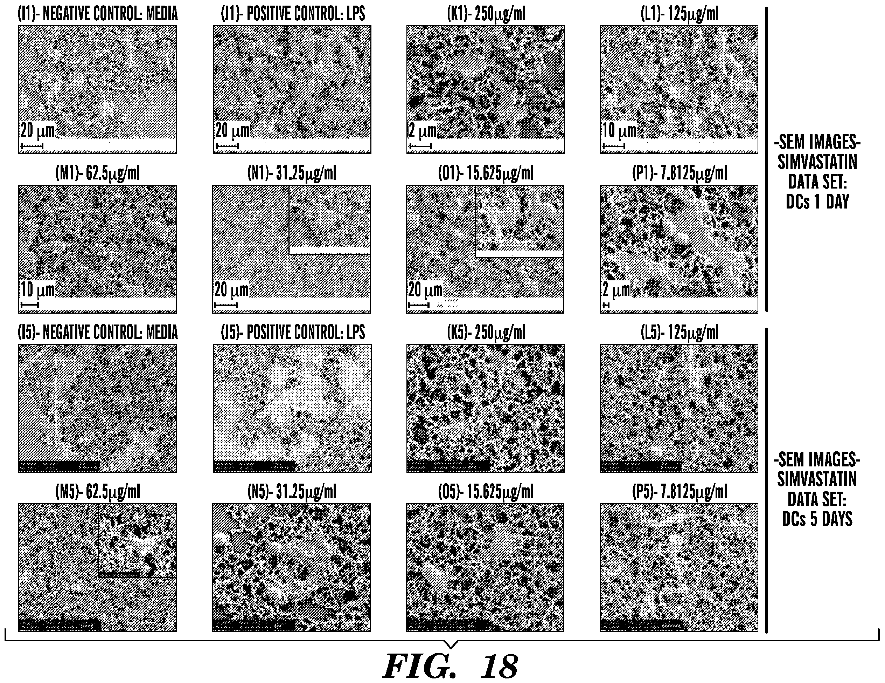

**Figure 18** depicts SEM images of primary dendritic cells seeded on SP0.5 particles and stimulated with simvastatin. **Figure 18** also depicts SEM images of primary dendritic cells seeded on SP0.5 particles in culture medium alone (negative control) and in the presence of LPS (positive control).

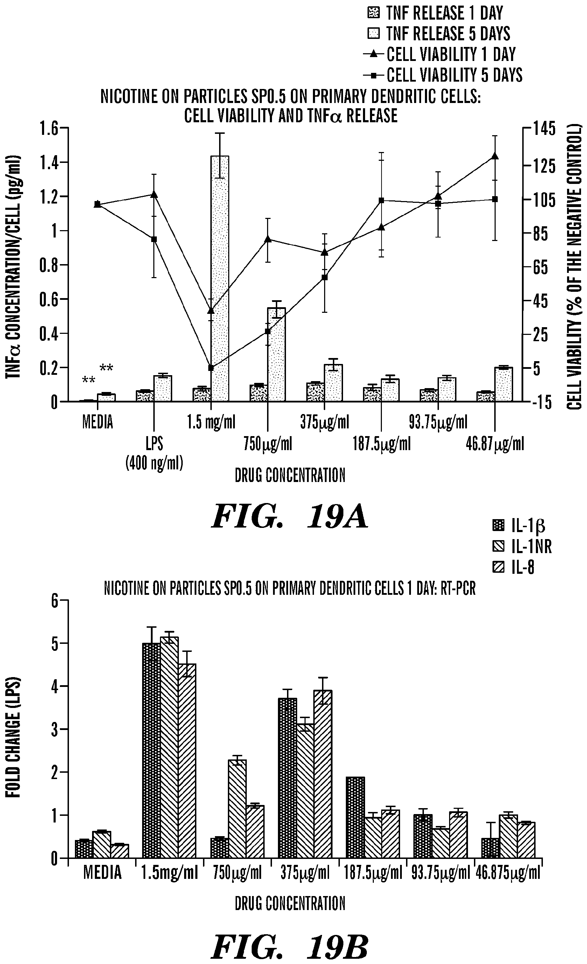

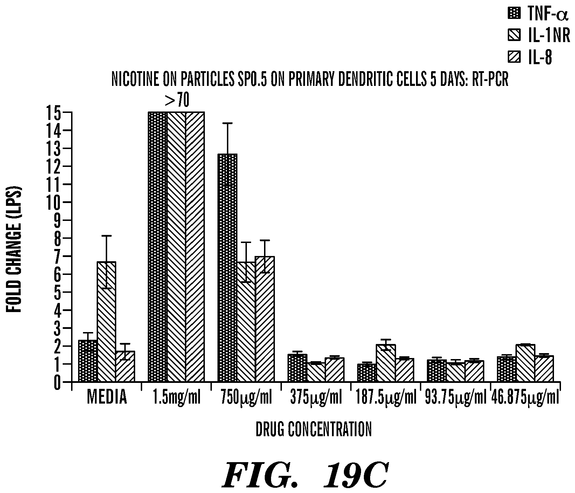

**Figures 19A-19C** are bar graphs showing responses of primary dendritic cells seeded on SP0.5 particles and stimulated with nicotine. **Figure 19A** is a graph of cell viability and TNF-α release at Day 1 and Day 5. **Figures 19B** and **19C** display IL-1β, IL-1RA and IL-8 gene expression at Day 1 **(****Figure 19B****)** and Day 5 **(****Figure 19C****).**

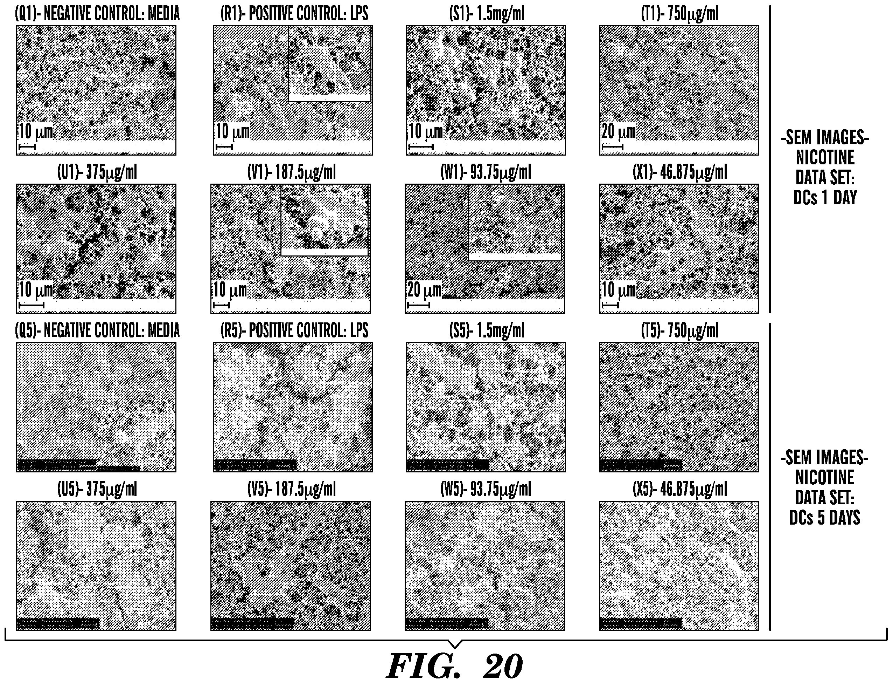

**Figure 20** depicts SEM images of primary dendritic cells seeded on SP0.5 particles and stimulated with nicotine. **Figure 20** also depicts SEM images of primary dendritic cells seeded on SP0.5 particles in culture medium alone (negative control) and in the presence of LPS (positive control).

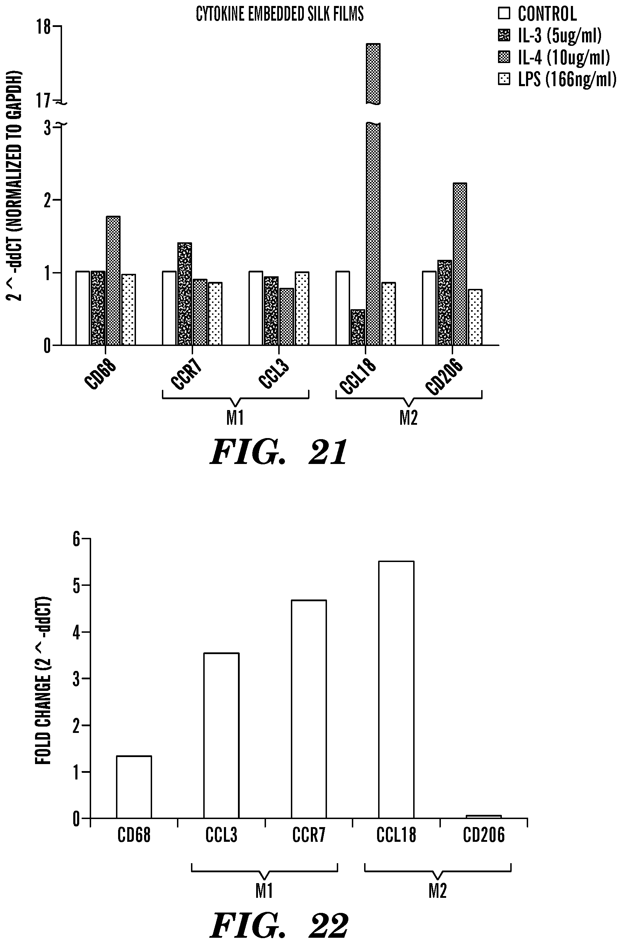

**Figure 21** depicts a graph demonstrating that at least about 10 µg/ml IL-4 embedded silk films induce M2 phenotype but ∼166 ng/ml LPS does not induce M1 phenotype. The addition of 10 µg/ml interleukin-4 (IL-4) to silk films is capable of upregulating the markers of M2 macrophages (*e.g.,* CCL18 and CD206) in THP-1 monocytes. M2 macrophages are typically involved in regenerating damaged tissues. LPS did not stimulate M1 differentiation at the low level of LPS that was added to the silk films.
**Figure 22** depicts a graph demonstrating the effects of INF-γ embedded silk films on macrophage differentiation or skewing. With 10 µg/ml interferon-gamma (INF-γ) in silk films the THP-1 monocytes upregulate the markers of M1 macrophages (*e.g.,* CCL3 and CCR7). CCL18, an M2 marker, also appears to be upregulated, whereas CD206, a common marker of M2 was strongly downregulated relative to control.

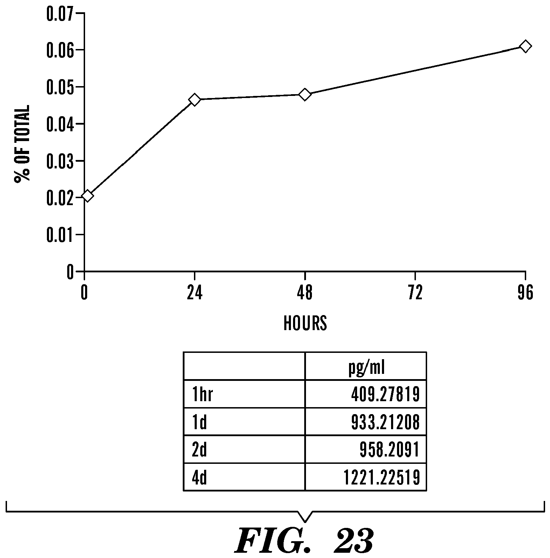

**Figure 23** demonstrates the release rate of IFN-γ from INF-γ embedded silk films described in **Figure 22****.** An ELISA was run to show the release rate of INF-γ from the silk films. The films were made from a silk fibroin solution of about 10 µg/ml and 0.2ml used per film (for a total loading of about 2 µg INF-γ). Within one hour a large amount of INF-γ is released but as a percentage of the total loading it is small and by 4 days more INF-γ is released showing that there is a continued or constant release of INF-γ. While the levels of INF-γ release are low, they are physiologically relevant.

### DETAILED DESCRIPTION OF THE INVENTION

In order to realize the full beneficial effects of biomaterial scaffolds, *e.g.,* to promote the constructive remodeling of injured tissues through mechanisms that include, *e.g.,* angiogenesis, the recruitment of multipotential progenitor cells to the site of tissue reconstruction, and/or the release of antimicrobial peptides, there is a need for an improved biomaterial that can selectively control immune response, *e.g.,* to optimize host-mediated degradation of the biomaterial and/or tissue repair and/or regeneration. To this end, the inventors have discovered that biomaterials can be modified, for example, chemically and/or via inclusion of appropriate active agents, particularly immune cell-modulating agents and/or macrophage-skewing agents, during processing or post processing, to regulate immune cells (*e.g.,* macrophages) and/or other cellular responses *in vitro* or *in vivo.* For example, introduction of a selective compound that can promote inflammation or reduce inflammation, to a cell population can influence degradation of the biomaterial, *e.g.,* degrading the biomaterial faster, slower, or in a more selective way, *e.g.,* to control inflammatory responses and/or regeneration responses for biomaterials and/or tissues. The control of the immune responses can be mediated by the nature of the cell populations (*e.g.,* macrophages) activated or promoted in the presence of the selective immune cell-modulating agents and/or macrophage-skewing agents, *e.g.,* but not limited to, small molecules, peptides, antimicrobial peptides, and/or lipopolysaccharides.

Accordingly, described herein are compositions and methods directed to functionalization of biomaterials with at least one active agent (*e.g.,* at least one immune cell-modulating agent and/or macrophage-skewing agent) that can modulate or skew the response of at least one type of immune cells (*e.g.,* but not limited to macrophages and/or dendritic cells) to a target stimulus (*e.g.,* but not limited to tissue damage, an implant, and/or a cytokine) and/or to the biomaterial itself *in vitro* or *in vivo.* As described herein, compositions comprising biomaterials functionalized with at least one immune cell-modulating agent (including, *e.g.,* macrophage-skewing agents) can promote or reduce inflammation mediated by immune cells such as macrophages, thereby modulating the immune response (*e.g.,* inducing inflammation or regeneration) to a target stimulus (*e.g.,* but not limited to tissue damage, an implant, and/or a cytokine) and/or to the biomaterial itself.

Thus, described herein are compositions, *e.g.,* for controlling, modulating or skewing immune response (*e.g.,* macrophage response to a target stimulus) *in vitro* or in a subject. The composition comprises a biomaterial functionalized with at least one immune cell-modulating agent (*e.g.,* at least one macrophage-skewing agent), wherein the immune cell-modulating agent (*e.g.,* the macrophage-skewing agent) selectively controls activation state of immune cells (*e.g.,* macrophages) upon contact with the at least one immune cell-modulating agent (*e.g.,* at least one macrophage-skewing agent). The biomaterial can comprise a silk fibroin-based matrix.

to the invention finds application in methods, *e.g.,* for controlling immune response (*e.g.,* macrophage response to a target stimulus) *in vitro* or in a subject. The method comprises placing in close proximity to the target stimulus one or more embodiments of the composition described herein.

In a further aspect, provided herein relate to a method for producing a biomedical material comprising coating a first material with one or more embodiments of the composition described herein. In some embodiments, the biomaterial can comprise a silk fibroin-based matrix. The first material can include any object, matter and/or composition that can be coated with a silk fibroin-based matrix. Examples of the first material can include, are not limited to, an implantable structure (*e.g.,* scaffold, allograft tissue, medical device), a pharmaceutical composition, a biomaterial, and any combinations thereof.

The first material can be coated with one or more embodiments of the composition described herein by any methods known in the art, *e.g.,* but not limited to, dip-coating, and/or spray coating.

### Compositions, e.g., for controlling immune responses in vitro or in vivo

As used herein, the term "selectively control or alter" refers to the ability of a selective immune cell-modulating agent to control or alter activation state of target immune cells with a greater likelihood and/or potency than it controls or alter activation state of non-target immune cells. In some embodiments, selective control or alteration refers to controlling or altering target immune cells with a likelihood that is at least 2, 5, 10, 25, 50, 75, 100, 150, 200, 250, 500, 1000 or more times greater than the likelihood for non-target immune cells. In some embodiments, selective control or alteration refers to controlling or altering target immune cells with a potency that is at least 2, 5, 10, 25, 50, 75, 100, 150, 200, 250, 500, 1000 or more times greater than the potency for non-target immune cells. In some embodiments, the selective immune cell-modulating agent can control or alter activation state of target immune cells but not non-target immune cells.

IThe immune cell-modulating agent can be selected to control or alter activation state of dendritic cells. The immune cell-modulating agent, *e.g.,* macrophage-skewing agent, can be selected to control or alter activation state of macrophages.

As used herein, the term "activation state" of an immune cell is understood to mean that the immune cell is in a phase between different activation states or between an activation state and a non-activation state. Depending on the activation state of an immune cell, the immune cell can produce a different immune response and/or function. Accordingly, in some embodiments, the term "activation state" of an immune cell refers to the ability of the immune cell to display a specific function depending on the state of the immune cell. By way of example only where an immune cell is a macrophage, the macrophage can be activated to display different functions in response to immune cell-modulating agents present in the surrounding environment. In response to Th1 cytokines, such as IFN-γ and/or pathogen-associated molecular patterns (PAMPs) such as LPS, macrophages adopting a M1 phenotype display a classical activation phenotype. They generally produce proinflammatory cytokines such as TNF-α, IL-1, IL-6, IL-12 and IL-23, and possess anti-proliferative functions (30, 35-37). Alternatively, macrophages can be activated by Th2 cytokines, such as IL-4 and IL-13, and adopt M2 phenotype. M2 macrophages can be subdivided into three subsets: M2a, M2b and M2c based on their phenotype. M1 and M2b are generally inflammatory and microbicidal whereas M2a and M2c generally have anti-inflammatory and/or tissue repair properties and secrete IL-10, IL-1 antagonist receptor (IL-1Ra), and Transforming Growth Factor β (TGF-β) cytokines. The M1 macrophage can elicit an inflammatory response, *e.g.,* to protect newly damaged tissue from infection, while the M2 macrophage can elicit a regenerative response, *e.g.,* to regenerate a tissue. Accordingly, the compositions and methods described herein, in some embodiments, can be used to selectively switch macrophages between a M1 state and a M2 state. In some embodiments, the compositions and methods described herein can be used to induce macrophages to produce an inflammatory response or a regenerative response.

Methods for determining the activation state of immune cells (*e.g.,* macrophages and dendritic cells) are known in the art. In some embodiments, the activation state of immune cells can be determined by microscopy and/or imaging. For example, when the immune cells, *e.g.,* macrophages and dendritic cells are activated, their morphology can change from round cells to extended cells with pseudopods. In addition, the activated immune cells (*e.g.,* activated macrophages and dendritic cells) can produce extracellular matrix to move and/or adhere more easily to the surrounding environment. During the inflammatory response, the activated immune cells (*e.g.,* activated macrophages) can fuse together to form giant cells, *e.g.,* to engulf larger materials. This cell morphology is characteristic of activated macrophages, which can be analyzed by imaging, *e.g.,* SEM imaging (see, *e.g.,* Examples 1-3). In some embodiments, the activation state of immune cells can be determined by detecting the presence of gene expression markers (*e.g.,* specific markers for M1 macrophages vs. markers for M2 macrophages) and/or cytokine release (*e.g.,* TNF-α released by M1 macrophages) of activated immune cells (see Examples 1-3).

In order to control or alter activation state of immune cells (*e.g.,* macrophages), the biomaterial (*e.g.,* a biomaterial comprising a silk fibroin-based matrix) contained in the composition described here comprises at least one immune cell-modulating agent, *e.g.,* at least one macrophage-skewing agent, which is described in detail below.

In some embodiments, at least one immune cell-modulating agent (*e.g.,* at least one macrophage-skewing agent) can be present in a biomaterial (*e.g.,* a silk fibroin-based matrix) in an effective amount sufficient to control or alter target immune cells (*e.g.,* macrophages and/or dendritic cells) with a likelihood that is at least about at least 2, 5, 10, 25, 50, 75, 100, 150, 200, 250, 500, 1000 or more times greater than the likelihood for non-target immune cells. In some embodiments, at least one immune cell-modulating agent *(e.g.,* at least one macrophage-skewing agent) can be present in a biomaterial (*e.g.,* a silk fibroin-based matrix) in an effective amount sufficient to control or alter or target immune cells (*e.g.,* macrophages and/or dendritic cells) with a potency that is at least about at least 2, 5, 10, 25, 50, 75, 100, 150, 200, 250, 500, 1000 or more times greater than the potency for non-target immune cells.

In some embodiments, at least one immune cell-modulating agent *(e.g.,* at least one macrophage-skewing agent) can be present in a biomaterial (*e.g.,* a silk fibroin-based matrix) in an effective amount sufficient to induce at least about 10% or more (including, *e.g.,* at least about 20%, at least about 30%, at least about 40%, at least about 50%, at least about 60%, at least about 70%, at least about 80%, at least about 90%, at least about 95%, at least about 98% or more) of macrophages in close proximity and/or at a site of a target stimulus to M1 phenotype. In some embodiments, at least one immune cell-modulating agent (*e.g.,* at least one macrophage-skewing agent) can be present in a biomaterial (*e.g.,* a silk fibroin-based matrix) in an effective amount sufficient to increase the number of M1 macrophages in close proximity and/or at a site of a target stimulus by at least about 10% or more (including, *e.g.,* at least about 20%, at least about 30%, at least about 40%, at least about 50%, at least about 60%, at least about 70%, at least about 80%, at least about 90%, at least about 95%, at least about 98% or more), as compared to the number of M1 macrophages in close proximity and/or at the site of the target stimulus when at least one immune cell-modulating agent is not administered or placed.

Without wishing to be bound by theory, as M1 macrophages are generally associated with induction of inflammatory responses, and/or degradation of the biomaterial (*e.g.,* the silk fibroin-based matrix). Accordingly, in some embodiments, at least one immune cell-modulating agent (*e.g.,* at least one macrophage-skewing agent) can be present in a biomaterial *(e.g.,* a silk fibroin-based matrix) in an effective amount sufficient to increase at least one inflammatory response (*e.g.,* release of at least one inflammatory factors such as TNF-α) in close proximity and/or at a site of a target stimulus by at least about 10% or more (including, *e.g.,* at least about 20%, at least about 30%, at least about 40%, at least about 50%, at least about 60%, at least about 70%, at least about 80%, at least about 90%, at least about 95%, at least about 98% or more), as compared to the degree of inflammatory responses observed in close proximity and/or at the site of the target stimulus when at least one immune cell-modulating agent is not administered or placed. In some embodiments, at least one immune cell-modulating agent (*e.g.,* at least one macrophage-skewing agent) can be present in a biomaterial (*e.g.,* a silk fibroin-based matrix) in an effective amount sufficient to increase degradation of the biomaterial described herein (*e.g.,* silk fibroin-based matrix) by at least about 10% or more (including, *e.g.,* at least about 20%, at least about 30%, at least about 40%, at least about 50%, at least about 60%, at least about 70%, at least about 80%, at least about 90%, at least about 95%, at least about 98% or more), as compared to the degradation of the biomaterial described herein (*e.g.,* silk fibroin-based matrix) comprising no immune cell-modulating agent (*e.g.,* no macrophage-skewing agent) .

In some embodiments, at least one immune cell-modulating agent (*e.g.,* at least one macrophage-skewing agent) can be present in a biomaterial (*e.g.,* a silk fibroin-based matrix) in an effective amount sufficient to induce at least about 10% or more (including, *e.g.,* at least about 20%, at least about 30%, at least about 40%, at least about 50%, at least about 60%, at least about 70%, at least about 80%, at least about 90%, at least about 95%, at least about 98% or more) of macrophages in close proximity and/or at a site of a target stimulus to M2 phenotype. In some embodiments, at least one immune cell-modulating agent (*e.g.,* at least one macrophage-skewing agent) can be present in a biomaterial (*e.g.,* a silk fibroin-based matrix) in an effective amount sufficient to increase the number of M2 macrophages in close proximity and/or at a site of a target stimulus by at least about 10% or more (including, *e.g.,* at least about 20%, at least about 30%, at least about 40%, at least about 50%, at least about 60%, at least about 70%, at least about 80%, at least about 90%, at least about 95%, at least about 98% or more), as compared to the number of M2 macrophages in close proximity and/or at the site of the target stimulus when at least one immune cell-modulating agent (*e.g.,* at least one macrophage-skewing agent) is not administered or placed.

Without wishing to be bound by theory, as M2 macrophages are generally associated with induction of anti-inflammatory and/or regenerative responses. Accordingly, in some embodiments, at least one immune cell-modulating agent (*e.g.,* at least one macrophage-skewing agent) can be present in a biomaterial (*e.g.,* a silk fibroin-based matrix) in an effective amount sufficient to increase at least one anti-inflammatory response (*e.g.,* release of at least one anti-inflammatory factors such as IL-10) in close proximity and/or at a site of a target stimulus by at least about 10% or more (including, *e.g.,* at least about 20%, at least about 30%, at least about 40%, at least about 50%, at least about 60%, at least about 70%, at least about 80%, at least about 90%, at least about 95%, at least about 98% or more), as compared to the degree of anti-inflammatory responses observed in close proximity and/or at the site of the target stimulus when at least one immune cell-modulating agent is not administered or placed. In some embodiments, at least one immune cell-modulating agent (*e.g.,* at least one macrophage-skewing agent) can be present in a biomaterial (*e.g.,* a silk fibroin-based matrix) in an effective amount sufficient to increase the number and/or proliferation of stromal cells (*e.g.,* tissue cells such as fibroblasts) at a site of a target stimulus (*e.g.,* site of tissue damage) by at least about 10% or more (including, *e.g.,* at least about 20%, at least about 30%, at least about 40%, at least about 50%, at least about 60%, at least about 70%, at least about 80%, at least about 90%, at least about 95%, at least about 98% or more), as compared to the number and/or proliferation of stromal cells at the site of the target stimulus (*e.g.,* site of tissue damage) in the absence of no immune cell-modulating agent.

In some embodiments, the biomaterial or the silk fibroin-based matrix can comprise or can be functionalized with at least two (including, *e.g.,* at least three, at least four, at least five or more) immune cell-modulating agents. In some embodiments, the biomaterial or the silk fibroin-based matrix can comprise or be functionalized with at least two (including, *e.g.,* at least three, at least four, at least five or more) macrophage-skewing agents. In these embodiments, the biomaterial or the silk fibroin-based matrix can be adapted to release a first immune cell-modulating agent (*e.g.,* a first macrophage-skewing agent) at a first time point and a second immune cell-modulating agent (*e.g.,* a second macrophage-skewing agent) at a second time point. In some embodiments, the first time point and the second time point are the same. In other embodiments, the first time point and the second time point are different, *e.g.,* by at least about 5 mins, at least about 15 mins, at least about 30 mins, at least 1 hour, at least about 3 hours, at least about 6 hours, at least about 12 hours, at least about 24 hours or more. In some embodiments, the biomaterial or the silk fibroin-based matrix can be adapted to delay the release of a second immune cell-modulating agent after the release of a first immune cell-modulating agent by at least about 1 day, at least about 3 days, at least about 1 week, at least about 2 weeks, at least about 3 weeks, at least about 1 month or more. In some embodiments, the biomaterial or the silk fibroin-based matrix can be adapted to delay the release of a second immune cell-modulating agent after the release of a first immune cell-modulating agent by at least about 1 day, at least about 3 days, at least about 1 week, at least about 2 weeks, at least about 3 weeks, at least about 1 month or more

As used herein, the term "functionalized" refers to a biomaterial that is provided with at least one immune cell-modulating agent (*e.g.,* at least one macrophage-skewing agent). The immune cell-modulating agent (*e.g.,* the macrophage-skewing agent) can be provided in the biomaterial by any means. In some embodiments of this aspect and other aspects described herein, at least one immune cell-modulating agent (*e.g.,* at least one macrophage-skewing agent) can be encapsulated into the biomaterial. For example, in some embodiments, at least one immune cell-modulating agent(s) (*e.g.,* the macrophage-skewing agent(s)) can be mixed into a biomaterial solution (*e.g.,* a silk fibroin solution) during processing, or perfused into the formed biomaterial (*e.g.,* a silk fibroin-based matrix). In some embodiments, at least one immune cell-modulating agent (*e.g.,* at least one macrophage-skewing agent) can be present on a surface of the biomaterial. In these embodiments, the immune cell-modulating agent(s) (*e.g.,* the macrophage-skewing agent(s)) can be coated on the biomaterial (*e.g.,* a silk fibroin-based matrix). In some embodiments, the immune cell-modulating agent(s) (*e.g.,* the macrophage-skewing agent(s)) can be covalently linked to the biomaterial (*e.g.,* a silk fibroin-based matrix). In some embodiments, biomaterial precursors (*e.g.,* silk fibroin) can be conjugated to or fused with the immune cell-modulating agent (*e.g.,* macrophage-skewing agent), *e.g.,* by genetic engineering, and the biomaterial precursors can then be used to form a biomaterial (*e.g.,* silk fibroin to form a silk fibroin-based matrix).

Distribution of immune cell-modulating agents (*e.g.,* macrophage-skewing agents) in the biomaterial or silk fibroin-based matrix can vary, depending on a number of factors, *e.g.,* but not limited to, number and/or types of immune cell-modulating agents (*e.g.,* macrophage-skewing agents), form of the matrix (*e.g.,* a film vs. particles), desired release profiles of the immune cell-modulating agent(s) (*e.g.,* macrophage-skewing agent(s)), applications of the compositions described herein, biomaterial functionalization, and any combinations thereof. Immune cell-modulating agents (*e.g.,* macrophage-skewing agents) can be dispersed homogenously or heterogeneously, or dispersed in a gradient in the biomaterial or silk fibroin-based matrix. Additional information about methods for loading at least one immune cell-modulating agent (*e.g.,* at least macrophage-skewing agent) into a silk fibroin-based matrix is further discussed below.

The biomaterial or silk fibroin-based matrix can form a homogenous matrix, a composite matrix (*e.g.,* a blend of two or more biomaterials), and/or a multi-layered matrix. In some embodiments, at least one or more immune cell-modulating agent (*e.g.,* at least two or more immune cell-modulating agents) can be distributed in the homogenous matrix. In some embodiments, at least one or more immune cell-modulating agent (*e.g.,* at least two or more immune cell-modulating agents) can be distributed in a composite matrix (*e.g.,* a blend of two or more biomaterials in a matrix). In these embodiments where two or more immune cell-modulating agents are involved, a first immune cell-modulating agent can have a higher solubility or partition coefficient in a first biomaterial than in a second biomaterial, and a second immune cell-modulating agent can have a higher solubility or partition coefficient in a second biomaterial than in a first biomaterial. Thus, the first immune cell-modulating agent can be preferentially distributed in the first biomaterial of the composite matrix and the second immune cell-modulating agent can be preferentially distributed in the second biomaterial of the composite matrix, forming a composite biomaterial comprising two or more immune cell-modulating agents distributed in different biomaterials.

In some embodiments, at least one or more immune cell-modulating agent (*e.g.,* at least two or more immune cell-modulating agents) can be distributed in a multi-layered biomaterial. In some embodiments, two or more immune cell-modulating agents (*e.g.,* macrophage-skewing agents) can be dispersed in separate layers of a multi-layered biomaterial or silk fibroin-based matrix. For example, a multi-layered biomaterial or silk fibroin-based matrix can comprise a first immune cell-modulating agent (*e.g.,* a first macrophage-skewing agent) in its first layer and a second immune cell-modulating agent (*e.g.,* a second macrophage-skewing agent) in its second layer. Methods for fabricating a multi-layered biomaterial are known in the art, *e.g.,* but not limited to, layer-by-layer deposition or dipping methods.

The biomaterial or silk fibroin-based matrix described herein can be present in any material format, *e.g.,* a film, a fiber, a particle, a tube, a gel, a microsphere, a hydrogel, a mat, a mesh, a fabric, or any combinations thereof. Any ratio of biomaterial (*e.g.,* silk fibroin) to an immune cell-modulating agent (*e.g.,* macrophage-skewing agent) can be used. In various embodiments, the ratio of a biomaterial (*e.g.,* silk fibroin) to an immune cell-modulating agent (*e.g.,* macrophage-skewing agent) can be about 1:1000 to about 1000: 1, about 1:500 to about 500: 1, about 1: 250 to about 250:1, about 1: 125 to about 125: 1, about 1: 100 to about 100: 1, about 1: 50 to about 50: 1, about 1: 25 to about 25:1, about 1:10 to about 10:1, about 1:5 to about 5:1, about 1:3 to about 3: 1, or about 1:1. The ratio of a biomaterial (*e.g.,* silk fibroin) to an immune cell-modulating agent (*e.g.,* macrophage-skewing agent) can vary with a number of factors, including the selection of an immune cell-modulating agent (*e.g.,* macrophage-skewing agent), the storage condition and duration, the concentration of the biomaterial (*e.g.,* silk fibroin-based matrix) and/or the format of the biomaterial (*e.g.,* silk matrix). One of skill in the art can determine appropriate ratio of the biomaterial (*e.g.,* silk fibroin-based matrix) to the immune cell-modulating agent (*e.g.,* macrophage-skewing agent), *e.g.,* by determining effects of the immune cell modulating agent (*e.g.,* macrophage-skewing agent) at various concentrations on activation of the immune cells of interest (*e.g.,* macrophages), for example as described in Examples 1-3. Methods for determining activation of immune cells, *e.g.,* by microscopy and imaging, gene expression and/or cytokine release analyses, are well known in the art.

In some embodiments of this aspect and other aspects described herein, the biomaterial or the silk fibroin-based matrix can be adapted for a sustained release of said at least one immune cell-modulating agent (*e.g.,* at least one macrophage-skewing agent). In some embodiments, the biomaterial or the silk fibroin-based matrix can be adapted for a sustained release of at least two or more immune cell-modulating agents (*e.g.,* at least two or more macrophage-skewing agents). As used herein, the term "sustained release" refers to delivery of at least one immune cell-modulating agent (*e.g.,* at least one macrophage-skewing agent) from the biomaterial or silk fibroin-based matrix for a period of time, *e.g.,* at least about 1 week, at least about 2 weeks, at least about 3 weeks, at least about 4 weeks, at least about 5 weeks, at least about 6 weeks, at least about 7 weeks, at least about 8 weeks, at least about 12 weeks, at least about 4 months, at least about 5 months, at least about 6 months, at least about 9 months, at least about 12 months or longer.

The immune cell-modulating agent(s) (*e.g.,* macrophage-skewing agent(s)) can be released, continuously or intermittently, from the biomaterial or silk fibroin-based matrix for a period of time, *e.g.,* for a period of hours, days, weeks, or months. In some embodiments, the immune cell-modulating agent(s) (*e.g.,* macrophage-skewing agent(s)) can be released at a rate at which at least about 1% (including at least about 5%, at least about 10%, at least about 20%, at least about 30%, at least about 40%, at least about 50%, at least about 60%, at least about 70%, at least about 80%, at least about 90%, at least about 95%, or more) of the total loading can be released over a period of at least 1 hour, at least 2 hours, at least 3 hours, at least about 4 hours, at least about 5 hours, at least about 6 hours, at least about 12 hours, at least about 24 hours or longer. In some embodiments, the immune cell-modulating agent(s) (*e.g.,* macrophage-skewing agent(s)) can be released at a rate at which at least about 10% (including at least about 20%, at least about 30%, at least about 40%, at least about 50%, at least about 60%, at least about 70%, at least about 80%, at least about 90%, at least about 95%, or more) of the total loading can be released over a period of 5 days, a period of 1 week, at least about 2 weeks, at least about 3 weeks, at least about 1 month, at least about 2 months, at least about 3 months or longer.

Selection of immune cell-modulating agent(s) (*e.g.,* macrophage-skewing agent(s)) can be determined, *e.g.,* depending on types of immune cells to be targeted, intended immune responses (*e.g.,* inflammatory responses vs. regenerative responses) and/or applications of the compositions described herein. In some embodiments, at least one immune cell-modulating agent (*e.g.,* at least one macrophage-skewing agent) can be selected to control response of at least one type (including, *e.g.,* at least two types or more) of immune cells, *e.g.,* macrophages. In some embodiments, at least one immune cell modulating agent (*e.g.,* at least one macrophage-skewing agent) can be selected to control response of at least one type (including, *e.g.,* two or more types) of immune cells (*e.g.,* macrophages) to a target stimulus *in vitro* or *in vivo.* In one embodiment, at least one immune cell-modulating agent (*e.g.,* at least one macrophage-skewing agent) can be selected to control response of at least one type (including, *e.g.,* at two types or more) of immune cells (*e.g.,* macrophages) to a target stimulus in a subject.

As further described in the section *"Target stimuli"* below, a target stimulus can comprise any *in vitro* or *in vivo* condition, object, and/or matter that can cause or induce immune cells (*e.g.,* macrophages) to respond or react, *e.g.,* inducing inflammatory responses and/or regenerative responses. In some embodiments, a target stimulus can comprise a macrophage-associated condition. Exemplary macrophage-associated conditions can include, but are not limited to, bacterial infection, tissue regeneration, tissue damage, tissue reconstruction, including, e.g., tissue repair and/or augmentation (e.g., soft tissue repair and/or augmentation), arthritis, obesity, diabetes, arteriosclerosis, allograft transplantation, Langerhans cell histiocytosis (LCH), osteoporosis, glomerulonephritis, cancer, and any combinations thereof. In some embodiments, the target stimulus can comprise an implantable structure (*e.g.,* but not limited to, scaffolds, allograft tissues, medical devices). In some embodiments, the target stimulus can comprise at least one cytokine (*e.g.,* including a chemokine).

In some embodiments, at least one immune cell-modulating agent (*e.g.,* at least one macrophage-skewing agent) can be selected to induce presence of M1 phenotypic macrophages. In some embodiments, at least one immune cell-modulating agent (*e.g.,* at least one macrophage-skewing agent) can be selected to induce presence of M2 phenotypic macrophages. In some embodiments, at least one immune cell-modulating agent (*e.g.,* at least one macrophage-skewing agent) can be selected to induce at least one inflammatory response *(*e*.g.,* but not limited to, release of inflammatory factors such as TNF-α). In some embodiments, at least one immune cell-modulating agent (*e.g.,* at least one macrophage-skewing agent) can be selected to induce degradation of the biomaterial described herein (*e.g.,* a silk fibroin-based matrix) upon placement in close proximity and/or at a target site. In some embodiments, at least one immune cell-modulating agent (*e.g.,* at least one macrophage-skewing agent) can be selected to induce at least one anti-inflammatory response (*e.g.,* but not limited to, release of anti-inflammatory factors such as IL-10). In some embodiments, at least one immune cell-modulating agent (*e.g.,* at least one macrophage-skewing agent) can be selected to induce proliferation and/or the number of stromal cells (*e.g.,* parenchymal cells) at a target site (*e.g.,* a damaged tissue).

The compositions and/or the biomaterials (*e.g.,* silk fibroin-based matrices) described herein can be adapted for various applications. For example, the composition can be formulated for use in treatment of the macrophage-associated condition described herein. Here, the composition can further comprise at least one therapeutic agent for treatment of the macrophage-associated condition described herein, *e.g.,* but not limited to, tissue regeneration and/or wound healing. Exemplary therapeutic agents for tissue regeneration and/or wound healing can include, but are not limited to, dexpanthenol; growth factors; enzymes, hormones; povidon-iodide; fatty acids; anti-inflammatory agents; antibiotics; antimicrobials; antiseptics; cytokines; thrombin; analgesics; opioids; aminoxyls; furoxans; nitrosothiols; nitrates and anthocyanins; nucleosides, such as adenosine; and nucleotides, such as adenosine diphosphate (ADP) and adenosine triphosphate (ATP); neutotransmitter/ neuromodulators, such as acetylcholine and 5-hydroxytryptamine (serotonin/5-HT); histamine and catecholamines, such as adrenalin and noradrenalin; lipid molecules, such as sphingosine-1-phosphate and lysophosphatidic acid; amino acids, such as arginine and lysine; peptides such as the bradykinins, substance P and calcium gene-related peptide (CGRP); nitric oxide; and any combinations thereof.

Exemplary growth factors include, but are not limited to, fibroblast growth factor (FGF), FGF-1, FGF-2, FGF-4, FGF-α, FGF- β, platelet-derived growth factor (PDGF), insulin-binding growth factor (IGF), IGF-1, IGF-2, heparin-binding growth factor-1, heparin-binding growth factor-2, epidermal growth factor (EGF), transforming growth factor (TGF), TGF-α, TGF-β, cartilage inducing factors-A and -B, osteoid-inducing factors, osteogenin, vascular endothelial growth factor, bone growth factors, collagen growth factors, insulin-like growth factors, and their biologically active derivatives.

In some embodiments, the composition can be formulated for topical application, for direct application, for injection, for implantation, or any other suitable form of administration.

The composition comprising a biomaterial functionalized with at least one immune cell-modulating agent (*e.g.,* at least one macrophage-skewing agent) can be used in the manufacture of a pharmaceutical composition. The composition comprising a biomaterial functionalized with a macrophage-skewing agent can be used in the manufacture of a pharmaceutical composition additionally comprising a pharmaceutically acceptable carrier.

In some embodiments, the composition or the biomaterial can be adapted for use as a wound dressing, or as part of wound dressings, *e.g.,* but not limited to, bandage, gauzes, tapes, meshes, nets, adhesive plasters, films, membranes, patches, or any combinations thereof. In some embodiments, the composition can further comprise a therapeutic agent to facilitate wound healing as described above.

In some embodiments, the composition or the biomaterial can be adapted for use as a coating. For example, in some embodiments, the composition can be adapted to form a coating of an implantable structure, *e.g.,* but not limited to, a scaffold, an allograft tissue, and/or a medical device, *e.g.,* but not limited to, stents, ports, screws, catheters, sutures, staples, artificial organs, neurostimulators, pumps, drug delivery pumps, pacemakers, defibrillators, stent-grafts, grafts, artificial heart valves, foramen ovale closure devices, cerebrospinal fluid shunts, pacemaker electrodes, guide wires, ventricular assist devices, cardiopulmonary bypass circuits, blood oxygenators, vena cava filters, endocardial leads, endotracheal tubes, nephrostomy tube and orthopedic devices. In some embodiments, the composition can be used to coat a pharmaceutical composition, or a drug delivery vehicle. In some embodiments, the composition can be used to form a drug delivery vehicle.

In some embodiments, the composition or the biomaterial can be adapted to form a scaffolding structure (e.g., a prosthetic device) for use, for example, in reconstructive surgery (including, e.g., but not limited to, cosmetic surgery, and soft tissue repair and/or augmentation). In one embodiment, the composition or the biomaterial can form an injectable scaffold. For example, injectable silk fibroin particles, e.g., described in the International Application No. PCT/US12/64450 filed November 9, 2012, and injectable silk fibroin foams, e.g., described in the International Application No. PCT/US12/64471 filed November 9, 2012, can be employed in the compositions and methods described herein. In one embodiment, the composition or the biomaterial can form a knitted scaffolding structure (e.g., a knitted prosthetic device). For example, silk fibroin-based meshes and/or fabrics, e.g., described in the U.S. Pat. Appl. Nos. US 2012/0221105 and US 2012/0304702, can be employed to form a scaffolding structure (e.g., a prosthetic device).

Accordingly, depending on the applications and/or application formats, the biomaterial (*e.g.,* silk fibroin-based matrix) can exist in any form. In some embodiments, the biomaterial can be in a form of a film, a fiber, a collection of particles, a tube, a mat, a gel, a fabric, a mesh, or any combinations thereof. In some embodiments, the biomaterial comprising a silk fibroin-based matrix can be in a form of a film, a fiber, a collection of particles, a tube, a mat, a gel, a fabric, a mesh, or any combinations thereof. For example, to form a coating of an implantable structure, the composition or the biomaterial can form a film. In other embodiments where the composition is used as a scaffold at a target site (*e.g.,* a damaged tissue site), the composition and/or the biomaterial can form a gel, a tube, a collection of particles, or a 3-D structure suited to the shape and/or size of the target site (*e.g.,* a damaged tissue site).

In some embodiments, the composition comprising a biomaterial functionalized with at least one immune cell-modulating agent (*e.g.,* at least one macrophage-skewing agent) can comprise a coating. A coating can be in the form of a film, a fiber, a collection of particles, a gel, a mesh, a fabric or any combinations thereof. In some embodiments, the coating can be applied to a silk fibroin-based matrix, e.g., but not limited to, a silk fibroin-based hydrogel, a silk fibroin-based implant, a silk fibroin-based fabric or fiber, a silk fibroin-based mesh or other tissue engineering compositions as discussed in U.S. Patent Publications 2011/0189773; 2011/0052695; 2011/0009960; 2010/0256756; and 2010/0209405.

In some embodiments, the composition comprising a biomaterial functionalized with at least one immune cell-modulating agent (*e.g.,* at least one macrophage-skewing agent) can comprises a drug delivery vehicle. In some embodiments, the composition can comprise a controlled-release pharmaceutical composition.

In some embodiments, the compositions described herein can be adapted to be injectable. As used herein, the term "injectable" generally refers to a composition capable of being placed or administered into a target site with a minimally invasive procedure. The term "minimally invasive procedure" refers to a procedure that is carried out by entering a subject's body through the skin or through a body cavity or an anatomical opening, but with the smallest damage possible (*e.g.,* a small incision, injection). In some embodiments, the injectable composition can be administered or placed into a target site by injection. In some embodiments, the injectable composition can be administered or placed through a small incision on the skin followed by insertion of a needle, a cannula, and/or tubing, *e.g.,* a catheter. Without wishing to be limited, the injectable composition can be administered or placed into a tissue by surgery, *e.g.,* implantation.

Without wishing to be limiting, at least one immune cell-modulating agent (including, *e.g.,* macrophage-skewing agent) can be also distributed in some embodiments of the compositions described herein.

### Methods, e.g., for controlling immune response in vitro or in vivo

The compositions described herein can be used to control immune response, *e.g.,* macrophage response *in vitro* or *in vivo.* Accordingly, described herein are methods, *e.g.,* for controlling response of immune cells to a target stimulus described herein. Exemplary immune cells can include, but are not limited to, monocytes, macrophages, dendritic cells, megakaryocytes, granulocytes, T cells, B cells, natural killer (NK) cells, and any combinations thereof.

IThe method can comprise: (a) placing in close proximity to or at a target site one or more embodiments of the compositions described herein; and (b) releasing the immune cell-modulating agent at a pre-determined rate from the biomaterial of the composition described herein, upon the placement at the target site, to alter the ratio of activated immune cells to inactivated immune cells surrounding the biomaterial. When the ratio of activated immune cells to inactivated immune cells reaches above a threshold, the biomaterial described herein can induce an inflammatory response; and when the ratio of activated immune cells to inactivated immune cells reaches below a threshold, the biomaterial described herein can induce a regenerative or anti-inflammatory response.

As used herein, the term "close proximity" generally refers to the spatial distance of a placement or administration site of the composition described herein from a target site (*e.g.,* site of a target stimulus) no more than 10 cm, including, *e.g.,* no more than 9 cm, no more than 8 cm, no more than 7 cm, no more than 6 cm, no more than 5 cm, no more than 4 cm, no more than 3 cm, no more than 2 cm, no more than 1 cm, no more than 0.5 cm, no more than 0.1 cm or less.

In some embodiments, the at least one immune cell-modulating agent can be present in an effective amount sufficient to selectively alter activation state of at least dendritic cells.

In some embodiments, the at least one immune cell-modulating agent can be present in an effective amount sufficient to selectively alter activation state of at least macrophages. In these embodiments, the at least one immune cell-modulating agent can comprise at least one macrophage-skewing agent. Thus, in particular embodiments, methods, *e.g.,* for controlling response of at least macrophages, are also provided herein. In some embodiments, the method can comprise (a) placing in close proximity to or at a target site one or more embodiments of the compositions described herein; and (b) releasing the macrophage-skewing agent at a pre-determined rate from the biomaterial, upon the placement at the target site, to alter the ratio of M1 macrophages to M2 macrophages surrounding the biomaterial. When the ratio of M1 macrophages to M2 macrophages is above a threshold, the biomaterial described herein can induce an inflammatory response; and when the ratio of M1 macrophages to M2 macrophages is below a threshold, the biomaterial described herein can induce a regenerative or anti-inflammatory response.

In various embodiments where the ratio of M1 macrophages to M2 macrophages is above a threshold, the inflammatory response induced by a biomaterial (*e.g.,* a silk fibroin-based matrix) described herein can comprise inducing degradation of the biomaterial (*e.g.,* the silk fibroin-based matrix). As used in reference to a biomaterial (*e.g.,* a silk fibroin-based matrix) described herein, the term "degrade" or "degradation" refers to a decrease in volume or size of a biomaterial (*e.g.,* a silk fibroin-based matrix). The degradation of the biomaterial *(e.g.,* silk fibroin-based matrix) can occur via cleavage of the biomaterial (*e.g.,* silk fibroin-based matrix) into smaller fragments and/or dissolution of the biomaterial (*e.g.,* silk fibroin-based matrix) or fragments thereof. In some embodiments, the immune cell-modulating agent (*e.g.,* the macrophage-skewing agent) can be released at a predetermined rate, for example, to achieve an optimum ratio of M1 macrophages to M2 macrophages surrounding the biomaterial, such that the biomaterial (*e.g.,* silk fibroin-based matrix) can degrade no more than 80% of its original volume (*e.g.,* volume prior to placement or implantation at a target site), including, for example, no more than 70%, no more than 60%, no more than 50%, no more than 40%, no more than 30%, no more than 20%, no more than 10% of its original volume or lower, upon placement and/or implantation of the biomaterial (*e.g.,* the silk fibroin-based matrix) for a period of time. In some embodiments, the immune cell-modulating agent (*e.g.,* the macrophage-skewing agent) can be released at a predetermined rate, for example, to achieve an optimum ratio of M1 macrophages to M2 macrophages surrounding the biomaterial, such that the biomaterial (*e.g.,* silk fibroin-based matrix) can exhibit no significant degradation (*e.g.,* no detectable changes in the volume) upon placement and/or implantation of the biomaterial (*e.g.,* the silk fibroin-based matrix).

In some embodiments, the immune cell-modulating agent (*e.g.,* the macrophage-skewing agent) can be released at a predetermined rate, for example, to achieve an optimum ratio of M1 macrophages to M2 macrophages surrounding the biomaterial, such that the biomaterial (*e.g.,* silk fibroin-based matrix) can be adapted to degrade at least a portion of its original volume over any period of time, *e.g.,* weeks, months, or years. In some embodiments, the immune cell-modulating agent (*e.g.,* the macrophage-skewing agent) can be released at a predetermined rate, for example, to achieve an optimum ratio of M1 macrophages to M2 macrophages surrounding the biomaterial, such that the biomaterial (*e.g.,* silk fibroin-based matrix) can be adapted to degrade at least a portion of its original volume, *e.g.,* no more than 50% of its original volume (including *e.g.,* no more than 40%, no more than 30%, no more than 20% or lower, of its original volume), in at least about 2 weeks, at least about 3 weeks, at least about 4 weeks, at least about 5 weeks, at least about 6 weeks, at least about 7 weeks, at least about 8 weeks, at least about 3 months, at least about 4 months, at least about 5 months, at least about 6 months, at least about 7 months, at least about 8 months, at least about 9 months, at least about 10 months, at least about 11 months, at least about 1 year, at least about 2 years, at least about 3 years, at least about 4 years, at least about 5 years or longer. In other embodiments, the immune cell-modulating agent (*e.g.,* the macrophage-skewing agent) can be released at a predetermined rate, for example, to achieve an optimum ratio of M1 macrophages to M2 macrophages surrounding the biomaterial, such that there can be no significant degradation (i.e., no detectable changes in the volume of the biomaterial (*e.g.,* silk fibroin-based matrix)) upon placement or implantation for at least about 3 months or longer.

In some embodiments, the immune cell-modulating agent (*e.g.,* the macrophage-skewing agent) can be released at a predetermined rate, for example, to achieve an optimum ratio of M1 macrophages to M2 macrophages surrounding the biomaterial, such that the original volume of the biomaterial (e*.g.,* silk fibroin-based matrix) gradually decreases (while still providing sufficient support) as the tissue at the target site placed with the biomaterial (*e.g.,* silk fibroin-based matrix) begins to regenerate. In such embodiments, the compositions described herein can comprise a first immune cell modulating agent (*e.g.,* a first macrophage-skewing agent) and a second immune cell modulating agent (*e.g.,* a second macrophage-skewing agent). In these embodiments, the first immune cell-modulating agent (*e.g.,* a first macrophage-skewing agent) can be released at a first predetermined rate, for example, to achieve an optimum ratio of M1 macrophages to M2 macrophages surrounding the biomaterial, in order to produce a regenerative response (*e.g.,* to increase proliferation of cells such as parenchymal cells at a target site, *e.g.,* comprising a damaged tissue). As the tissue begins to regenerate, the second immune cell-modulating agent (*e.g.,* the macrophage-skewing agent) can be released at a predetermined rate, for example, to achieve an optimum ratio of M1 macrophages to M2 macrophages surrounding the biomaterial, such that the biomaterial (*e.g.,* silk fibroin-based matrix) can be adapted to degrade at least about 5% of its original volume, for example, including at least about 10%, at least about 20%, at least about 30%, at least about 40%, at least about 50%, at least about 60%, at least about 70%, at least about 80%, at least about 90%, at least about 95% or more, of its original volume over a pre-determined period of time (*e.g.,* a period of at least about 2 weeks, including at least about 6 weeks, at least about 3 months, at least about 6 months or longer). For example, the first immune cell-modulating agent (*e.g.,* the first macrophage-skewing agent) and the second immune cell-modulating agent (*e.g.,* the second macrophage-skewing agent) can be distributed in different layers of a biomaterial (*e.g.,* a silk fibroin-based matrix) of the compositions described herein, where the corresponding layer of the biomaterial (*e.g.,* silk fibroin-based matrix) is adapted to release the respective cell-modulating agent at a pre-determined rate, *e.g.,* by altering an amount of β-sheet in silk fibroin and/or porosity of the silk fibroin-based matrix, and/or modifying the layer, *e.g.,* by coating the layer with a polymeric material.

In some embodiments where the ratio of M1 macrophages to M2 macrophages is above a threshold, the inflammatory response induced by a biomaterial (*e.g.,* a silk fibroin-based matrix) described herein can comprise protecting tissue in close proximity and/or at the target site from infection (*e.g.,* bacterial infection). In some embodiments, the immune cell-modulating agent(s) released from the biomaterial can induce or activate target immune cells to release a sufficient amount of an antimicrobial agent such as antimicrobial peptides, in order to reduce or prevent tissue in close proximity and/or at the target site from infection (*e.g.,* bacterial infection). Thus, in some embodiments, the methods described herein can be used to treat an infection (*e.g.,* a bacterial infection).

In various embodiments where the ratio of M1 macrophages to M2 macrophages is below a threshold, the regenerative or anti-inflammatory response induced by a biomaterial (*e.g.,* a silk fibroin-based matrix) described herein can comprise facilitating tissue repair and/or regeneration at the target site. In some embodiments, the immune cell-modulating agent(s) released from the biomaterial can induce or activate target immune cells to release a sufficient amount of proliferative factors such as growth factors (*e.g.,* but not limited to, TGF-β), in order to stimulate growth and/or proliferation of tissue cells in close proximity and/or at the target site (*e.g.,* damaged tissue). Thus, in some embodiments, the methods described herein can be used to treat a tissue damage.

The terms "treatment" and "treat" as used herein, with respect to treatment of a condition, *e.g.,* an infection or a tissue damage described herein, means preventing the progression of the condition, or altering the course of the condition (for example, but are not limited to, slowing the progression or worsening of the condition), or reversing a symptom of the condition (*e.g.,* slowing and/or reversing bacterial infection or tissue damage) or reducing one or more symptoms and/or one or more biochemical markers in a subject, preventing one or more symptoms from worsening or progressing, promoting recovery or improving prognosis. For example, in the case of treating a bacterial infection, therapeutic treatment refers to induction of at least one inflammatory response, *e.g.,* release of an inflammatory agent (*e.g.,* TNF-α) and/or antimicrobial agent (*e.g.,* antimicrobial peptide), after administration of the composition described herein. In another embodiment, the therapeutic treatment refers to alleviation of at least one symptom associated with a bacterial infection. Measurable lessening includes any statistically significant decline in a measurable marker or symptom, such as a fever (having a body temperature of over 100 degrees Fahrenheit) subsiding after treatment. In one embodiment, at least one symptom of a bacterial infection is alleviated by about 10%, about 15%, about 20%, about 30%, about 40%, or about 50%, as compared to a control (*e.g.,* in the absence of the composition described herein). In another embodiment, at least one symptom is alleviated by more than 50%, *e.g.,* about 60%, or about 70%, as compared to a control (*e.g.,* in the absence of the composition described herein). In one embodiment, at least one symptom is alleviated by about 80%, or about 90%, as compared to a control (*e.g.,* in the absence of the composition described herein). In the case of treating a tissue damage, therapeutic treatment refers to induction of at least one regenerative response, *e.g.,* release of an anti-inflammatory agent (*e.g.,* IL-10) and/or a proliferative factor (*e.g.,* TGF-β) after administration of the composition described herein. In another embodiment, the therapeutic treatment refers to alleviation of at least one symptom associated with a tissue damage. Measurable lessening includes any statistically significant decline in a measurable marker or symptom, such as pain reduction after treatment. In one embodiment, at least one symptom of a tissue damage is alleviated by about 10%, about 15%, about 20%, about 30%, about 40%, or about 50%, as compared to a control (*e.g.,* in the absence of the composition described herein). In another embodiment, at least one symptom is alleviated by more than 50%, *e.g.,* about 60%, or about 70%, as compared to a control (*e.g.,* in the absence of the composition described herein). In one embodiment, at least one symptom is alleviated by about 80%, or about 90%, as compared to a control (*e.g.,* in the absence of the composition described herein).

In some embodiments where the ratio of M1 macrophages to M2 macrophages is below a threshold, the regenerative or anti-inflammatory response induced by a biomaterial (*e.g.,* a silk fibroin-based matrix) described herein can comprise reducing rejection of the biomaterial by a host's immune system.

In some embodiments of various methods described herein, the target site to be treated can comprise a target stimulus described herein to which the response of immune cells (*e.g.,* macrophages) is to be controlled. The target site to be treated can be present *in vitro* or *in vivo.* In some embodiments, provided herein is the method for controlling macrophage response to a target stimulus in a subject. The method comprises placing in close proximity to or at the site of a target stimulus one or more embodiments described herein, wherein at least one macrophage-skewing agent described herein is present in a biomaterial (*e.g.,* a biomaterial comprising a silk fibroin-based matrix) in an effective amount sufficient to (a) switch at least a population of macrophages to M1 phenotypic state, thereby inducing an inflammatory response to the target stimulus; (b) switch at least a population of macrophages to M2 phenotypic state, thereby inducing a regenerative response to the stimulus; or (c) both (a) and (b). In some embodiments, the biomaterial can comprise a silk fibroin-based matrix.

In various methods described herein, the composition comprising the biomaterial (*e.g.,* a biomaterial comprising a silk fibroin-based matrix) can be placed in close proximity or at the target site by any methods known in the art. In some cases, the composition comprising the biomaterial (*e.g.,* silk fibroin-based matrix) can be placed by injection. In some cases, the composition comprising the biomaterial (*e.g.,* silk fibroin-based matrix) can be placed by implantation, *e.g.,* by surgery.

The methods described herein can be used in any situations where control of an immune response is desirable, e.g., a situation comprising a target stimulus described herein. By way of example only, the methods described herein can be used to facilitate wound healing. Here, the method can comprise contacting a wound (e.g., a surgical wound or open wound) with the compositions described herein (e.g., comprising a silk fibroin-based mat or mesh, and/or an injectable silk fibroin-based matrix described herein); and releasing at least one immune cell-modulating agent (e.g., at least one macrophage-skewing agent) at a pre-determined rate from the biomaterial (e.g., silk fibroin-based mat or mesh, and/or injectable silk fibroin-based matrix described herein) to the wound, upon the contact, in order to optimize the immune response in close proximity or at the wound and thus improve wound healing process.

The methods described herein can be used to facilitate tissue reconstruction (e.g., tissue repair and/or augmentation). Tissue reconstruction can include, but are not limited to, hernia repair, pelvic floor reconstruction, periurethral support, repair and/or augmentation of, e.g., urinary bladder tissues and slings, peritoneal wall tissues, vessels (e.g., arteries), muscle tissue (abdominal smooth muscle, cardiac), hemostats, cartilage, bone, skin, and ligaments and tendons of the knee and/or shoulder as well as other frequently damaged structures due to trauma or chronic wear, and any combinations thereof. In some embodiments, the methods described herein can be used to facilitate reconstruction (e.g., repair and/or augmentation) of a soft tissue. Examples of soft tissues can include, without limitations, a skin, a breast tissue, tendon, a ligament, a fibrous tissue, a connective tissue, a muscle, and any combinations thereof. In one embodiment, the method described herein can be used to reconstruct, augment or support a breast tissue or a breast implant in a subject. In this embodiment, the method can comprise placing one or more embodiments of the compositions described herein (e.g., comprising a silk fibroin-based mesh or fabric, and/or an injectable silk fibroin-based matrix) into a breast tissue of a subject; and releasing at least one immune cell-modulating agent (e.g., at least one macrophage-skewing agent) at a pre-determined rate from the biomaterial (e.g., the silk fibroin-based mesh or fabric, and/or the injectable silk fibroin-based matrix) to a target site of the breast tissue, upon the placement, in order to optimize the immune response in close proximity or at the target site and thus improve tissue reconstruction process, e.g., but not limited to, reducing inflammatory response, and/or scar formation, and/or enhancing tissue regeneration. Methods for using a silk fibroin-based mesh or fabric in a variety of reconstructive or support applications, e.g., described in U.S. Pat. Appl. No. US 2012/0221105, can be adapted and used in the methods described herein.

The methods described herein can be adapted for use in cosmetic surgery procedures, including, e.g., but not limited to, breast enhancement, mastopexy, face lift, forehead lift, upper and lower eyelid surgery (blepharoplasty), nose reshaping (rhinoplasty), nasal reconstruction, dermal tissue augmentation; filling of lines, folds, wrinkles, minor facial depressions, and cleft lips, especially in the face and neck; correction of minor deformities due to aging or disease, including in the hands and feet, fingers and toes; augmentation of the vocal cords or glottis to rehabilitate speech; dermal filling of sleep lines and expression lines; replacement of dermal and subcutaneous tissue lost due to aging; lip augmentation; filling of crow's feet and the orbital groove around the eye; chin augmentation; augmentation of the cheek and/or nose; filling of indentations in the soft tissue, dermal or subcutaneous, due to, e.g., overzealous liposuction or other trauma; filling of acne or traumatic scars; filling of nasolabial lines, nasoglabellar lines and intraoral lines, and any combinations thereof.

The methods described herein can be used to improve skin appearance and/or condition, e.g., by reducing wrinkles, and/or increasing skin elasticity. Here, the method can comprise injecting the compositions described herein (e.g., an injectable silk fibroin-based composition) into a dermal region in need thereof of a subject, and releasing at least one immune cell-modulating agent (e.g., at least one macrophage-skewing agent) at a pre-determined rate from the biomaterial (e.g., the injectable silk fibroin-based matrix) to a target site of dermal region, upon the injection, in order to optimize the immune response in close proximity or at the target site. Thus, the method can reduce inflammation at the target site after injection. Alternatively or additionally, the method can protect the injection site from infection and/or bacterial infection.

### Biomaterials (e.g., silk fibroin-based matrices)

Described herein are methods and compositions, *e.g.,* for controlling an immune response, directed to biomaterials functionalized with at least one immune cell-modulating agent (*e.g.,* at least one macrophage-skewing agent) described herein. A biomaterial for use in the methods and compositions described herein can comprise one or more individual biomaterials. For example, the biomaterial can comprise a single biomaterial, *e.g.,* silk or two or more biomaterials, *e.g.,* silk and collagen. Biomaterials are well-known in the art. For example, any biocompatible material can be employed as a biomaterial in the compositions described herein. Examples of biocompatible materials can include, but are not limited to, polymers, hydrogels, proteins, and any combinations thereof.

As used herein, the term "biocompatible material" refers to any material that does not deteriorate appreciably and does not induce a significant immune response or deleterious tissue reaction, *e.g.,* toxic reaction or significant irritation, over time when implanted into or placed adjacent to the biological tissue of a subject, or induce blood clotting or coagulation when it comes in contact with blood. In some embodiments, a biocompatible material does not induce an acute or chronic inflammatory response or prevent proper differentiation of surrounding tissues. Suitable biocompatible materials can include, but are not limited to, silk fibroin, derivatives and copolymers of polyimides, polyvinyl alcohol, polyethyleneimine, polyvinylamine, polyacrylates, polyamides, polyesters, polycarbonates, polydimethylsiloxane, polyimide, polyethylene terephthalate, polymethylmethacrylate, polyurethane, polyvinylchloride, polystyrene, polysulfone, polycarbonate, polymethylpentene, polypropylene, a polyvinylidine fluoride, polysilicon, polytetrafluoroethylene, polysulfone, acrylonitrile butadiene styrene, polyacrylonitrile, polybutadiene, poly(butylene terephthalate), poly(ether sulfone), poly(ether ether ketones), poly(ethylene glycol), styrene-acrylonitrile resin, poly(trimethylene terephthalate), polyvinyl butyral, polyvinylidenedifluoride, poly(vinyl pyrrolidone).

The biomaterial can be biodegradable. In some cases, the biomaterial can comprise a biodegradable material, *e.g.,* a biodegradable polymer. As used herein, the term "biodegradable" describes a material which can decompose under physiological conditions into breakdown products. Such physiological conditions include, for example, hydrolysis (decomposition via hydrolytic cleavage), enzymatic catalysis (enzymatic degradation), and mechanical interactions. As used herein, the term "biodegradable" also encompasses the term "bioresorbable", which describes a substance that decomposes under physiological conditions to break down to products that undergo bioresorption into the host-organism, namely, become metabolites of the biochemical systems of the host organism.

The term "biodegradable polymer", as used herein, refers to a polymer that at least a portion thereof decomposes under physiological conditions. The polymer can thus be partially decomposed or fully decomposed under physiological conditions. Exemplary biodegradable polymers can include, but are not limited to, silk; silk fibroin glycosaminoglycan; fibrin; collagen; cross-linked collagen; polylactic acid; bone; poly-ethyleneglycol (PEG); C2 to C4 polyalkylene glycols (*e.g.,* propylene glycol); polyhydroxy ethyl methacrylate; polyvinyl alcohol; polyacrylamide; poly (N-vinyl pyrolidone); poly glycolic acid (PGA); poly lactic-co-glycolic acid (PLGA); poly e-carpolactone (PCL); polyethylene oxide; poly propylene fumarate (PPF); poly acrylic acid (PAA); hydrolysed polyacrylonitrile; polymethacrylic acid; polyethylene amine; polyanhydrides; polyhydroxybutyric acid; polyorthoesters; polysiloxanes; polycaprolactone; poly(lactic acid); poly(glycolic acid); alginic acid; esters of alginic acid; pectinic acid; esters of pectinic acid; carboxy methyl cellulose; hyaluronic acid; esters of hyaluronic acid; heparin; heparin sulfate; chitosan; carboxymethyl chitosan; chitin; pullulan; gellan; xanthan; collagen; carboxymethyl starch; carboxymethyl dextran; chondroitin sulfate; cationic guar; cationic starch as well as salts and esters thereof.

The biomaterial can comprise a silk fibroin-based matrix.

Accordingly, a composition comprising a silk-based matrix (*e.g.,* a silk fibroin-based matrix) comprising or functionalized with at least one immune cell-modulating agent (*e.g.,* macrophage-skewing agents) is also provided herein. The at least one immune cell-modulating agent can be present in the silk fibroin-based matrix in an effective amount sufficient to selectively control or alter activation state of immune cells upon contact with said at least one immune cell-modulating agent.

***Silk fibroin-based matrices.*** Silk fibroin is a particularly appealing biopolymer, *e.g.,* because of its versatile processing *e.g.,* all-aqueous processing (Sofia et al., 54 J. Biomed. Mater. Res. 139 (2001); Perry et al., 20 Adv. Mater. 3070-72 (2008)), relatively easy functionalization (Murphy et al., 29 Biomat. 2829-38 (2008)), and biocompatibility (Santin et al., 46 J. Biomed. Mater. Res. 382-9 (1999)). For example, silk has been approved by U.S. Food and Drug Administration as a tissue engineering scaffold in human implants. See Altman et al., 24 Biomaterials: 401 (2003).

As used herein, the term "silk fibroin" includes silkworm fibroin and insect or spider silk protein. See *e.g.,* Lucas et al., 13 Adv. Protein Chem. 107 (1958). Any type of silk fibroin can be used in different embodiments described herein. Silk fibroin produced by silkworms, such as Bombyx mori, is the most common and represents an earth-friendly, renewable resource. For instance, silk fibroin used in a silk film may be attained by extracting sericin from the cocoons of B. mori. Organic silkworm cocoons are also commercially available. There are many different silks, however, including spider silk (*e.g.,* obtained from Nephila clavipes), transgenic silks, genetically engineered silks, such as silks from bacteria, yeast, mammalian cells, transgenic animals, or transgenic plants (see, *e.g.,* WO 97/08315; U.S. Patent No. 5,245,012), and variants thereof, that can be used. In some embodiments, silk fibroin can be derived from other sources such as spiders, other silkworms, bees, and bioengineered variants thereof.

Silk for use in the methods and compositions described herein can be obtained from a silkworm, a spider, or from a recombinant organism engineered to produce silk (*e.g., Escherichia Coli,* tobacco and potato plants, or goats (silk protein secreted in their milk)). Non-limiting examples of silkworm silk include silk obtained from spider, *Bombyx mori, Antheraea mylytta, A. assamentis, Nephila clavipes,* and *Samia cyntia.*

In some embodiments, the biomaterial can comprise spider silk. Non-limiting examples of spider silk can include silk obtained from *Nephila clavipes* and *Araneus diadematus.* The major component of spider silk is spidroin. A single spider can produce multiple varieties of spider silk, any of which can be used in the methods and compositions described herein. The strongest type of spider silk is the dragline silk produced by the ampullate gland. It is composed of two major proteins: major ampullate spidroin 1 and 2 (MaSp1 and MaSp2). MaSp1 from N. *clavipes* is mainly composed of the amino acids glycine, alanine, glutamic acid, proline, and arginine. Like silkworm silk, the β-sheet crystalline domain contributes to the high tensile strength but the domains of MaSp1 and MaSp2 contain repeats of alanine or glycine-alanine. The difference in amino acid composition and sequence lead to superior mechanical properties compared to silkworm silk. In some embodiments, the biomaterial comprises spidroin. In some embodiments, the biomaterial comprises dragline silk. In some embodiments, the biomaterial comprises major ampulltae spidroin 1. In some embodiments, the biomaterial comprises major ampulltae spidroin 2.

In some embodiments, the biomaterial can comprise silk fibroin. In various embodiments, the silk fibroin can be modified for different applications and/or desired mechanical or chemical properties (*e.g.,* to facilitate formation of a gradient of an immune cell-modulating agent (*e.g.,* a macrophage-skewing agent) in silk fibroin-based matrices). One of skill in the art can select appropriate methods to modify silk fibroins, *e.g.,* depending on the side groups of the silk fibroins, desired reactivity of the silk fibroin and/or desired charge density on the silk fibroin. In one embodiment, modification of silk fibroin can use the amino acid side chain chemistry, such as chemical modifications through covalent bonding, or modifications through charge-charge interaction. Exemplary chemical modification methods include, but are not limited to, carbodiimide coupling reaction (see, *e.g.,* U.S. Patent Application. No. US 2007/0212730), diazonium coupling reaction (see, *e.g.,* U.S. Patent Application No. US 2009/0232963), avidin-biotin interaction (see, *e.g.,* International Application No.: WO 2011/011347) and pegylation with a chemically active or activated derivatives of the PEG polymer (see, e*.g.,* International Application No. WO 2010/057142). Silk fibroin can also be modified through gene modification to alter functionalities of the silk protein (see, *e.g.,* International Application No. WO 2011/006133). For instance, the silk fibroin can be genetically modified, which can provide for further modification of the silk such as the inclusion of a fusion polypeptide comprising a fibrous protein domain and a mineralization domain, which can be used to form an organic-inorganic composite. See WO 2006/076711. Additionally, the silk fibroin-based matrix can be combined with a chemical, such as glycerol, that, *e.g.,* affects flexibility of the matrix. See, *e.g.,* WO 2010/042798, Modified Silk films Containing Glycerol. In some embodiments, silk fibroin can be genetically engineered to be conjugated with an active agent (*e.g.,* an immune cell-modulating agent or a macrophage-skewing agent).

As used herein, the phrase "silk fibroin-based matrix" generally refer to a matrix comprising silk fibroin. In some embodiments, the phrase "silk fibroin-based matrix" refer to a matrix in which silk fibroin constitutes at least about 30% of the total composition, including at least about 40%, at least about 50%, at least about 60%, at least about 70%, at least about 80%, at least about 90%, at least about 95% or higher, of the total composition. In certain embodiments, the silk fibroin-based matrix can be substantially formed from silk fibroin. In various embodiments, the silk fibroin-based matrix can be substantially formed from silk fibroin comprising at least one immune cell-modulating agent (*e.g.,* at least one macrophage-skewing agent).

The silk fibroin-based matrix described herein can be adapted to be any shape, *e.g.,* a spherical shape, polygonal-shaped, elliptical-shaped, cylindrical-shaped, tubular-shaped, or any art-recognized shapes. The size of the silk fibroin-based matrix can vary with a number of factors including, without limitations, types of applications, the size of a target site for implantation, and/or desired properties of the silk fibroin-based matrix, *e.g.,* degradation profile. In some embodiments, the silk fibroin-based matrix can have a size of void or defect (*e.g.,* tissue damage) at a target site. In some embodiments, the silk fibroin-based matrix can have a surface area sufficient to form a coating of an implantable structure described herein.

The silk fibroin-based matrices can be produced from aqueous-based or organic solvent-based silk fibroin solutions. In some embodiments, the silk fibroin-based matrices produced from organic solvent-based silk fibroin solution can be more resistant to degradation than the aqueous-based silk fibroin-based matrices. The aqueous- or organic solvent-based silk fibroin solution used for making silk fibroin-based matrices described herein can be prepared using any techniques known in the art. The concentration of silk fibroin in solutions used for producing a silk fibroin-based matrix can be suited to needs. By way of example only, for tissue repair or augmentation, the concentration of silk fibroin in solutions can be suited to a particular degradation profile, *e.g.,* higher concentrations of silk fibroin solutions can be used when higher resistance to degradation is desired tissue repair or augmentation. In some embodiments, the silk fibroin solution for making the silk fibroin-based matrices described herein can vary from about 0.1% (w/v) to about 30% (w/v), inclusive. In some embodiments, the silk fibroin solution can vary from about 0.5% (w/v) to about 10% (w/v). In some embodiments, the silk fibroin solution can vary from about 1% (w/v) to about 6% (w/v).Suitable processes for preparing silk fibroin solution are disclosed, for example, in U.S. Patent No.: US 7635755; and International Application Nos: WO/2005/012606; and WO/2008/127401. A micro-filtration step can be used herein. For example, the prepared silk fibroin solution can be processed further, *e.g.,* by centrifugation and/or syringe based micro-filtration before further processing into silk fibroin-based matrices described herein.

In some embodiments, the silk fibroin can be also mixed with other biocompatible and/or biodegradable polymers described herein to form mixed polymer matrices comprising silk fibroin. One or more biocompatible and/or biodegradable polymers (*e.g.,* two or more biocompatible polymers) can be added to the silk fibroin solution. Additional biocompatible polymer that can be used herein include, but are not limited to, polyethylene oxide (PEO), polyethylene glycol (PEG), collagen, fibronectin, keratin, polyaspartic acid, polylysine, alginate, chitosan, chitin, hyaluronic acid, pectin, polycaprolactone, polylactic acid, polyglycolic acid, polyhydroxyalkanoates, dextrans, polyanhydrides, polymer, PLA-PGA, polyanhydride, polyorthoester, polycaprolactone, polyfumarate, collagen, chitosan, alginate, hyaluronic acid and other biocompatible and/or biodegradable polymers. See, *e.g.,* International Application Nos.: WO 04/062697; WO 05/012606.

In some embodiments, at least one immune cell-modulating agent (*e.g.,* at least one macrophage-skewing agent) described herein can be added to a silk fibroin solution before further processing into silk fibroin-based matrices described herein. In some embodiments, at least one immune cell-modulating agent (*e.g.,* at least one macrophage-skewing agent) can be dispersed homogeneously or heterogeneously within the silk fibroin, or dispersed in a gradient, *e.g.,* using the carbodiimide-mediated modification method described in the U.S. Patent Application No. US 2007/0212730.

In some embodiments, the silk fibroin-based matrices can be first formed and then contacted with (*e.g.,* dipped into) at least one immune cell-modulating agent (*e.g.,* at least one macrophage-skewing agent) such that the open or exposed surface of the matrices can be coated with at least one immune cell-modulating agent (*e.g.,* at least one macrophage-skewing agent) .

In some embodiments, the silk fibroin-based matrices described herein can comprise porous structures, *e.g.,* to mimic the structural morphology of a native tissue, to modulate the degradation rate of the silk fibroin-based matrices, and/or to modulate release profile of an immune cell-modulating agent (*e.g.,* at least one macrophage-skewing agent) embedded therein. As used herein, the terms "porous" and "porosity" are generally used to describe a structure having a connected network of pores or void spaces (which can, for example, be openings, interstitial spaces or other channels) throughout its volume. The term "porosity" is a measure of void spaces in a material, and is a fraction of volume of voids over the total volume, as a percentage between 0 and 100% (or between 0 and 1).

In some embodiments, the porous silk fibroin-based matrices can be configured to have any porosity, depending on the desired properties. For example, in some embodiments, the porous silk fibroin-based matrix can have a porosity of at least about 1%, at least about 3%, at least about 5%, at least about 10%, at least about 15%, at least about 20%, at least about 25%, at least about 30%, at least about 40%, at least about 50%, at least about 60%, at least about 70%, at least about 80%, at least about 90% or higher. In some embodiments, the porosity can range from about 70% to about 99%, or from about 80% to about 98%. The pore size and total porosity values can be quantified using conventional methods and models known to those of skill in the art. For example, the pore size and porosity can be measured by standardized techniques, such as mercury porosimetry and nitrogen adsorption. One of ordinary skill in the art can determine the optimal porosity of the silk fibroin-based matrices for various purposes. For example, the porosity and/or pore size of the silk fibroin-based matrices can be optimized based on the desired degradation rate or volume retention rate of the silk fibroin-based matrices, release profiles of an active agent from the silk fibroin-based matrices, and/or the structural morphology of the tissue to be repaired or augmented.

The pores can be adapted to have any shape, *e.g.,* circular, elliptical, or polygonal. The porous silk fibroin-based matrices can be adapted to have a pore size ranging from nanometers to micron-meters. In some embodiments, the porous silk fibroin-based matrices can be adapted to have a pore size of about 1 nm to about 1000 µm, about 50 nm to about 900 µm, about 100 nm to about 800 µm, about 500 nm to about 700 µm, about 1 µm to about 600 µm, about 10 µm to about 500 µm, about 50 µm to about 400 µm, or about 75 µm to about 250 µm. In some embodiments, the silk fibroin-based matrix can have a pore size of more than 1000 µm. In other embodiments, the silk fibroin-based matrix needs not be porous. In such embodiments, the pore size of the silk fibroin-based matrix can be less than 10 nm or non-detectable. The term "pore size" as used herein refers to a dimension of a pore. In some embodiments, the pore size can refer to the longest dimension of a pore, *e.g.,* a diameter of a pore having a circular cross section, or the length of the longest cross-sectional chord that can be constructed across a pore having a non-circular cross-section. In other embodiments, the pore size can refer the shortest dimension of a pore.

Methods for generating porous structures within silk fibroin-based matrix, *e.g.,* freeze-drying, porogen-leaching method (*e.g.,* salt-leaching), and gas foaming methods, are well known in the art and have been described in, *e.g.,* U.S. Patent No. US 7842780; and US Patent Application Nos: US 2010/0279112; and US 2010/0279112.

In some embodiments, porous silk fibroin-based matrices can be produced by freeze-drying method. See, *e.g.,* US 7842780, and US 2010/0279112. In such embodiments, the silk fibroin solution placed in a non-stick container can be frozen at sub-zero temperatures, *e.g.,* from about -80 °C to about -20 °C , for at least about 12 hours, at least about 24 hours, or longer, followed by lyophilization. In one embodiment, the silk fibroin solution can be frozen from one direction. In some embodiments, the silk fibroin solution can contain no salt. In some embodiments, alcohol such as 15%-25% of methanol or propanol can be added to the silk fibroin solution.

In certain embodiments, porous silk fibroin-based matrices can be produced by freezing the silk fibroin solution at a temperature range between about -1 °C and about -20 °C or between about -5 °C and -10 °C, for at least about 2 days, at least about 3 days or longer, followed by lyophilization for at least about 2 days, at least about 3 days or longer. See, *e.g.,* WO 2012/145594. The freezing temperature and/or duration, and/or lyophilization duration can be adjusted to generate a silk fibroin-based matrix of different porous structures and/or mechanical properties.

The biodegradability of a biomaterial composition comprising silk fibroin can be controlled by varying the structure and molecular weight of silk fibroin, where, without wishing to be bound by theory, most proteolytic enzymes are better at degrading silk fibroin with low molecular weight and non-compact structures (22). The silk fibroin composition can be altered by changing the crystallinity (by increasing or decreasing the amount of β-sheets), pore size, porosity, molecular weight distribution, and/ or controlling the physical geometry of the silk.

In some embodiments, silk fibroin-based matrices described herein can be subjected to a post-treatment that will affect at least one silk fibroin property. For example, post-treatment of silk fibroin-based matrices can affect silk fibroin properties including β-sheet content, solubility, active agent loading capacity, degradation time, drug permeability or any combinations thereof. Silk post-processing options include controlled slow drying (Lu et al., 10 Biomacromolecules 1032 (2009)), water annealing (Jin et al., Water-Stable Silk Films with Reduced β-Sheet Content, 15 Adv. Funct. Mats. 1241 (2005)), stretching (Demura & Asakura, Immobilization of glucose oxidase with Bombyx mori silk fibroin by only stretching treatment and its application to glucose sensor, 33 Biotech & Bioengin. 598 (1989)), compressing, and solvent immersion, including methanol (Hofmann et al., 2006), ethanol (Miyairi et al., 1978), glutaraldehyde (Acharya et al., 2008) and 1-ethyl-3-(3-dimethyl aminopropyl) carbodiimide (EDC) (Bayraktar et al., 2005).

In some embodiments, post-treatment of the silk fibroin-based matrices, *e.g.,* water-annealing or solvent immersion, can permit controlling the release of at least one immune cell-modulating agent (*e.g.,* at least one macrophage-skewing agent) from the silk fibroin-based matrices. In some embodiments, post-treatment of the silk fibroin-based matrices, *e.g.,* water-annealing or solvent immersion, can permit modulating the degradation or solubility properties of the silk fibroin-based matrices used in the compositions and methods described herein. In some embodiments, post-treatment of the silk fibroin-based matrices, *e.g.,* water-annealing or solvent immersion, can permit modulating the degradation properties of the silk fibroin-based matrices used in the compositions and methods described herein, where the degradation is induced by, *e.g.,* inflammatory responses.

In some embodiments, the silk fibroin-based matrices described herein can comprise a coating, *e.g.,* coated with at least one layer of a biocompatible and/or biodegradable polymer described herein, *e.g.,* to modulate the degradation properties of the silk fibroin-based matrices and/or to modulate the rate of at least one immune cell-modulating agent (*e.g.,* at least one macrophage-skewing agent) released from the silk fibroin-based matrices. In some embodiments, the biocompatible and/or biodegradable polymer can also comprise at least one immune cell-modulating agent (*e.g.,* at least one macrophage-skewing agent).

In some embodiments, the silk fibroin-based matrices described herein can be modified, *e.g.,* coated with cell adhesion molecules or peptides, *e.g.,* but not limited to, fibronectin, vitronectin, laminin, collagen, RGD peptides any art-recognized extracellular matrix molecules, and any combinations thereof.

In some embodiments, the silk fibroin-based matrices described herein can be sterilized. Sterilization methods for biomedical devices are well known in the art, including, but not limited to, gamma or ultraviolet radiation, autoclaving (*e.g.,* heat/ steam); alcohol sterilization (*e.g.,* ethanol and methanol); and gas sterilization (*e.g.,* ethylene oxide sterilization).

Further, the silk fibrin matrices described herein can take advantage of the many techniques developed to functionalize silk fibroin (*e.g.,* active agents such as dyes and sensors). See, *e.g.,* U.S. Patent No. 6,287,340, Bioengineered anterior cruciate ligament; WO 2004/000915, Silk Biomaterials & Methods of Use Thereof; WO 2004/001103, Silk Biomaterials & Methods of Use Thereof; WO 2004/062697, Silk Fibroin Materials & Use Thereof; WO 2005/000483, Method for Forming inorganic Coatings; WO 2005/012606, Concentrated Aqueous Silk Fibroin Solution & Use Thereof; WO 2011/005381, Vortex-Induced Silk fibroin Gelation for Encapsulation & Delivery; WO 2005/123114, Silk-Based Drug Delivery System; WO 2006/076711, Fibrous Protein Fusions & Uses Thereof in the Formation of Advanced Organic/Inorganic Composite Materials; U.S. Application Pub. No. 2007/0212730, Covalently immobilized protein gradients in three-dimensional porous scaffolds; WO 2006/042287, Method for Producing Biomaterial Scaffolds; WO 2007/016524, Method for Stepwise Deposition of Silk Fibroin Coatings; WO 2008/085904, Biodegradable Electronic Devices; WO 2008/118133, Silk Microspheres for Encapsulation & Controlled Release; WO 2008/108838, Microfluidic Devices & Methods for Fabricating Same; WO 2008/127404, Nanopatterned Biopolymer Device & Method of Manufacturing Same; WO 2008/118211, Biopolymer Photonic Crystals & Method of Manufacturing Same; WO 2008/127402, Biopolymer Sensor & Method of Manufacturing Same; WO 2008/127403, Biopolymer Optofluidic Device & Method of Manufacturing the Same; WO 2008/127401, Biopolymer Optical Wave Guide & Method of Manufacturing Same; WO 2008/140562, Biopolymer Sensor & Method of Manufacturing Same; WO 2008/127405, Microfluidic Device with Cylindrical Microchannel & Method for Fabricating Same; WO 2008/106485, Tissue-Engineered Silk Organs; WO 2008/140562, Electroactive Biopolymer Optical & Electro-Optical Devices & Method of Manufacturing Same; WO 2008/150861, Method for Silk Fibroin Gelation Using Sonication; WO 2007/103442, Biocompatible Scaffolds & Adipose-Derived Stem Cells; WO 2009/155397, Edible Holographic Silk Products; WO 2009/100280, 3-Dimensional Silk Hydroxyapatite Compositions; WO 2009/061823, Fabrication of Silk Fibroin Photonic Structures by Nanocontact Imprinting; WO 2009/126689, System & Method for Making Biomaterial Structures.

In an alternative embodiment, the silk fibroin-based matrices can include plasmonic nanoparticles to form photothermal elements. This approach takes advantage of the superior doping characteristics of silk fibroin. See, for example, WO/2012/031282 *"Plasmonic nanoparticle-doped silk materials*,". Thermal therapy has been shown to aid in the delivery of various agents, see Park et al., Effect of Heat on Skin Permeability, 359 Intl. J. Pharm. 94 (2008). In one embodiment, short bursts of heat on limited areas can be used to maximize permeability with minimal harmful effects on surrounding tissues. Thus, plasmonic particle-doped silk fibroin-based matrices can add specificity to thermal therapy by focusing light to locally generate heat only via the silk fibroin-based matrices. In some embodiments, the silk fibroin-based matrices can include photothermal agents such as gold nanoparticles.

In some embodiments, the silk fibroin-based matrices used in the methods described herein can include an amphiphilic peptide. In other embodiments, the silk fibroin-based matrices used in the methods described herein can exclude an amphiphilic peptide. "Amphiphilic peptides" possess both hydrophilic and hydrophobic properties. Amphiphilic molecules can generally interact with biological membranes by insertion of the hydrophobic part into the lipid membrane, while exposing the hydrophilic part to the aqueous environment. In some embodiment, the amphiphilic peptide can comprise a RGD motif. An example of an amphiphilic peptide is a 23RGD peptide having an amino acid sequence: HOOC-Gly-ArgGly-Asp-Ile-Pro-Ala-Ser-Ser-Lys-Gly-Gly-Gly-Gly-SerArg-Leu-Leu-Leu-Leu-Leu-Leu-Arg-NH2. Other examples of amphiphilic peptides include the ones disclosed in the U.S. Patent App. No.: US 2011/0008406.

In some embodiments, the biomaterial can comprise silk from the *B. mori* silkworm. Silk fibers from the *B. mori* silkworm are composed of two types of proteinaceous polymers, fibroin and sericin. Fibroin is the inner-core protein filament and consists of hydrophobic amino acids accounting for up to 90 % of the total molecular weight. It is characterized by a highly repetitive primary sequence: glycine-alanine-glycine-alanine-glycine-serine (GAGAGS). These repeats leading to significant homogeneity in secondary structure, an anti-parallel β-sheet structure. This structure is responsible for unique silk elasticity and tensile strength. These β-sheets are highly crystalline and essentially crosslink the protein through strong intra- and inter- molecular hydrogen bonds, as well as strong Van Der Waals interactions between stacked β-sheets (10). This particular structure gives the material impressive mechanical properties. For instance, silk fibroin exhibits a greater elasticity than fibers of comparable tensile integrity (6-7 times higher than Kevlar™ 49 for example) (11). In some embodiments, the biomaterial composition can comprise fibroin.

The other protein found in *B. mori* silk, sericin, is a glue-like protein forming a hydrophobic coat around silk fibers. It constitutes 25-30% of silk protein and binds together two fibroin threads (12-14). Sericin protein can be crosslinked or used as a coating to enhance its antibacterial properties and its resistance to UV and oxidative degradation (15). Sericin has various applications in the cosmetic and pharmaceutical industries as wound healing agents, bioadhesives, and anti-wrinkle and anti-aging moisturizers (16). In some embodiments, the biomaterial composition can comprise sericin.

Sericin can cause allergic reactions in some subjects. In some embodiments, the biomaterial or the composition described herein does not include sericin, *e.g.,* sericin in an amount of about 1% or less of the silk protein contained in the biomaterial composition, *e.g.,* about 1% or less, about 0.5% or less, about 0.1% or less, or about 0.01% or less. In some embodiments, the biomaterial or the composition can comprise fibroin, but not sericin, *e.g.,* sericin in an amount of about 1% or less of the silk protein contained in the biomaterial or composition, *e.g.,* about 1% or less, about 0.5% or less, about 0.1% or less, or about 0.01% or less. In some embodiments, the biomaterial or the composition described herein does comprise any detectable level of sericin. Purification protocols to remove sericin to yield silk fibroin with no antigenic effects are well-known in the art. By way of non-limiting example, sericin can be removed by cutting *Bombyx mori* cocoons into small pieces and boiling for 30 min in an aqueous solution of about 0.02 M sodium carbonate. Alternatively, sericin can be removed by boiling cocoons in an aqueous solution of about 9.3 M LiBr, *e.g.,* as described in Example 1.

In some embodiments, the biomaterial can comprise a silk fibroin-based matrix. A silk fibroin-based matrix can be in the form of a film, a fiber, a collection of particles, a mat, a tube, a gel, a fabric, a mesh, or any combinations thereof. Silk fibroin-based matrices can be produced according to the following protocols or any other method known in the art. After removing the sericin from the silk, a fibroin solution is produced. Several protocols have been developed to process the silk fibroin solution into different forms suitable for the specific biomedical applications such as wound dressings and scaffolds for tissue engineering (*e.g.,* see U.S. Patents ; 6,175,053 and U.S. Patent Publications 2011/0111031; 2003/0165548; and 2011/0171239). Fibroin solution can be also electrospun to produce fibers that may be later wound into yarns (see Altman et al. 2003, Biomaterials, Vols. Volume 24, Issue 3, pp. 401-416). In its simplest form, silk fibroin can be regenerated into film or coated onto other materials. It can also be used in combination with other materials such as gelatin to generate hydrogels for example and hydroxyapatite to make silk porous scaffold for bone regeneration (see U.S. Patent Publication 2011/0046686). Silk fibroin can also be chemically modified through amino acid side chains to alter surface properties or to immobilize RGD (Arginine-Glycine-Aspartic) sequences or other cellular growth factors to enhance the cell attachment and proliferation (see Kearns et al. Silk-based Biomaterials for Tissue Engineering. Eds. N Ashammakhi, R Reis, & F Chiellini s.l. : Tissue Engineering, 2008. Vol. 4). Silk fibroin can be processed aqueously and the size, secondary structure, and zeta potential controlled (see Lammel et al. Biomaterials, 2010 Jun, Vol. 31(16), pp. 4583-91). Use of an aqueous salt (potassium phosphate) out process enables production of particles with controlled and reproducible sizes depending on silk fibroin concentration. Furthermore, secondary structure remains stable upon sterilization by autoclaving or ethanol and methanol treatment. In some embodiments, by way of example only, aqueous processing can be performed as follows.

Following removal of sericin, extracted silk fibroin can be rinsed thoroughly 3 times every 20 min in distilled water to remove the sodium carbonate. After at least 12 h of air drying, the extracted silk fibroin can be dissolved in ∼9.3 M LiBr solution at ∼60°C for about 4 h minimum. Once completely dissolved, the solution can be dialyzed against distilled water using Slide-a-lyzer dialysis cassettes (MWCO 3500, Pierce) for 3 days to remove the salt (thrice the first day, twice the second, and once the third). The solution can then be centrifuged twice at 10,000 rpm for ∼20 min at (5-10) °C to remove silk aggregates as well as debris from original cocoons. The final concentration of silk fibroin aqueous solution can be determined, for example, by weighing about 1 ml of the solution, drying, and comparing against its residual solid after drying at ∼60°C.

In some embodiments, the silk fibroin-based matrix can comprise particles. In some embodiments, the silk fibroin-based matrix can comprise nanoparticles. In some embodiments, the silk fibroin-based matrix can comprise microparticles. Particles can be prepared, for example, by using an aqueous salt out process with potassium phosphate solution that induces a phase separation (27). In general, the size of the silk fibroin particles can be correlated with the concentration of silk fibroin aqueous solution used. The concentration of silk fibroin solution used can determine the size of the resulting particles; *e.g.,* about 1 % of silk fibroin stock solution diluted in distilled water can result in ∼2 µm sized-particles; about 0.5 % stock solution can yield ∼1 µm particles and about 0.1% stock solution can yield particles smaller than 500 nm. Briefly, a solution of ∼1.5 M K₂HPO₄ (pH 8) can be prepared. Silk fibroin solution with the appropriate concentration can be prepared by diluting the stock solution with distilled water. The silk fibroin solution can then be added quickly, using a pipette, into the potassium phosphate solution in pre-determined volumetric ratios, *e.g.,* a volumetric ratio of about 1:5. The resulting particles can be stored for about 2 h to overnight at ∼4°C. The solutions can then be centrifuged, *e.g.,* four times: three times in distilled water and once in 70 % ethanol at 2500xg for about 15 min. After each centrifugation, the supernatant can be removed and the silk particles can be redispersed in the appropriate solution. After the last centrifugation in ethanol, the supernatant can be removed, and the particle pellets can be dried at a temperature, *e.g.,* of about 70°C.

In some embodiments, the silk fibroin-based matrix can comprise a film. For example, a film can be prepared using a silk fibroin solution having about 2 % w/v silk fibroin in distilled water. The solution can be allowed to evaporate and reconstituted with methanol to crosslink the silk proteins by β-sheet formation. After removing the excess methanol, the silk films can be sterilized under by exposing to UV light and allowed to dry.

In some embodiments, the silk fibroin-based matrix can comprise a silk fibroin-based mesh and/or a silk fibroin-based fabric. Methods for producing silk fibroin-based meshes and silk fibroin-based fabrics are known in the art, e.g., by interweaving silk fibroin-based yarns into a pre-determined pattern. The silk fibroin-based yarns can be produced from a silk fiber (e.g., a raw silk fiber or a silk fiber formed from a silk fibroin solution, e.g., by electrospinning). In one embodiment, silk fibroin-based yarns can be produced from a combination of silk yarns or fibers and non-silk yarns or fibers, e.g., man-made yarns or fibers such as, but not limited to, ultra high molecular weight polyethylene yarns (e.g., DYNEEMA®). The use of non-silk yarns or fibers in combination with silk yarns or fibers can, for example, enhance the mechanical properties of silk fibroin-based yarns and the resulting mesh. The silk fibroin-based meshes and/or silk fibroin-based fabrics can be, for example, weft-knitted and/or warp-knitted. In one embodiment, silk fibroin-based meshes and fabrics as well as methods of making the same described, e.g., in the U.S. Pat. Appl. Nos. US 2012/0304702 and US 2012/0221105, can be employed in the compositions and methods described herein.

In some embodiments, the silk fibroin-based matrix can comprise a sponge or a porous sponge. In some embodiments, the silk fibroin-based matrix can comprise a wound dressing. In some embodiments, the silk fibroin-based matrix can comprise a bone tissue scaffold. In some embodiments, the silk fibroin-based matrix can comprise a hydrogel. In some embodiments, the silk fibroin-based matrix can comprise a non-woven mat. In some embodiments, the silk fibroin-based matrix can comprise a cartilage tissue scaffold. In some embodiments, the silk fibroin-based matrix can comprise a fiber. In some embodiments, the silk fibroin-based matrix can be adapted for use in ligament tissue engineering, tendon tissue engineering, hepatic tissue engineering, connective tissue engineering, endothelial tissue engineering, blood vessel engineering, and/or anti-thrombogenesis. In some embodiments, the silk fibroin-based matrix can comprise at least one silk fibroin hydrogel, silk fibroin implant, silk fibroin fabric or fiber, or other tissue engineering composition as described in U.S. Patent Publications 2011/0189773; 2011/0052695; 2011/0009960; 2010/0256756; and 2010/0209405.

### Immune cell-modulating agents (e.g., macrophage-skewing agents)

The compositions and methods described herein are directed to biomaterials comprising at least one immune cell-modulating agent. An immune cell-modulating agent can be any agent that can alter the activation state of at least one immune cell type, for example, but not limited to, monocytes, macrophages, dendritic cells, megakaryocytes, granulocytes, T cells, B cells, natural killer (NK) cells, neutrophils, and any combinations thereof.

The term "agent" refers generally to any entity which is normally not present or not present at the levels being administered to a cell, tissue or subject. Examples of immune cell-modulating agents include, but are not limited to, proteins (*e.g.,* polypeptides), peptides, antigens, immunogens, antibodies or portions thereof (*e.g.,* antibody-like molecules), enzymes, nucleic acids (*e.g.,* oligonucleotides, polynucleotides, siRNA, shRNA), aptamers, bacteria, virus, small molecules, functional fragments, and any combinations thereof.

Selection of immune cell-modulating agent(s) (*e.g.,* macrophage-skewing agent(s)) can vary with a number of factors, including, *e.g.,* types of immune cells to be targeted, and/or applications of the compositions described herein. In some embodiments, the immune cell-modulating agent (*e.g.,* at least one macrophage-skewing agent) can be selected to control activation state of a specific type of immune cells (*e.g.,* macrophages). In some embodiments, the immune cell-modulating agent(s) can be selected to control response of immune cells (*e.g.,* macrophages) to a target stimulus described herein *in vitro* or *in vivo.* In one embodiment, at least one immune cell-modulating agent (*e.g.,* at least one macrophage-skewinag agent) can be selected to control or alter response of immune cells (*e.g.,* macrophages) to a target stimulus described herein in a subject. Examples of response of immune cells (*e.g.,* macrophages) to a target stimulus can include, but are not limited to, inflammatory response (*e.g.,* release of proinflammatory or inflammatory factors such as TNF-α), degradation of a biomaterial implant, regenerative response, cell viability, phagocytosis, cell fusion, release of anti-inflammatory factors such as IL-10, change in cell morphology in response to the target stimulus (*e.g.,* round cells or elongated cells with or without pseudopods), production of extracellular matrix, and any combinations thereof.

***Macrophage Response and Differentiation.*** Biomaterials derived from an exogenous source (i.e., not from a host) will elicit some level of foreign body response following implantation *in vivo.* Following implantation, the first stage induced is an inflammatory response initiated by neutrophils and/or macrophages. Along with dendritic cells, lymphocytes are activated leading to an adaptive response in case of complication. For example, silk fibroin in film or fiber form induces low levels of inflammatory responses, but no significant macrophage activation (20).

An inflammatory response that lasts only a few days is called acute inflammation, while a response of longer duration (weeks to months to years) is referred to as chronic inflammation. Macrophages (Mcps) and dendritic cells (DCs) are critical antigen presenting cells (APCs) that play pivotal roles in host responses to biomaterial implants. Moreover, Mϕs and, DCs interact and stimulate helper T cells which also play an important role.

Phagocytes, including neutrophils, monocytes and Mcps, play a crucial role in host defense by recognition and elimination of invading pathogens. Mϕs generally phagocytose small materials or foreign components to quickly eliminate them. They produce reactive oxygen species (ROS), inflammatory cytokines and chemokines, leading to bacterial killing, recruitment, and activation of additional immune cells (30).

Tissue Mϕs are derived from circulating monocytes in response to microenvironmental factor such as macrophage colony-stimulating factor (M-CSF), granulocytes macrophage colony stimulating factor (GM-CSF) and IL-3 during extra-vascularization and via PMA (phorbol 12-myristate 13-acetate) *in vitro* (31-34). Upon differentiation, Mϕs can be further activated by extracellular signals and display different functions in response to cytokines and microbial products in the environment. There are two types of Mϕs: classically activated macrophage (M1) and alternatively activated Mϕs (M2). In response to Th1 cytokines, such as IFN-γ and pathogen-associated molecular patterns (PAMPs) such as LPS, M1 macrophages display a classical activation phenotype. They produce mainly proinflammatory cytokines such as TNF-α, IL-1, IL-6, IL-12 and IL-23, and possess anti-proliferative functions (30, 35-37). Alternatively, Mϕs can be activated by Th2 cytokines, such as IL-4 and IL-13. M2 are subdivided into three subsets: M2a, M2b and M2c based on their phenotype. M1 and M2b are inflammatory and microbicidal whereas M2a and M2c have anti-inflammatory and tissue repair properties and secrete IL-10, IL-1 antagonist receptor (IL-1Ra), and Transforming Growth Factor β(TGF-β) cytokines. The M1 macrophage can generally elicit an inflammatory response, *e.g.,* to protect newly damaged tissue from infection. In some embodiments, the inflammatory response elicited by the M1 macrophages can be undesirable for tissue remodeling. In some embodiments, the inflammatory response elicited by the M1 macrophages can be used for rapid degradation of biomaterials such as silk.

Cell surface markers expressed by immune cells, *e.g.,* macrophages are known in the art. For example, macrophages generally express cell surface markers, *e.g.,* but not limited to, CD11b and F4/80 (mice). M1 macrophages express the cell surface marker, *e.g.,* but not limited to, MCP-1, CCR7, and CCL3; and M2 macrophages express the cell surface marker, *e.g.,* CD206 and CCL18. See, for example, Table 1 below for some specific markers of macrophages and dendritic cells in human and mouse. Means of detecting the presence of these markers on a cell or a population of cells are well-known in the art (*e.g.,* gene arrays, PCR, quantitative PCR, FACS, western blot, and any combinations thereof).

**Table 1: Specific markers of macrophages and DC's in human and mouse**

| **Specificity** | **Markers** | **Human** | **Mouse** |
|---|---|---|---|
| **Mϕs and monocytes** | CD11b | Yes | Yes |
| | F4/80 | No | Yes |
| **M1** | MCP-1/CCR2 | Yes | Yes |
| **M2** | CD206 | Yes | Yes |
| | CX3CR1 | No | Yes |
| **DCs** | CD11c | Yes | Yes |
| | CD80 | No | Yes |
| | CD205 | No | Yes |

***Macrophage-skewing agents.*** In some embodiments, the immune cell-modulating agent(s) can be selected to control or alter response of macrophages to a target stimulus described herein. For example, the immune cell-modulating agent can be selected to induce M1 macrophage phenotype; or alternatively, the immune cell-modulating agent(s) can be selected to induce M2 macrophage phenotype (*e.g.,* M2a, M2b, and M2c). In these embodiments, the immune cell-modulating agent can comprise at least one macrophage-skewing agent, including, *e.g.,* at least two, at least three or more macrophage-skewing agents.

Accordingly, described herein are also methods and compositions directed to biomaterial compositions which are functionalized with at least one macrophage-skewing agent. The at least one macrophage-skewing agent can be present in the silk fibroin-based matrix in an effective amount sufficient to selectively control or alter activation state of macrophages upon contact with the at least one macrophage-skewing agent.

A macrophage-skewing agent to be employed in the compositions and methods described herein can include any agent that can alter activation state of macrophages between M1 phenotype and M2 phenotype, or differentiate precursor cells (*e.g.,* monocytes) to M1 macrophages or M2 macrophages. In some embodiments, a macrophage-skewing agent can be an agent that can promote the differentiation of macrophages to either an M1 or M2 phenotype or switch at least a population of macrophages to either an M1 or M2 phenotypic state. Promotion of differentiation to a particular phenotype can be an increase in the total number of cells displaying that phenotype; an increase in the percentage of a population of cells displaying that phenotype, or an increase in the rate at which cells differentiate to that phenotype. In some embodiments, a macrophage-skewing agent can be an agent that switches at least a population of the macrophages to either an M1 or M2 phenotypic state. As used herein "at least a population" comprises two or more macrophages, *e.g.,* 2 macrophages, 100 macrophages, 1000 macrophages, 1 x10⁵ macrophages or more. As used herein "switching" a population of macrophages to, for example, the M1 phenotype can encompass differentiating monocytes or other precursors of a mature, activated macrophage to a mature, activated M1 or M2 phenotype, causing M1 macrophages to become M2 macrophages, and/or causing M2 macrophages to become M1 macrophages. In some embodiments, a macrophage-skewing agent can be an agent that selectively controls the activation state of macrophages which are exposed to the agent. In some embodiments, a macrophage-skewing agent can be an agent that promotes the proliferation of macrophages of the M1 and/or M2 phenotype.

Non-limiting examples of a macrophage-skewing agent can include glucocorticoid (*e.g.,* dexamethasone); nicotine; statins (*e.g.,* simvastatin); an antimicrobial peptide (*e.g.,* LL-37 peptide, apolipoprotein E); LPS; INF-γ; TNF-α; prolactin; Notch activators (*e.g.,* delta or jagged ligands); IL-4; IL-13; IL-10; insulin sensitizer and PPAR-γ inducer (*e.g.,* rosiglitazone or other thiazolidinediones); HDAC inhibitors (*e.g.,* VPA); Notch signaling inhibitors (*e.g.,* GSI or DAPT); JAK inhibitors (*e.g.,* AG490); inhibitors of 3-hydroxy-3-methylglutaryl-coenzyme A (HMG-CoA) reductase; LiCl; thymosin; PLA2, and any combinations thereof.

In some embodiments, at least one macrophage-skewing agent can comprise an agent selected to switch at least a population of the macrophages to M1 phenotypic state. In some embodiments, a macrophage-skewing agent can be an agent that promotes the differentiation of an M1 phenotype. In some embodiments, a macrophage-skewing agent that promotes the differentiation of an M1 phenotype can induce an inflammatory response. Non-limiting examples of such agents include LPS; INF-γ; TNF-α; prolactin; an anti-microbial peptide (*e.g.*) LL-37 peptide and Delta or Jagged ligands.

In some embodiments, at least one macrophage-skewing agent can comprise an agent selected to switch at least a population of the macrophages to M2 phenotypic state (*e.g.,* M2a, M2b, and M2c). In some embodiments, a macrophage-skewing agent can include an agent that promotes the differentiation of an M2 phenotype (*e.g.,* M2a, M2b, and M2c). In some embodiments, a macrophage-skewing agent that promotes the differentiation of an M2 phenotype can induce a regenerative response. Non-limiting examples of such agents can include, but are not limited to, IL-4; IL-13; IL-10; rosiglitazone ((RS)-5-[4-(2-[methyl(pyridine-2-yl)amino]ethoxy)benzyl]thiazolidine-2,4-dione; Avandia; Formula II; Cayman Chemical; Ann Arbor, MI Catalog No: 71740); or other thiazolidinediones (insulin sensitization and PPAR-γ induction)(*e.g*., pioglitazone ((RS)-5-(4-[2-(5-ethylpryidin-2-yl)ethyloxy]benzyl)thiazolidine-2,4-dione; Actos; Cayman Chemical; Ann Arbor, MI; Catalog No: 71745); troglitazone ((RS)-5-4-[(6-hydroxy-2,5,7,8-tetramethylchroman-2-yl)methoxy]benzyl)thiazolidine-2,4-dione; Rezulin; Cayman Chemical; Ann Arbor, MI; Catalog No: 71750); netoglitazone (5-[[6-[(2-fluorophenyl)methoxy]naphthalen-2-yl]methyl]-1,3-thiazolidine-2,4-dione; MCC-555;); rivoglitazone ((5-[[4-[2-(methyl-2-pyridinylamino)ethoxy]phenyl]methyl]-2,4-thiazolidinedione); ciglitazone (5-(4-[(1-methylcyclohexyl)methoxy]benzyl)-1,3-thiazolidine-2,4-dione; Cayman Chemical; Ann Arbor, MI; Catalog No: 71730); and balaglitzaone (5-[[4-[(3,4-dihydro-3-methyl-4-oxo-2-quinazolinyl) methoxy]phenyl]methyl]-2,4-thiazolidinedione); HDAC inhibitors (*e.g.,* VPA); Notch signaling inhibitors (*e.g.,* GSI or DAPT); an antimicrobial peptide (*e.g.,* Apopoliprotein E (ApoE)); or JAK inhibitors (*e.g.,* AG490).

Non-limiting examples of HDAC inhibitors can include, but are not limited to, valproic acid (VPA), hydroxamic acid derivatives, short-chain fatty acids such as butyrate, 4-phenylbutyrate or valproic acid; hydroxamic acids such as suberoylanilide hydroxamic acid (SAHA), biaryl hydroxamate A-161906, bicyclic arylN-hydroxycarboxamides, CG-1521, PXD-101, sulfonamide hydroxamic acid, LAQ-824, oxamflatin, scriptaid, m-carboxy cinnamic acid bishydroxamic acid, trapoxin-hydroxamic acid analogue, trichostatin A, trichostatin C, m-carboxycinnamic acid bis-hydroxamideoxamflatin (CBHA), ABHA, Scriptaid, pyroxamide, propenamides; epoxyketone-containing cyclic tetrapeptides such as trapoxins, apidicin, depsipeptide, HC-toxin, chlamydocin, diheteropeptin, WF-3161, Cyl-1 and Cyl-2; benzamides or non-epoxyketone-containing cyclic tetrapeptides such as FR901228, apicidin, cyclic-hydroxamic-acid-containing peptides (CHAPs), benzamides, MS-275 (MS-27-275), and CI-994; depudecin; PXD101; organosulfur compounds; aroylpyrrolylhydroxy-amides (APHAs), SAHA (Zolinza), trichostatin A, MS-275, LBH-589, PXD-101, MGCD-0103, JNJ-26481585, R306465 (J&J), and sodium butyrate.

Non-limiting examples of Notch signaling inhibitors can include, but are not limited to, gamma secretase inhibitor (GSI), LY-411575, DAPT (LY-374973, N-[N-(3,5-Difluorophenacetyl)-L-alanyl]-S-phenylglycine t-butyl ester), arylsulfonamides (AS), dibenzazepines (DBZ), benzodiazepines (BZ), L-685,458 (Sigma-Aldrich), and MK0752 (Merck).

Non-limiting examples of JAK inhibitors can include, but are not limited to, tyrphostins, tyrphostin AG490 and 2-naphthyl vinyl ketone.

Non-limiting examples of Delta and/or Jagged ligands can include Delta-1, Delta-2, Delta-3, Delta-4, Jagged-1 and Jagged-2 (Mumm J. S. et al., Dev. Biol., 228, 151-165, 2000) and functional polypeptide fragments thereof.

Further non-limiting examples of macrophage-skewing agents can include glucocorticoids (*e.g.,* dexamethasone, prednisone, methylprednisone, hydrocortisone, cortisone acetate, betamethasone, triamcinolone, ceblometasone, fludrocortisone acetate, deoxycorticosterone acetate, and aldosterone); nicotine; simvastatin; statins; inhibitors of 3-hydroxy-3-methylglutaryl-coenzyme A (HMG-CoA) reductase; LiCl; thymosin, or PLA2.

Non-limiting examples of HMG-CoA reductase inhibitors can include, but are not limited to, atorvastatin MEVACOR® (lovastatin), ZOCOR® (simvastatin), PRAVACHOL0(pravastatin), LESCOL® (fiuvastatin), and rivastatin or any of the compounds described in the following patent publications: U.S. Pat. No. 4,231, 938 (including *e.g.,* lovastatin); EP0033538 (including *e.g.,* simvastatin); GB2077264 (including *e.g.,* pravastatin); EP0114027 (including *e.g.,* fluvastatin); EP0247633 (including *e.g.,* atorvastatin); U.S. Pat. No. 3,983,140 (including *e.g.,* mevastatin); EP0491226 (including *e.g.,* rivastatin; cer(i)vastatin); U.S. Pat. No. 5,011,930 (including *e.g.,* pitavastatin, Nissan/Sankyo's nisvastatin (NK-104) or itavastatin); U.S. Pat. No. 5,260,440 (including *e.g.,* rosuvastatin or visastatin (ZD-4522) of Shionogi-Astra/Zeneca); and U.S. Pat. No. 5,753,675 (including statins related to statins as described above).

Additional macrophage-skewing agents can be identified by contacting a population of macrophages with a candidate agent and determining if there is an increase in the differentiation of macrophages to either an M1 or M2 phenotype. Markers for the identification of M1 and M2 phenotypes are known in the art and some makers are discussed above herein.

In some aspects, the inflammatory response induced by a biomaterial can be balanced by an anti-inflammatory or antimicrobial active agent. Accordingly, in some embodiments, the macrophage-skewing agent can comprise an agent that induces an inflammatory response and an anti-inflammatory or antimicrobial agent that balances the inflammatory response. Both agents can be released from the biomaterial (*e.g.,* a silk fibroin-based matrix) at different times and/or at different rates.

As described earlier, the macrophage-skewing agent(s) can be present in a biomaterial or silk fibroin-based matrix in any manner. The macrophage-skewing agent(s) can be distributed homogenously or heterogeneously, or distributed in a gradient in the biomaterial or silk fibroin-based matrix. In some embodiments, the macrophage-skewing agent(s) can be distributed in the same layer and/or in separate layers, *e.g.,* in a multi-layered biomaterial or silk fibroin-based matrix. In some embodiments, the macrophage-skewing agent can be encapsulated into the biomaterial composition. In some embodiments, the macrophage-skewing agent can be present on the surface of the biomaterial composition, *e.g.,* by dipping the biomaterial into a solution of the macrophage-skewing agent. In some embodiments, the macrophage-skewing agent can be encapsulated into the silk fibroin-based matrix. In some embodiments, the macrophage-skewing agent can be present on the surface of the silk fibroin-based matrix, *e.g.,* by dipping the silk fibroin-based matrix into a solution of the macrophage-skewing agent. In some embodiments, the macrophage-skewing agent can be bound or cross-linked to the biomaterial composition. In some embodiments, the macrophage-skewing agent can be bound or cross-linked to the silk fibroin-based matrix. Without wishing to be limiting, the macrophage-skewing agent can also be distributed in some embodiments of the compositions described herein.

### Target stimuli

Described herein are methods and compositions directed to controlling response of immune cells (*e.g.,* macrophages) to a target stimulus. As used herein, a target stimulus can comprise any *in vitro* or *in vivo* object, composition, disease, disorder, condition or matter that can cause or induce immune cells (*e.g.,* macrophages) to respond or react, *e.g.,* inducing inflammatory responses and/or regenerative responses. In some embodiments, a target stimulus can cause a change in the activation state of a population of immune cells (*e.g.,* macrophages) or cause a change in the number or percentage of macrophages in a population of cells which are characterized by either an M1 or M2 phenotype. In these embodiments, the compositions described herein can comprise an immune cell-modulating agent (*e.g.,* a macrophage-skewing agent) that can reduce or balance the reaction of immune cells to a target stimulus, and/or the change in the activation state of the immune cells induced by the target stimulus.

The target stimulus can comprise a macrophage-associated condition. Conditions, diseases and disorders which can induce immune cells (*e.g.,* macrophages) to respond, and/or which can cause a change in the activation state of a population of macrophages and/or which cause a change in the number or percentage of macrophages characterized by either an M1 or M2 phenotype are well-known in the art. Non-limiting examples of macrophage-associated conditions include bacterial infection, tissue regeneration, tissue damage, tissue reconstruction, including, e.g., tissue repair and/or augmentation (e.g., soft tissue repair and/or augmentation), arthritis, obesity, diabetes, arteriosclerosis, allograft transplantation, Langerhans cell histiocytosis (LCH), osteoporosis, glomerulonephritis, cancer, wound healing, and any combinations thereof.

The target stimulus can comprise an implantable structure, e.g., any structure that can be implanted into a tissue or void of a subject. Non-limiting examples of implantable structures can include scaffolds, stents, grafts, allograft tissues and medical devices.

Non-limiting examples of grafts include autografting, Epicel ® cultured epidermal autograft (Genzyme Cambridge, MA); Integra™ bilayer matrix wound dressing (Integra Lifescience Holdings Corp., Ontario, Canada); Alloderm ® acellular dermal matrix (LifeCell Corp., Branchburg, NJ); OrCel ® bilayered cellular matrix (Forticell Bioscience, Inc., New York, NY); and Apligraf ® living skin patch (Organogenesis Inc., Canton, MA). In some embodiments, the target stimulus can comprise a silk fiber based matrix, silk fibroin hydrogels, silk fibroin implants, silk fibroin fabrics or fibers, or other tissue engineering compositions as discussed in U.S. Patent Publications 2011/0189773; 2011/0052695; 2011/0009960; 2010/0256756; and 2010/0209405.

Non-limiting examples of medical devices can include stents, ports, catheters, sutures, staples, artificial organs, neurostimulators, pumps, drug delivery pumps, pacemakers, defibrillators, stent-grafts, grafts, artificial heart valves, foramen ovale closure devices, cerebrospinal fluid shunts, pacemaker electrodes, guide wires, ventricular assist devices, cardiopulmonary bypass circuits, blood oxygenators, vena cava filters, endocardial leads, endotracheal tubes, nephrostomy tube and orthopedic devices.

The target stimulus can comprise a cytokine. Non-limiting examples of cytokines include, but are not limited to chemokines interleukins, interferons, colony stimulating factors, IL-la, IL-10, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, leukocyte inhibitory factor (LIF), IFN-α, IFN-γ, TNF, TNF-α, TGF-β, G-CSF, M-CSF, GM-CSF; C-chemokines, CC-chemokines, CXCchemokines, CX3C-chemokines, MCP-1, MCP-2, MCP-3, MIP-lo/P, IP-10, MIG, IL-8, RANTES, lymphotactin and any combinations thereof. In some embodiments, the cytokine can be added exogenously *in vitro* or *in vivo.* In some embodiments, the cytokine can be produced *in situ, e.g.,* in response to or associated with a macrophage-associated condition and/or an implant.

The target stimulus can comprise a device with at least one coating of the composition described herein.

The target stimulus can comprise one or more embodiments of the compositions described herein. For example, the biomaterial (*e.g.,* silk fibroin-based matrix), and/or at least one immune cell-modulating agent (*e.g.,* macrophage-skewing agent) present in the biomaterial can each independently constitute a target stimulus. In some embodiments, the compositions described herein can comprise at least one immune cell-modulating agent (*e.g.,* macrophage-skewing agent) as a target stimulus, and at least one immune cell-modulating agent (*e.g.,* macrophage-skewing agent) as an agent that reduce or balance the effect of the target stimulus, *e.g.,* but not limited to, the inflammatory response induced by the target stimulus.

### Pharmaceutical compositions

The compositions described herein can be administered *in vivo.* Depending on the selected administration route, the compositions can be in any form, *e.g.,* but not limited to, a scaffold, a gel, a tablet, capsule, lozenge, suspension, free-flowing powder, or any combinations thereof. In some embodiments, the compositions described herein can be injectable. In some embodiments, the injectable compositions can further comprise a pharmaceutically acceptable carrier. The term "pharmaceutically acceptable," as used herein, refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "pharmaceutically acceptable carrier" refers to a pharmaceutically-acceptable material, composition or vehicle for administration of at least one immune cell-modulating agent (*e.g.,* macrophage-skewing agent) described herein. Pharmaceutically acceptable carriers can include any and all solvents, dispersion media, coatings, antibacterial and antifungal agents, isotonic and absorption delaying agents, and the like which are compatible with the activity of the immune cell-modulating agent and are physiologically acceptable to the subject. Some examples of materials which can serve as pharmaceutically-acceptable carriers include: (i) sugars, such as lactose, glucose and sucrose; (ii) starches, such as corn starch and potato starch; (iii) cellulose, and its derivatives, such as sodium carboxymethyl cellulose, methylcellulose, ethyl cellulose, microcrystalline cellulose and cellulose acetate; (iv) powdered tragacanth; (v) malt; (vi) gelatin; (vii) lubricating agents, such as magnesium stearate, sodium lauryl sulfate and talc; (viii) excipients, such as cocoa butter and suppository waxes; (ix) oils, such as peanut oil, cottonseed oil, safflower oil, sesame oil, olive oil, corn oil and soybean oil; (x) glycols, such as propylene glycol; (xi) polyols, such as glycerin, sorbitol, mannitol and polyethylene glycol (PEG); (xii) esters, such as ethyl oleate and ethyl laurate; (xiii) agar; (xiv) buffering agents, such as magnesium hydroxide and aluminum hydroxide; (xv) alginic acid; (xvi) pyrogen-free water; (xvii) isotonic saline; (xviii) Ringer's solution; (xix) ethyl alcohol; (xx) pH buffered solutions; (xxi) polyesters, polycarbonates and/or polyanhydrides; (xxii) bulking agents, such as polypeptides and amino acids (xxiii) serum component, such as serum albumin, HDL and LDL; (xxiv) C2-C12 alcohols, such as ethanol; and (xxv) other non-toxic compatible substances employed in pharmaceutical formulations. Wetting agents, coloring agents, release agents, coating agents, sweetening agents, flavoring agents, perfuming agents, preservative and antioxidants can also be present in the formulation. For compositions described herein to be administered orally, pharmaceutically acceptable carriers include, but are not limited to pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate, and lactose, while corn starch and alginic acid are suitable disintegrating agents. Binding agents may include starch and gelatin, while the lubricating agent, if present, will generally be magnesium stearate, stearic acid or talc. If desired, the tablets may be coated with a material such as glyceryl monostearate or glyceryl distearate, to delay absorption in the gastrointestinal tract.

Pharmaceutically acceptable carriers can vary in a composition described herein, depending on the administration route and formulation. The compositions described herein can be delivered via any administration mode known to a skilled practitioner. For example, the compositions described herein can be delivered in a systemic manner, via administration routes such as, but not limited to, oral, and parenteral including intravenous, intramuscular, intraperitoneal, intradermal, and subcutaneous. In some embodiments, the compositions described herein are in a form that is suitable for injection.

When administering parenterally, a composition described herein can be generally formulated in a unit dosage injectable form (solution, suspension, emulsion). The compositions suitable for injection include sterile aqueous solutions or dispersions. The carrier can be a solvent or dispersing medium containing, for example, water, cell culture medium, buffers (*e.g*., phosphate buffered saline), polyol (for example, glycerol, propylene glycol, liquid polyethylene glycol, and the like), suitable mixtures thereof. In some embodiments, the pharmaceutical carrier can be a buffered solution (*e.g*., PBS).

The compositions can also contain auxiliary substances such as wetting or emulsifying agents, pH buffering agents, gelling or viscosity enhancing additives, preservatives, colors, and the like, depending upon the route of administration and the preparation desired. Standard texts, such as "REMINGTON'S PHARMACEUTICAL SCIENCE", 17th edition, 1985, may be consulted to prepare suitable preparations, without undue experimentation. With respect to compositions described herein, however, any vehicle, diluent, or additive used should have to be biocompatible with the immune cell-modulating agents (*e.g*., macrophage-skewing agents) described herein. Those skilled in the art will recognize that the components of the compositions should be selected to be biocompatible with respect to the immune cell-modulating agents (*e.g*., macrophage-skewing agents). This will present no problem to those skilled in chemical and pharmaceutical principles, or problems can be readily avoided by reference to standard texts or by simple experiments (not involving undue experimentation).

In some embodiments, the compositions described herein can be formulated in an emulsion or a gel. Such gel compositions can be implanted locally in close proximity or at a target site (*e.g*., diseased or damaged tissue) of a subject.

The injectable compositions described herein can be administered with a delivery device, *e.g*., a syringe. In some embodiments, the compositions described herein can form part of an implant such as a microchip, a scaffold, an allograft tissue, or a medical device, *e.g.,* for sustained-release or controlled release of any composition described herein.

The silk fibroin-based matrix itself can be modified, as described earlier, to control its degradation and thus the release of immune cell-modulating agents, *e.g*., such that release occurs over a period of time ranging from hours to days, or months. In some embodiments, the compositions described herein can be combined with other types of delivery systems available and known to those of ordinary skill in the art. For example, one or more immune cell-modulating agents (*e.g.,* macrophage-skewing agents) can be encapsulated in a separate delivery system, *e.g.,* silk fibroin-based particles, polymer-based particles, and/or non-polymer based particles, prior to being dispersed in a biomaterial (*e.g*., silk fibroin-based matrix). Other types of delivery systems can include, for example, polymer-based systems such as polylactic and/or polyglycolic acids, polyanhydrides, polycaprolactones, copolyoxalates, polyesteramides, polyorthoesters, polyhydroxybutyric acid, and/or combinations thereof. Other examples include nonpolymer systems that are lipid-based including sterols such as cholesterol, cholesterol esters, and fatty acids or neuka1 fats such as mono-, di- and triglycerides; hydrogel release systems; liposome-based systems; phospholipid based- systems; silastic systems; peptide based systems; or partially fused implants. Specific examples include, but are not limited to, erosional systems in which the composition is contained in a form within a matrix, or diffusional systems in which an active component (*e.g*., an immune cell-modulating agent or a macrophage-skewing agent) controls the release rate. The formulation may be as, for example, microspheres, hydrogels, polymeric reservoirs, cholesterol matrices, or polymeric systems. In some embodiments, the system may allow sustained or controlled release of the composition to occur, for example, through control of the diffusion or erosion/degradation rate of the formulation containing the composition. In addition, a pump-based hardware delivery system can be used to deliver one or more embodiments of the compositions described herein. Use of a long-term sustained release formulations or implants can be particularly suitable for treatment of chronic conditions, such as diabetes, wound healing, and/or tissue regeneration. Long-term release, as used herein, means that a formulation or an implant is made and arranged to deliver compositions described herein at a therapeutic level for at least 30 days, or at least 60 days. In some embodiments, the long-term release refers to a formulation or an implant being configured to deliver an immune cell-modulating agent (*e.g.,* a macrophage-skewing agent) at a therapeutic level over several months.

### Some selected definitions

For convenience, certain terms employed herein, in the specification, examples and appended claims are collected here. Unless stated otherwise, or implicit from context, the following terms and phrases include the meanings provided below. Unless explicitly stated otherwise, or apparent from context, the terms and phrases below do not exclude the meaning that the term or phrase has acquired in the art to which it pertains. The definitions are provided to aid in describing particular embodiments, and are not intended to limit the claimed invention, because the scope of the invention is limited only by the claims. Unless otherwise defined, all technical and scientific terms used herein have the same meaning as commonly understood by one of ordinary skill in the art to which this invention belongs.

As used herein the term "comprising" or "comprises" is used in reference to compositions, methods, and respective component(s) thereof, that are essential to the method or composition, yet open to the inclusion of unspecified elements, whether essential or not.

As used herein the term "consisting essentially of" refers to those elements required for a given embodiment. The term permits the presence of elements that do not materially affect the basic and novel or functional characteristic(s) of that embodiment.

The term "consisting of" refers to compositions, methods, and respective components thereof as described herein, which are exclusive of any element not recited in that description of the embodiment.

As used in this specification and the appended claims, the singular forms "a," "an," and "the" include plural references unless the context clearly dictates otherwise. Thus for example, references to "the method" includes one or more methods, and/or steps of the type described herein and/or which will become apparent to those persons skilled in the art upon reading this disclosure and so forth. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "*e.g.*" is derived from the Latin exempli gratia, and is used herein to indicate a non-limiting example. Thus, the abbreviation "*e.g*." is synonymous with the term "for example."

Definitions of common terms in cell biology and molecular biology can be found in "The Merck Manual of Diagnosis and Therapy", 19th Edition, published by Merck Research Laboratories, 2006 (ISBN 0-911910-19-0); Robert S. Porter et al. (eds.), The Encyclopedia of Molecular Biology, published by Blackwell Science Ltd., 1994 (ISBN 0-632-02182-9); The ELISA guidebook (Methods in molecular biology 149) by Crowther J. R. (2000); Fundamentals of RIA and Other Ligand Assays by Jeffrey Travis, 1979, Scientific Newsletters; Immunology by Werner Luttmann, published by Elsevier, 2006. Definitions of common terms in molecular biology can also be found in Benjamin Lewin, Genes X, published by Jones & Bartlett Publishing, 2009 (ISBN-10: 0763766321); Kendrew et al. (eds.), , Molecular Biology and Biotechnology: a Comprehensive Desk Reference, published by VCH Publishers, Inc., 1995 (ISBN 1-56081-569-8) and Current Protocols in Protein Sciences 2009, Wiley Intersciences, Coligan et al., eds.

The terms "decrease," "reduce," "reduced", "reduction" , "decrease," and "inhibit" are all used herein generally to mean a decrease by a statistically significant amount relative to a reference. However, for avoidance of doubt, "reduce," "reduction" or "decrease" or "inhibit" typically means a decrease by at least 10% as compared to a reference level and can include, for example, a decrease by at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99% , up to and including, for example, the complete absence of the given entity or parameter as compared to a reference level, or any decrease between 10-99% as compared to a reference level.

The terms "increased" "increase" or "enhance" or "activate" or "promote" are all used herein to generally mean an increase by a statically significant amount; for the avoidance of any doubt, the terms "increased", "increase" or "enhance" or "activate" or "promote" means an increase of at least 10% as compared to a reference level, for example an increase of at least about 20%, or at least about 30%, or at least about 40%, or at least about 50%, or at least about 60%, or at least about 70%, or at least about 80%, or at least about 90% or up to and including a 100% increase or any increase between 10-100% as compared to a reference level, or at least about a 2-fold, or at least about a 3-fold, or at least about a 4-fold, or at least about a 5-fold or at least about a 10-fold increase, or any increase between 2-fold and 10-fold or greater as compared to a reference level.

As used herein, the term "proteins" and "polypeptides" are used interchangeably herein to designate a series of amino acid residues connected to the other by peptide bonds between the alpha-amino and carboxy groups of adjacent residues. The terms "protein", and "polypeptide", which are used interchangeably herein, refer to a polymer of protein amino acids, including modified amino acids (*e.g*., phosphorylated, glycated, glycosylated, etc.) and amino acid analogs, regardless of its size or function. "Protein" and "polypeptide" are often used in reference to relatively large polypeptides, whereas the term "peptide" is often used in reference to small polypeptides, but usage of these terms in the art overlaps. The terms "protein" and "polypeptide" are used interchangeably herein when referring to a gene product and fragments thereof. Thus, exemplary polypeptides or proteins include gene products, naturally occurring proteins, homologs, orthologs, paralogs, fragments and other equivalents, variants, fragments, and analogs of the foregoing.

The term "nucleic acids" used herein refers to polynucleotides such as deoxyribonucleic acid (DNA), and, where appropriate, ribonucleic acid (RNA), polymers thereof in either single- or double-stranded form. Unless specifically limited, the term encompasses nucleic acids containing known analogs of natural nucleotides, which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g*., degenerate codon substitutions) and complementary sequences, as well as the sequence explicitly indicated. Specifically, degenerate codon substitutions may be achieved by generating sequences in which the third position of one or more selected (or all) codons is substituted with mixed-base and/or deoxyinosine residues (Batzer, et al., Nucleic Acid Res. 19:5081 (1991); Ohtsuka, et al., J. Biol. Chem. 260:2605-2608 (1985), and Rossolini, et al., Mol. Cell. Probes 8:91-98 (1994)). The term "nucleic acid" should also be understood to include, as equivalents, derivatives, variants and analogs of either RNA or DNA made from nucleotide analogs, and, single (sense or antisense) and double-stranded polynucleotides.

The term "short interfering RNA" (siRNA), also referred to herein as "small interfering RNA" is defined as an agent which functions to inhibit expression of a target gene, *e.g.,* by RNAi. An siRNA can be chemically synthesized, it can be produced by *in vitro* transcription, or it can be produced within a host cell. siRNA molecules can also be generated by cleavage of double stranded RNA, where one strand is identical to the message to be inactivated. The term "siRNA" refers to small inhibitory RNA duplexes that induce the RNA interference (RNAi) pathway. These molecules can vary in length (generally 18-30 base pairs) and contain varying degrees of complementarity to their target mRNA in the antisense strand. Some, but not all, siRNA have unpaired overhanging bases on the 5' or 3' end of the sense 60 strand and/or the antisense strand. The term "siRNA" includes duplexes of two separate strands, as well as single strands that can form hairpin structures comprising a duplex region.

The term "shRNA" as used herein refers to short hairpin RNA which functions as RNAi and/or siRNA species but differs in that shRNA species are double stranded hairpin-like structure for increased stability. The term "RNAi" as used herein refers to interfering RNA, or RNA interference molecules are nucleic acid molecules or analogues thereof for example RNA-based molecules that inhibit gene expression. RNAi refers to a means of selective post-transcriptional gene silencing. RNAi can result in the destruction of specific mRNA, or prevents the processing or translation of RNA, such as mRNA.

The term "enzymes" as used here refers to a protein molecule that catalyzes chemical reactions of other substances without it being destroyed or substantially altered upon completion of the reactions. The term can include naturally occurring enzymes and bioengineered enzymes or mixtures thereof. Examples of enzyme families include kinases, dehydrogenases, oxidoreductases, GTPases, carboxyl transferases, acyl transferases, decarboxylases, transaminases, racemases, methyl transferases, formyl transferases, and ketodecarboxylases.

As used herein, the term "aptamers" means a single-stranded, partially single-stranded, partially double-stranded or double-stranded nucleotide sequence capable of specifically recognizing a selected non-oligonucleotide molecule or group of molecules. In some embodiments, the aptamer recognizes the non-oligonucleotide molecule or group of molecules by a mechanism other than Watson-Crick base pairing or triplex formation. Aptamers can include, without limitation, defined sequence segments and sequences comprising nucleotides, ribonucleotides, deoxyribonucleotides, nucleotide analogs, modified nucleotides and nucleotides comprising backbone modifications, branchpoints and nonnucleotide residues, groups or bridges. Methods for selecting aptamers for binding to a molecule are widely known in the art and easily accessible to one of ordinary skill in the art.

As used herein, the term "antibody" or "antibodies" refers to an intact immunoglobulin or to a monoclonal or polyclonal antigen-binding fragment with the Fc (crystallizable fragment) region or FcRn binding fragment of the Fc region. The term "antibodies" also includes "antibody-like molecules", such as fragments of the antibodies, *e.g*., antigen-binding fragments. Antigen-binding fragments can be produced by recombinant DNA techniques or by enzymatic or chemical cleavage of intact antibodies. "Antigen-binding fragments" include, inter alia, Fab, Fab', F(ab')₂, Fv, dAb, and complementarity determining region (CDR) fragments, single-chain antibodies (scFv), single domain antibodies, chimeric antibodies, diabodies, and polypeptides that contain at least a portion of an immunoglobulin that is sufficient to confer specific antigen binding to the polypeptide. Linear antibodies are also included for the purposes described herein. The terms Fab, Fc, pFc', F(ab') 2 and Fv are employed with standard immunological meanings (Klein, Immunology (John Wiley, New York, N.Y., 1982); Clark, W. R. (1986) The Experimental Foundations of Modern Immunology (Wiley & Sons, Inc., New York); and Roitt, I. (1991) Essential Immunology, 7th Ed., (Blackwell Scientific Publications, Oxford)). Antibodies or antigen-binding fragments specific for various antigens are available commercially from vendors such as R&D Systems, BD Biosciences, e-Biosciences and Miltenyi, or can be raised against these cell-surface markers by methods known to those skilled in the art.

As used herein, the term "Complementarity Determining Regions" (CDRs; i.e., CDR1, CDR2, and CDR3) refers to the amino acid residues of an antibody variable domain the presence of which are necessary for antigen binding. Each variable domain typically has three CDR regions identified as CDR1, CDR2 and CDR3. Each complementarity determining region may comprise amino acid residues from a "complementarity determining region" as defined by Kabat (i.e. about residues 24-34 (L1), 50-56 (L2) and 89-97 (L3) in the light chain variable domain and 31-35 (HI), 50-65 (H2) and 95-102 (H3) in the heavy chain variable domain; Kabat et al. , Sequences of Proteins of Immunological Interest, 5th Ed. Public Health Service, National Institutes of Health, Bethesda, Md. (1991)) and/or those residues from a "hypervariable loop" (i.e. about residues 26-32 (L1), 50-52 (L2) and 91-96 (L3) in the light chain variable domain and 26-32 (HI), 53-55 (H2) and 96-101 (H3) in the heavy chain variable domain; Chothia and Lesk J. Mol. Biol. 196:901-917 (1987)). In some instances, a complementarity determining region can include amino acids from both a CDR region defined according to Kabat and a hypervariable loop.

The expression "linear antibodies" refers to the antibodies described in Zapata et al., Protein Eng., 8(10):1057-1062 (1995). Briefly, these antibodies comprise a pair of tandem Fd segments (VH -CH1-VH-CH1) which, together with complementary light chain polypeptides, form a pair of antigen binding regions. Linear antibodies can be bispecific or monospecific.

The expression "single-chain Fv" or "scFv" antibody fragments, as used herein, is intended to mean antibody fragments that comprise the VH and VL domains of antibody, wherein these domains are present in a single polypeptide chain. Preferably, the Fv polypeptide further comprises a polypeptide linker between the VH and VL domains which enables the scFv to form the desired structure for antigen binding. (Plückthun, The Pharmacology of Monoclonal Antibodies, vol. 113, Rosenburg and Moore eds., Springer- Verlag, New York, pp. 269-315 (1994)).

The term "diabodies," as used herein, refers to small antibody fragments with two antigen-binding sites, which fragments comprise a heavy-chain variable domain (VH) Connected to a light-chain variable domain (VL) in the same polypeptide chain (VH - VL). By using a linker that is too short to allow pairing between the two domains on the same chain, the domains are forced to pair with the complementary domains of another chain and create two antigen-binding sites. (EP 404,097; WO 93/11161; Hollinger et ah, Proc. Natl. Acad. Sd. USA, P0:6444-6448 (1993)).

As used herein, the term "small molecules" refers to natural or synthetic molecules including, but not limited to, peptides, peptidomimetics, amino acids, amino acid analogs, polynucleotides, polynucleotide analogs, aptamers, nucleotides, nucleotide analogs, organic or inorganic compounds (i.e., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

The term "bacteria" as used herein is intended to encompass all variants of bacteria, for example, prokaryotic organisms and cyanobacteria. Bacteria are small (typical linear dimensions of around 1 m), non-compartmentalized, with circular DNA and ribosomes of 70S.

As used herein, the term "antigens" refers to a molecule or a portion of a molecule capable of being bound by a selective binding agent, such as an antibody, and additionally capable of being used in an animal to elicit the production of antibodies capable of binding to an epitope of that antigen. An antigen may have one or more epitopes. The term "antigen" can also refer to a molecule capable of being bound by an antibody or a T cell receptor (TCR) if presented by MHC molecules. The term "antigen", as used herein, also encompasses T-cell epitopes. An antigen is additionally capable of being recognized by the immune system and/or being capable of inducing a humoral immune response and/or cellular immune response leading to the activation of B- and/or T-lymphocytes. This may, however, require that, at least in certain cases, the antigen contains or is linked to a Th cell epitope and is given in adjuvant. An antigen can have one or more epitopes (Band T-epitopes). The specific reaction referred to above is meant to indicate that the antigen will preferably react, typically in a highly selective manner, with its corresponding antibody or TCR and not with the multitude of other antibodies or TCRs which may be evoked by other antigens. Antigens as used herein may also be mixtures of several individual antigens.

As used herein, the term "viruses" refers to an infectious agent composed of a nucleic acid encapsidated in a protein. Such infectious agents are incapable of autonomous replication (i.e., replication requires the use of the host cell's machinery). Viral genomes can be single-stranded (ss) or double-stranded (ds), RNA or DNA, and can or cannot use reverse transcriptase (RT). Additionally, ssRNA viruses can be either sense (+) or antisense (-). Exemplary viruses include, but are not limited to, dsDNA viruses (*e.g*., Adenoviruses, Herpesviruses, Poxviruses), ssDNA viruses (*e.g.,* Parvoviruses), dsRNA viruses (*e.g.,* Reoviruses), (+)ssRNA viruses (*e.g*., Picornaviruses, Togaviruses), (-)ssRNA viruses(*e.g*., Orthomyxoviruses, Rhabdoviruses), ssRNA-RT viruses, i.e., (+)sense RNA with DNA intermediate in life-cycle (*e.g.,* Retroviruses), and dsDNA-RT viruses (*e.g*., Hepadnaviruses). In some embodiments, viruses can also include wild-type (natural) viruses, killed viruses, live attenuated viruses, modified viruses, recombinant viruses or any combinations thereof. Other examples of viruses include, but are not limited to, enveloped viruses, respiratory syncytial viruses, non-enveloped viruses, bacteriophages, recombinant viruses, and viral vectors. The term "bacteriophages" as used herein refers to viruses that infect bacteria.

The term "therapeutic agents" is art-recognized and refers to any chemical moiety that is a biologically, physiologically, or pharmacologically active substance that acts locally or systemically in a subject. Examples of therapeutic agents, also referred to as "drugs", are described in well-known literature references such as the Merck Index, the Physicians Desk Reference, and The Pharmacological Basis of Therapeutics, and they include, without limitation, medicaments; vitamins; mineral supplements; substances used for the treatment, prevention, diagnosis, cure or mitigation of a disease or illness; substances which affect the structure or function of the body; or pro-drugs, which become biologically active or more active after they have been placed in a physiological environment. Various forms of a therapeutic agent may be used which are capable of being released from the subject composition into adjacent tissues or fluids upon administration to a subject. Examples include steroids and esters of steroids (*e.g*., estrogen, progesterone, testosterone, androsterone, cholesterol, norethindrone, digoxigenin, cholic acid, deoxycholic acid, and chenodeoxycholic acid), boron-containing compounds (*e.g*., carborane), chemotherapeutic nucleotides, drugs (*e.g.,* antibiotics, antivirals, antifungals), enediynes (*e.g.,* calicheamicins, esperamicins, dynemicin, neocarzinostatin chromophore, and kedarcidin chromophore), heavy metal complexes (*e.g.,* cisplatin), hormone antagonists (*e.g.,* tamoxifen), non-specific (non-antibody) proteins (*e.g.,* sugar oligomers), oligonucleotides (*e.g.,* antisense oligonucleotides that bind to a target nucleic acid sequence (*e.g*., mRNA sequence)), peptides, proteins, antibodies, photodynamic agents (*e.g.,* rhodamine 123), radionuclides (*e.g.,* 1-131, Re-186, Re-188, Y-90, Bi-212, At-211, Sr-89, Ho-166, Sm-153, Cu-67 and Cu-64), toxins (*e.g*., ricin), and transcription-based pharmaceuticals.

As used herein, a "subject" means a human or animal. Usually the animal is a vertebrate such as a primate, rodent, domestic animal or game animal. Primates include chimpanzees, cynomologous monkeys, spider monkeys, and macaques, *e.g*., Rhesus. Rodents include mice, rats, woodchucks, ferrets, rabbits and hamsters. Domestic and game animals include cows, horses, pigs, deer, bison, buffalo, feline species, *e.g.,* domestic cat, canine species, *e.g.,* dog, fox, wolf, avian species, *e.g.,* chicken, emu, ostrich, and fish, *e.g.,* trout, catfish and salmon. Patient or subject includes any subset of the foregoing, *e.g*., all of the above. In certain embodiments, the subject is a mammal, *e.g.,* a primate, *e.g.,* a human.

Preferably, the subject is a mammal. The mammal can be a human, non-human primate, mouse, rat, dog, cat, horse, or cow, but is not limited to these examples.

Other than in the operating examples, or where otherwise indicated, all numbers expressing quantities of ingredients or reaction conditions used herein should be understood as modified in all instances by the term "about." The term "about" when used in connection with percentages can mean ±1%.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments can perform functions in a different order, or functions can be performed substantially concurrently. The teachings of the disclosure provided herein can be applied to other procedures or methods as appropriate. The various embodiments described herein can be combined to provide further embodiments. Aspects of the disclosure can be modified, if necessary, to employ the compositions, functions and concepts of the above references and application to provide yet further embodiments of the disclosure. These and other changes can be made to the disclosure in light of the detailed description.

Specific elements of any of the foregoing embodiments can be combined or substituted for elements in other embodiments. Furthermore, while advantages associated with certain embodiments of the disclosure have been described in the context of these embodiments, other embodiments can also exhibit such advantages, and not all embodiments need necessarily exhibit such advantages to fall within the scope of the disclosure.

All patents and other publications identified serve the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

Materials, procedures and considerations necessary to understand and use the disclosed methods are described herein, as are experimental results and non-limiting examples that demonstrate and illustrate various embodiments of the methods and compositions described herein. Some embodiments of various aspects described herein are further illustrated by the following examples which should not be construed as limiting.

### EXAMPLES

Described herein are experiments investigating the role of morphological and chemical characteristics of a biomaterial play in modulating the inflammatory response at the level of the implant. The modulation of the inflammatory response induced by three different sizes of silk fibroin particles (2 µm, 1 µm, and less than 500 nm) was evaluated. Three antiinflammatory drugs were investigated to reduce the inflammatory response: dexamethasone, a well-known anti-inflammatory glucocorticoid; simvastin, a hypolipedimic drug; and nicotine, a psychostimulant and painkiller that has recently been shown to have anti-inflammatory properties. These drugs have distinct mechanisms of action and secondary effects. To assess the inflammatory response, *in vitro* studies on immortalized macrophages (*e.g*., THP1), primary macrophages, and dendritic cells (DCs) differentiated from monocytes extracted from peripheral blood were conducted. Cytokine and chemokine production, gene expression, and cell morphology were evaluated.

One aim of the experiments described herein was to investigate the effect of silk particle size (3 sizes: 2µm, 1 µm, and <500nm) on the inflammatory response *in vitro* by evaluating Mϕ (stand for "macrophage") behavior (*e.g*., but not limited to, morphology, cytokine secretion and expression) and *in vivo* by identifying inflammatory cells using tissue histology and cell sorting. An additional aim focused on modulating the inflammatory response induced by the particles using anti-inflammatory drugs and peptides tethered to the particles. The behavior of Mϕs and DCs *in vitro* was observed by examining their morphology, cytokine secretion, and gene expression.

Described herein is information about the inflammatory response to silk fibroin particles *in vitro* and *in vivo.* Further described herein is the functionalization of silk fibroin particles to reduce their inflammatory effect and promote healing and tissue remodeling. *In vitro* experiments were carried out on THP1 differentiated Mϕs (human monocytic leukemia cell line) and on human primary Mϕs and DCs isolated from human peripheral blood mononuclear cells (PBMCs).

### Example 1. Exemplary materials and methods for assessing effects of candidate drugs associated with silk matrix on the inflammatory response

*Methods.* Experiments were designed to assess the effect of the drugs associated with silk particles on the inflammatory response. The drugs and a macrophage-skewing agent (*e.g.,* LPS) were added to the media to simulate an infectious environment that favors activation to a proinflammatory phenotype. In some embodiments, silk fibroin particles and anti-inflammatory drugs can be blended together. In some embodiments, the anti-inflammatory drugs can be absorbed on silk fibroin particles. In these embodiments, the drugs can be released slowly from the silk into the medium, and thus cell response can be monitored at later time points. In some embodiments, the inflammatory drugs can be non-covalently attached to the silk fibroin particles or films.

As the silk fibroin-based matrix (*e.g*., silk fibroin particles) alone generally induce a low level of inflammatory response, increasing the inflammatory response facilitated evaluation of the drug effects. In addition, a negative and a positive control were added to the experiments to compare and evaluate different experimental groups. The negative control contained the cell media only, and the positive control contained the cell media contained a macrophage -skewing agent (*e.g.,* LPS) but no anti-inflammatory drugs. The following analyses were performed at two time points, *e.g.,* 1 day and 3 days, to evaluate the immediate and delayed drug effects.

The inflammatory response was analyzed on various silk fibroin-based matrix, *e.g*., silk fibroin particles and films. The experiments conducted on silk fibroin films served as controls because the inflammatory response induced by them has been well-characterized. This established data permitted comparison of the effect of shape on the inflammatory reaction and its ability to be modulated.

*Cytokine and chemokine expression: ELISA and RT-PCR method.* To assess inflammatory response, a large screen of cytokines and chemokines expressed and produced by THP1 differentiated cells and primary Mϕs and DCs were analyzed. Their production and release into the media environment were evaluated using an ELISA sandwich technique. This widely used technique employs monoclonal antibodies, which results in the sensitivity of the assay being 2 to 5 times greater than the direct or indirect ELISA techniques. TNF-α cytokine release was selected in the experiments described herein because it is the common proinflammatory cytokine produced by Mϕs as well as DCs. However, other inflammation-associated cytokines can also be measured.

Without wishing to be bound by theory, the gene expression of the cytokines and chemokines was evaluated by RT-PCR because it allows the response to be evaluated at an upstream point, before the protein is produced and secreted. Thus, cytokine and chemokine expression can be correlated to protein production and the cellular response. It should be noted that cytokine gene expression does not necessary mean cytokine production. For example, cells can produce many genes which are transcribed, stored as an inactive form, and then later released quickly if needed. For Mcps, the TNF-α gene expression was analyzed and correlated with its secretion. Gene expression of other cytokines were also analyzed. For example, TGF-β gene expression was analyzed, where TGF-β is a cytokine that promotes cell proliferation and is more involved in the healing and remodeling pathways. IL-10 was analyzed, where IL-10 is an anti-inflammatory cytokine and is expressed upon exposure to simvastatin and dexamethasone stimulation. For DCs, IL-1β gene expression was assessed, where IL-1β is a pro-inflammatory cytokine secreted in higher amounts by DCs. IL-1RA is an antagonist receptor to IL-1 so that it can generally counteract IL-1 β gene expression, thus gene expression of IL-1RA and IL-1β can be analyzed. IL-8 gene expression was analyzed, where IL-8 is a chemokine which is involved in the mechanism of the tolerance toward the material involving DCs. Table 2 below is a summary of some known cytokine and chemokine functions.

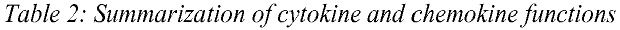

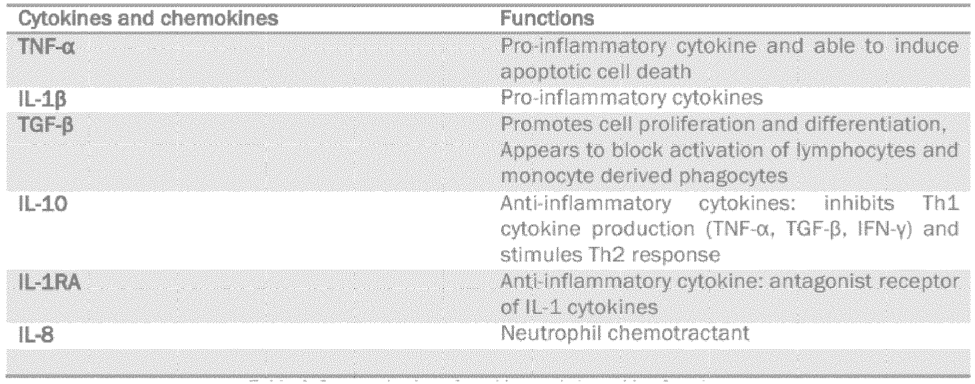

*Cell morphology and particle size: SEM method.* As Mϕs and DCs can act against small foreign materials (up to 20 µm) (69) by phagocytosis, the changes in their cell morphology were evaluated. Indeed, when the Mϕs and DCs are activated and start to phagocytose the foreign material, their morphology changes from round cells to extended cells with pseudopods. Activated Mϕs and DCs produce extracellular matrix (ECM), *e.g.,* to more easily move and/or adhere to the surrounding material. During the inflammatory response, Mϕs can fuse together, forming giant cells, to engulf larger materials. This morphology is one of the characteristics of activated macrophages. Cell morphology can be evaluated by any methods known in the art. For example, SEM imaging can be used to assess the cell morphology and correlation to cytokine expression and secretion. SEM imaging can also be used to assess cell viability, as it can provide information as to whether a decrease in cell number is due to death or lack of cellular adhesion. Further, SEM imaging can allow examination of the quality of the silk fibroin particles in term of size and agglomeration.

In some embodiments, phagocytosis and/or degradation of the silk fibroin particles can be characterized using any known methods in the art.

*Materials.* Silkworm cocoons used in the Examples described herein were supplied by Tajima Shoji Co. Ltd (Yokohama, Japan). RPMI 1640 medium, fetal bovine serum (FBS), DMEM medium, penicillin-streptomycin (Pen-Strep), phosphate buffered saline (PBS), Dulbecco's modified PBS, and ultra purified water were purchased from Invitrogen (Carlsbad, CA). Lympholyte® was purchased from Accurate Chemical (WESTBURY, NY). Lipopolysaccharide (LPS) from Escherichia coli EH100, Ethylendiamine tetra acetic acid (EDTA), sodium carbonate, and lithium bromide were purchased from Sigma Aldrich (St. Louis, MO). Dexamethasone, nicotine (PESTANAL®) and simvastatin were also purchased from Sigma Aldrich (Saint Louis, MO), and stock solutions of 1mg/ml, 50mg/ml and 5mM µg/ml respectively were stored at -20°C.

In some embodiments, in addition to anti-inflammatory molecules such as dexamethasone, nicotine, and simvastatin, anti-inflammatory peptides (*e.g.,* but not limited to anti-microbial LL-37 peptides, and apolipoprotein E) can also be used.

*Silk preparation.* About 5 g of *Bombyx mori* cocoons were first cut in small pieces and boiled for ∼30 min in an aqueous solution of ∼0.02 M sodium carbonate to remove the glue-like sericin protein as previously described (70). Then, the extracted silk fibroin was rinsed thoroughly 3 times every 20 min in distilled water to remove the sodium carbonate. After at least about 12 h of air drying, the extracted silk fibroin was dissolved in ∼9.3 M LiBr solution at 60°C for 4 h minimum. Once completely dissolved, the solution was dialyzed against distilled water using Slide-a-lyzer dialysis cassettes (MWCO 3500, Pierce) for 3 days to remove the salt (thrice the first day, twice the second, and once the third). The solution was then centrifuged twice at 10,000 rpm for 20 min at about 5-10 °C to remove silk aggregates as well as debris from original cocoons. The final concentration of silk fibroin aqueous solution was determined by weighing 1 ml of the solution, drying, and comparing against its residual solid after drying at 60°C. The aqueous silk fibroin solution was approximately 8 % (w/v). The aqueous silk fibroin solution appeared honey in color, which indicates a good extraction. The stock silk solution was stored at ∼4°C and was stable for at least about 4 weeks.

*Silk particles preparation.* Silk fibroin particles were prepared by using an aqueous salt out process with potassium phosphate solution that induces a phase separation, as previously described (27). The size of the silk fibroin particles can be directly related to the concentration of silk fibroin aqueous solution used. Different sizes of silk fibroin particles were evaluated, *e.g*., ∼2 µm sized-particles called SP1 (∼1 % of silk fibroin stock solution diluted in distilled water), 1 µm called SP0.5 (∼0.5 % of silk fibroin stock solution diluted in distilled water), and less than 500 nm called SP0.1 (∼0.1 % of silk fibroin stock solution diluted in distilled water). For example, a solution of about 1.5 M K₂HPO₄ (pH ∼8) was prepared, and ∼10 mL was poured into ∼15 mL falcon tubes. ∼2 mL of silk fibroin solution with the appropriate concentration was prepared by diluting the stock solution with distilled water. The silk fibroin solution was then added quickly, using a pipette, into the potassium phosphate solution in volumetric ratios of 1:5. The resulting silk fibroin particles were stored for ∼2 h to overnight at ∼4°C. The silk fibroin particles were rinsed to remove solvents used in the particle synthesis. For example, the solutions containing the silk fibroin particles were centrifuged four times: three times in distilled water and once in ∼70 % ethanol at 2500xg for 15 min. After each centrifugation, the supernatant was removed and the silk particles were redispersed in the appropriate solution. After the last centrifugation in ethanol, the supernatant was carefully removed, *e.g*., with a pipette, and the particle pellets were dried at ∼70°C. When the pellets were dry, they were weighed and an adequate volume of 70 % ethanol was added to make a final concentration of 20 mg/mL for each solution of silk fibroin particles. The solutions were stored at room temperature in tightly capped tubes to avoid evaporation of the ethanol and to prevent contamination. At least 4 h hours before the cell seeding, the 30 ul of the particle solution were plated in 96-well tissue plate allowed to dry under the tissue culture hood. Once completely dry, the plates containing the silk particles were ready for cell seeding.

*Silk film preparation.* From the stock silk fibroin solution, a ∼2 % solution was made in distilled water. The day before the cell seeding, ∼30 µl of silk fibroin solution was plated in the wells of a 96-well tissue plate allowed to dry under the tissue culture hood overnight. The following day, ∼50 µl of methanol was added to each well, allowed to incubate for about 0.5-1 h under the hood to crosslink the silk proteins by β-sheet formation. After removing the excess methanol, the silk films were sterilized under by exposing to UV light for about 30 min and allowed to dry for about 4 h under the hood, after which they were ready for cell seeding.

*THP1 cell culture.* A human monocytic leukemia cell line THP-1 (American Type Culture Collection, Manassas, VA) was cultured at 5 % CO₂ in RPMI 1640 medium supplemented with 10% fetal bovine serum (Invitrogen) and antibiotics (50 U/L penicillin G (sodium salt), 50 mg/mL streptomycin) (Invitrogen). For the experiments described herein, THP-1 cells were initially seeded into a 96-well microplate at similar densities (for example, around 1x10⁶ cells/well (∼200µl)) and incubated at 5 % CO₂ and 37°C. The number of cells was determined, *e.g*., by counting the cells at the beginning of the experiment and by using the alamar blue method after the seeding. On the day the cells were seeded at the appropriate number, phorbol 12-myristate 13-acetate (PMA) was added to the media at a final concentration of about 100 ng/ml to differentiate the THP-1 monocytes into THP-1 macrophages. The plates were then incubated for about 48 h at 37°C and ∼5 % CO₂.

*Primary monocytes extraction and differentiation into Mϕs and DCs.* ∼50 mL of peripheral blood from donors (Blood Component company, Boston, MA) were diluted in DPBS/EDTA *(e.g.,* in a ratio of about 5:1). Then, a primary Ficoll gradient was realized, *e.g.,* by aid of glass pipette placed at the bottom of a tube. Lympholyte® (ratio 2:1) was poured very slowly into the pipette to obtain two clearly separated phases. The blend was then centrifuged at ∼400xg for ∼20 min at room temperature. The white cell phase between the plasma and the Ficoll phase was collected, diluted with DPBS with ∼1mM of EDTA (v/v) and then centrifuged at ∼150xg for about 10 min at room temperature, again. Another rinse was made following the same conditions as the previous step. Subsequently, the pellet was resuspended in RPMI 1640 media and a second gradient was realized with Percoll (v/v) to extract the monocytes, following the same method as previously described. The blend was then centrifuged at ∼400xg for ∼30 min at room temperature. The white ring containing the monocytes was collected, diluted in iced cold DPBS with ∼1mM of EDTA (v/v), and centrifuged at ∼150xg for ∼10 min at ∼4°C using a centrifuge break. The pellet was then resuspended in iced cold PBS and centrifuged at 150xg 10 min at 4°C. Finally, the pellet was resuspended in RPMI 1640 medium supplemented with 10 % fetal bovine serum (Invitrogen) and antibiotics (50 U/L penicillin G (sodium salt), 50 mg/mL streptomycin) (Invitrogen). The cells were seeded at 50,000 cells per well in 200 µL of media and incubated at 5% CO₂ and 37°C. To differentiate the monocytes into macrophages and dendritic cells, the media was supplemented with GM-CSF, and IL-4 plus GM-CSF, respectively. The plates were then incubated for about 48 h at 37°C and 5 % CO₂.

*Lipopolysaccharide (LPS) and drug stimulation (Dexamethasone, Simvastatin, Nicotine).* For each type of cells (*e.g.,* macrophages and dendritic cells), after 48 h of differentiation, the culture media was replaced with RPMI 1640 media (Negative control) or RPMI 1640 media supplemented with a macrophage-skewing agent (*e.g.,* LPS at 200 ng/ml) (Positive control) or RPMI 1640 media supplemented with a macrophage-skewing agent *(e.g.,* LPS at 200 ng/ml) and the different concentrations of dexamethasone, simvastatin, or nicotine. The plates were then incubated at 37°C in 5% CO₂ for ~24 h and ~72 h. See **Figure 1** for a schematic of the 96-well tissue plate setup. The first two rows in the plate comprised the negative and positive controls, respectively. One day and three days after the macrophage-skewing agent *(e.g.,* LPS) and drug stimulation, macrophage tumor necrosis factor α (TNF-α) secretion in the medium was determined, *e.g*., with an ELISA kit (R&D Systems, Minneapolis, MN) with a detection limit of 5 pg/mL according to manufacturer's instructions. The kit uses a sandwich ELISA method, where, for example, a mouse monoclonal antibody to human TNF-α is immobilized on a 96-well microplate to bind to TNF-α contained in the cell culture supernatants.

Before adding the supernatant, a solution of 1 % Bovine Serum Albumin (BSA) in PBS was incubated in the wells to block the nonspecific binding to the plates sites. After incubation of the supernatant and washing, a biotinylated goat polyclonal anti-human TNF-α antibody was added followed by streptavidin conjugated to horseradish-peroxidase. The samples were washed, the substrate was added, and the wells were incubated for 20 min. The reaction was stopped with the addition of 1 M of sulfuric acid (H₂SO₄). The product of the reaction was measured at 450 nm (normalized for plate absorbance at 570 nm) and converted to a concentration of TNF-α in pg/mL using a standard curve linear over the concentrations ranging from about 62.5 to about 2,000 pg/mL. TNF-α release was normalized to sample volume and cell count as determined, *e.g*., by the AlamarBlue assay (Invitrogen, Carlsbad, CA). The AlamarBlue reagent is a vital reagent which is transformed into a pink color by the viable cells, which enabled quantification of the number of cells in reference to a standard. The test does not generally differentiate between a decrease in the number of cells due to cell death and the lack of cellular adhesion. All cell culture measurements were carried out with three replicates each.

*RNA isolation and real time reverse transcriptase polymerase chain reaction (RT-PCR).* One day and three days after the macrophage-skewing agent (*e.g.,* LPS) and drug stimulation, TNF-α, TGF-1β, and IL-10 gene expression were analyzed from THP1 activated macrophages; TNF-α, IL-1RN, and IL10 from primary Mcps; and IL-1RN, IL-1βand IL-8 from primary DCs. Following AlamarBlue assay, the RNA was isolated from the cells using RNeasy® Mini Kit according to manufacturer's protocol (Qiagen Valencia, CA). The RNA samples were reversed transcribed using the high capacity cDNA reverse transcription kit (Applied biosystems). The expression of the genes of interest were quantified by PCR using the Taqman® universal PCR master mix and the primers as given in Table 3 (Applied Biosystems, Carlsbad, CA).

**Table 3: Primers and their Applied Biosystems reference numbers**

| Primers | References |
|---|---|
| TNF-α | Hs00174128_m1 |
| IL-1β | Hs01555410_m1 |
| IL-1RN | Hs00893626_m1 |
| TGF-β1 | Hs00998130_m1 |
| IL-10 | Hs00893626_m1 |
| IL-8 | Hs99999034_m1 |
| ACTIN-β (housekeeping gene) | Hs99999906_m1 |

PCR reaction conditions were ∼2 min at ∼50 °C, ∼10 min at ∼95 °C, and then about 50 cycles at ∼95 °C for ∼15 s, and ∼1 min at ∼60 °C. The expression data were normalized to the expression of actin-β, a housekeeping gene. Primer sequences for the human actin-β were:
5'-GTGGGCCGCTCTAGGCACCAA
3'-CTCTTTGATGTCACGCACGATTTC

*Scanning Electron Microscopy (SEM).* After Alamar Blue essay, ∼100 µl of glutaraldehyde diluted at a volumetric ratio of ∼1:25 was added to the well and incubated overnight at ∼4°C. The samples were then dehydrated in aqueous ethanol solutions, with increasing concentrations of ethanol, for about 15 min each (10 %, 30 %, 60%, 80 %, 90 %, and two baths of 100 % ethanol). The samples were dried overnight and then cut and adhered to a SEM sample stub using carbon tape. The samples were then sputter coated with Platinum/Palladium (Pt/Pd) at 40 mA for approximately 60 s. Imaging was performed in ZEISS Supra 55 VP SEM and ZEISS.

In some embodiments, LDH cytotoxicity test can be performed (*e.g*., in addition to Alamar Blue and SEM imaging) to generate quantitative results for cytotoxicity.

*In vivo experiments:* Various size of the silk fibroin particles (*e.g.,* ∼2 µm, ∼1 µm, or less than 500 nm) prepared in PBS were injected subcutaneously in the back of the mice. Three replicates for each size were used for cell sorting analyses (*e.g*., FACS analysis) and for histological analyses. These analyses were performed at three time points: ∼1 day, ∼7 days, and ∼3 days after a second injection to evaluate the acute and chronic inflammatory responses, as well as the immunological memory effect involved, respectively.

*Statistical analysis.* Statistical analysis of data was performed by T-test of two samples assuming unequal variance. p<0.05 was considered statistically significant. * denotes p<0.05 and ** denotes p<0.01.

### Example 2. Cytokine expression and secretion from differentiated THP1 monocytes seeded on ∼2 µm-sized silk fibroin particles (SP1) and modulation with antiinflammatory drugs.

**Figures 2A-2C**, **3A-3C**, **4A-4C**, and **6** display the experimental results of differentiated THP1 monocytes seeded and cultured on SP1 particles (∼2 µm). **Figure. 2A****,** **3A** and **4A** show the concentration of TNF-α released per cell in pg/ml, and cell viability expressed as a percentage of the negative control (media only) when the THP1 macrophages were cultured in different anti-inflammatory drugs (*e.g*., dexamethasone, simvastatin, nicotine). The ELISA and Alamar Blue results are the average of about 20 replicates wells. **Figures 2B-2C****,** **3B-3C**, and **4B-4C** show TNF-α, TGF β, and IL-10 gene expression expressed as a fold change relative to the positive control (media with a macrophage-skewing agent *e.g.,* LPS) when the THP1 macrophages were cultured for about 1 day and 3 days, respectively. The RT-PCR results are the average of about 15 samples.

The cell viability of and cytokine release from THP1 macrophages seeded on SP1 particles stimulated with dexamethasone are shown in **Figures 2A-2C****.** The cell viability and TNF-α release at 1 day and 3 days are combined in **Figure 2A. Figures 2B** and **2C** display TNFα, TGF-β, and IL-10 gene expression at 1 and 3 days.

*Cell viability:* As shown in **Figure 2A****,** the amount of THP1 macrophages decreased by 80 to 90 % for the two highest dexamethasone concentrations used, namely ∼100 µg/ml and ∼50 µg/ml. As the dexamethasone concentration decreases (*e.g*., ∼12.5gg/ml or lower), the number of cells increases to about 100% of the two controls: media and LPS. The two higher concentrations (*e.g.,* ∼100 µg/ml and ∼50 µg/ml) of dexamethasone are thus cytotoxic and lower concentrations (*e.g.,* ∼12.5µg/ml or lower) of dexamethasone permit cell viability. The same profiles were observed at both 1 day and 3 days.

*TNF-α release:* As shown in **Figure 2A****,** for both 1 and 3 days, the positive and negative controls significantly different from each other, where more *TNF-α* was released with the LPS stimulated environment. For the cytotoxic concentrations (∼100 µg/ml and ∼50 µg/ml) of dexamethasone, both time points showed a larger amount of TNF-α released compared to the positive control. Without wishing to be bound by theory, dead cells can generally cause a burst of cytokine release. From dexamethasone concentrations of about 25 to 3.125 µg/ml, for both time points, the TNF-α release was stable at about 0.05 pg/ml, but not significantly less expressed than the LPS control.

*Gene expression:* As shown in **Figure 2B****,** 1 day after exposure to dexamethasone at a concentration of ∼25 to ∼3.125 µg/ml, the expression of TNF-α and TGF-β was down-regulated compared to LPS and media control; IL-10 was up-regulated compared to media control with a fold change in expression (relative to positive control) above 1 at a dexamethasone concentration of about 12.5 µg/ml. The two lower concentrations, 6.25 and 3.125 µg/ml of dexamethasone resulted in an increased anti-inflammatory profile than observed in the media control.

As shown in **Figure 2C****,** 3 days after exposure to different concentrations of dexamethasone, there is no significant decrease of the proinflammatory response of TNF-α compared to LPS control. For the cytotoxic concentrations of dexamethasone (*e.g*., 100 µg/mL), IL-10 gene expression was so highly up-regulated that the results (∼1011 fold-change relative to positive control) cannot be shown on the graph in **Figure 2C****.** TGF-β was also highly up-regulated for all dexamethasone concentrations except for ∼25 µg/ml. However, the dexamethasone concentrations of 12.5 µg/mL or lower showed the similar profile as media control, which can indicate that there is no anti-inflammatory effect at these concentrations. The similar release profile was observed at 1 and 3 days, but the gene expression profiles are totally different. It can be speculated that there is an anti-inflammatory effect of dexamethasone on the gene expression for the concentrations that showed good viability, even if this is not reflected in the release. The drug reduces the inflammatory response induced by the silk fibroin particles, as the TNFα gene expression is lower than in the negative control, particularly at 1 day.

The cell viability of and cytokine release from THP1 macrophages seeded on SP1 particles stimulated with simvastatin are shown in **Figures 3A-3C****.** The cell viability and TNF-α release at 1 day and 3 days are combined in **Figure 3A****.** **Figures 3B** and **3C** display TNF-α, TGF-β, and IL-10 gene expression at 1 and 3 days.

*Cell viability:* As shown in **Figure 3A****,** the amount of THP1 macrophages decreased by 80 to 90 % for the highest simvastatin concentrations used, namely, ∼250 µg/ml (both time points) and ∼125 µg/ml (day 3 time point only). With decreasing simvastatin concentration, the number of cells increased to around 90 %, when the cells were exposed to simvastatin at a concentration of about 62.5µg/ml for about 1 day, or when the cells were exposed to simvastatin at a concentration of about 31.25 µg/ml for about 3 days, compared to the two controls: media and LPS. The 250 µg/ml simvastatin concentration is cytotoxic, but lower concentrations enable good viability, depending on the length of the culture. For example, the 125 µg/ml and 62.5 µg/ml concentrations can be used if the THP1 macrophages are cultured for about one day.

*TNF-α release:* A shown in **Figure 3A****,** the two controls (negative: media and positive: media+LPS) are significantly different only at 1 day. For the higher simvastatin concentration, *TNF-α* release was higher than the positive control. At ∼125 µg/ml simvastatin, the *TNF-α* release was smaller than the amount released from the negative control on day 1, and the TNF-αrelease as still very high on day 3, *e.g.,* due to the number of high dead cells. For ∼31.5µg/ml simvastatin and less, the TNF-α release was stable at about 0.07pg/ml, but was not significantly different from the positive control.

*Gene expression:* As shown in **Figure 3B,** at 1 day, for all simvastatin concentrations, the expression of TNF-α and TGF-β were down-regulated compared to the positive control, and IL-10 was up-regulated compared to the positive control. The gene expression profiles corresponding to the 62.5µg/ml simvastatin treatment was nearly the same as the negative control, except that there was increased expression of IL-10, the anti-inflammatory cytokine.

As shown in **Figure 3C,** at 3 days, similar profiles of gene expression as measured on day 1 were determined for the anti-inflammatory cytokines, except the expression of IL-10 was higher. IL-10 gene expression was up-regulated with about 107-fold change (relative to positive control) at 250µg/ml simvastatin and decreased gradually to about 1 fold-change (relative to positive control) at the lowest concentration.

Taken together, the results of **Figures 3A-3C** show the anti-inflammatory effect of simvastatin on gene expression. The increased expression of IL-10 compared to the negative control indicates that simvastatin can activate IL-10 expression. However, the TNF release was not reduced with simvastatin.

The cell viability of and cytokine release from THP1 macrophages seeded on SP1 particles stimulated with nicotine are shown in **Figures 4A-4C****.** The cell viability and TNF-α release at 1 day and 3 days are combined in **Figure 4A. Figures 4B** and **4C** display TNF-α, TGF-β, and IL-10 gene expression at 1 and 3 days.

*Cell viability:* As shown in **Figure 4A****,** cell viability was observed to be similar for both time points. The 3 mg/ml nicotine treatment was cytotoxic for both time points, and ∼1.5 mg/ml became cytotoxic at 3 days. When the THP1 macrophages were cultured at about 1.5 mg/ml to 93.75 µg/ml nicotine for about 1 day, or at about 750 µg/ml to 98.75 µg/ml for about 3 days, the percentage of viable THP1 macrophages reached to ∼90% - ∼120%. Nicotine appeared to have a proliferative effect because there were more cells in cultures treated with the drug than in both the positive and negative controls. Nicotine may also prevent the differentiation of monocytes to macrophages, allowing a longer proliferation period. Nicotine concentrations below 750µg/ml can be used to evaluate the antiinflammatory effect of nicotine.

*TNF-α release:* As shown in **Figure 4A****,** the two controls were not significantly different after 1 and 3 days. For the higher concentrations (*e.g*., above 750 µg/ml) of nicotine, more TNF-α was released than the positive control (LPS without any drug associated) at both time points. Below 750 µg/ml nicotine concentrations for both time points, TNF-α release was reduced relative to the positive control level as well as negative control (level around 0.06 pg/ml).

*Gene expression:* As shown in **Figure 4B,** at 1 day, the nicotine concentrations (∼98.75 µg/ml to ∼1.5 mg/ml) with good cellular viability show downregulation of TNF-α at ∼375 µg/ml and ∼187.5 µg/ml, which approached to the level expressed in the negative control. In both the cytotoxic and viable environments, gene expression of TGF-β and IL-10 were unregulated compared to controls. IL-10 gene expression was increased relative to control to a larger extent than TGF-β.

As shown in **Figure 4C,** at 3 days a similar pattern of TNF-α expression was determined, with TNF-α expression higher than the negative control. TGF-β and IL-10 gene were downregulated compared to the same concentrations at 1 day.

Based on the results of **Figures 4A-4C****,** the anti-inflammatory effect of nicotine appears to be less overt than dexamethasone and simvastatin. In some embodiments, nicotine can induce a proliferative effect rather than an anti-inflammatory effect.

**Figures 5A-5C** depict cell viability and TNF-α release results from 5 experiments (20 samples) where THP1 macrophages were cultured under the same conditions using silk fibroin particles as described above except the cells were seeded on silk fibroin films.

*Cell viability:* As shown in **Figures 5A-5C****,** THP1 macrophages showed similar profiles of cell viability as compared to the data obtained from the SP1 particles. The concentrations of anti-inflammatory drugs that were cytotoxic in silk particles experiments remained cytotoxic in silk film experiments.

*TNF-α release:* Comparing 1 day and 3 days, the two controls are significantly different for both time points and in every antiinflammatory drug data set. **Figure 5A** shows significant reductions in TNF- α release (relative to LPS control) in the presence of dexamethasone at ∼25 µg/ml and ∼12.5 µg/ml. Same or similar profiles were observed for the THP1 macrophages when they were in contact with either SP1 particles or the silk fibroin films for simvastatin and nicotine (**Figures 5B-5C** vs. **Figures 3A** and **4A**).

At least one difference between cultures of THP1 macrophages in SP1 and silk fibroin films is that the concentration of TNF-α released from the cells is smaller than the ones cultured on SP1. The concentration of TNF-α release in the negative control (media) was about 0.02 pg/ml for cells seeded on silk fibroin films compared to 0.04 pg/ml for cells seeded on SP1. While the concentrations are extremely low, it is interesting that the SP1 particles induce more pro-inflammatory response than a silk film. Thus, the shape of the material itself can have an effect on the inflammatory response, where micro-particles appear to promote a pro-inflammatory pathway. The controls display better results on silk fibroin films than on silk fibroin particles, which can indicate that the results obtained from cells seeded on films are more reproducible.

The range of concentrations of anti-inflammatory drugs can be adjusted to provide a viable cellular environment and significant reduction in proinflammatory cytokines. While dexamethasone, simvastatin and nicotine do not appear to a significant decrease in TNF-α release, dexamethasone showed anti-inflammatory profile for gene expressions. In addition, the positive and negative controls were not significantly different in gene expression on SP1 particles, but they were on the silk fibroin films.

*Macrophage morphology visualized by SEM microscope:* This section shows data on cell viability, TNF-α release, and SEM images of THP1 macrophages seeded on SP1 particles. These cells were cultured with media, a macrophage-skewing agent (*e.g.,* LPS at ∼200ng/ml), or dexamethasone diluted in media containing a macrophage-skewing agent (*e.g.,* LPS at ∼200ng/ml). SEM images for the other drugs are not presented, as they are similar to the results shown for dexamethasone. The SEM images show particle size distribution and allow study of the decrease in cell viability to determine if the decreased cell viability is due to cell death and/or nonadherent cells. Further, these images show that the morphology of the macrophages matches well with the TNF-α release and gene expression results in term of activation characteristics.

TNF-α release and the cell viability of THP1 macrophages stimulated by SP1 particles and modulated with different concentrations of dexamethasone are shown in **Figure 6****.** Similar profiles were observed for TNF-α release and the cell viability as shown in **Figure 2A** earlier. Further, a significant decrease in TNF-α release at 3.125 µg/ml of dexamethasone was determined. **Figure 7** shows a set of the SEM images representing morphology of THP1 macrophages cultured on SP1 particles at different dexamethasone concentrations at 1 day (A1-G1) and at 3 days (A3-G3), respectively. The difference expected in the macrophage morphology between the negative and positive control was observed at 1 day (**Figure 7**: A1,B1) and at 3 days (**Figure 7****:** A3, B3). Indeed, as seen with the TNF-α release, the macrophages from the positive control are more activated than in the negative control. In the media control (**Figure 7**: A1, A3), the THP1 macrophages display a normal, rounded shape with low levels ECM production, *e.g.,* a level sufficient to allow adhesion to the particles. In contrast, in the positive control with LPS (**Figure 7**: B1, B3), there is enhanced production of ECM and even some cell fusion at 3 days (**Figure 7**: B3), both of which are the characteristics of activated macrophages in response to an infectious environment.

For cytotoxic concentration (**Figure 7**: C1, D1 and C3, D3) of dexamethasone, dead cells with no shape and/or with a perfectly rounded shape (indicating that the cells are nonadherent and will die) were observed. At dexamethasone concentrations of about 25 µg/ml (**Figure 7**: E1) and about 12.5µg/ml (**Figure 7**: F1), viable activated cells were observed, but to a less extent than the positive control. At these concentrations, THP1 macrophages have pseudopods traducing their activation but they secrete less ECM than in the positive control. Interestingly, at a dexamethasone concentration of about 3.125 µg/ml (**Figure 7**: G1), more rounded cells like in media control were observed, but these cells also have tiny pseudopods that traduce their activation. TNF-α release was significant reduced and approached the level detected in the negative control. At 3 days, at 25µg/ml (**Figure 7**: E3), non-viable cells with irregular shapes and ECM production were observed. As dexamethasone concentration decreased from 12.5 µg/ml to 3.125 µg/ml, it was detected an increase in the activation of the macrophages associated with more ECM production and large pseudopods. The cells produced more TNF-α at ∼3.125 µg/ml dexamethasone than at ∼12.5µg/ml, which indicates that the morphology of cell can be an accurate indicator of their activation and is in good agreement with the ELISA results. Further, the SEM images indicate that the SP1 particles were well dispersed on the tissue culture plate and they displayed homogeneous size of about 2 µm, as shown at **Figure 7****:C1,** E1 and F3 (scale of ∼2 µm).

### Example 3: Anti-inflammatory drug modulation of cytokine expression and release from differentiated primary macrophages and dendritic cells seeded on ∼1 µm-sized silk fibroin particles (SP0.5)

Described in this example are the results obtained from cultures of primary macrophages and dendritic cells after 1 day and 5 days. The cells were seeded on SP0.5 particles (∼1 µm in size) and treated with dexamethasone, simvastatin or nicotine at various concentrations as detailed in the scheme shown in **Figure 8****.** **Figures 9A****,** **11A****,** and **13A** depict the concentration of TNF-α released per cell in pg/ml, as well as the cell viability expressed as a percentage of the negative control (media) in primary macrophage cultures, while **Figures 15A****,** **17A****,** and **19A** depict the concentration of TNF-α released per cell in pg/ml, as well as the cell viability expressed as a percentage of the negative control (media) in dendritic cell (DC) cultures. **Figures 9B****,** **9C****,** **11B****,** **11C****,** **13B****,** and **13C** depict TNF-α, IL1-RA and IL-10 gene expression for primary macrophages, while **Figures 15B****,** **15C****,** **17B****,** **17C****,** **19B****,** and **19C** depict IL1-β, IL1-RA and IL-8 for DCs, wherein the fold change was expressed relative to the positive control (LPS), which was normalized to 1. This Example used SP0.5 particles, ∼400 ng/mL LPS, and the cultures were examined at a later time point, 5 days, to provide enough time for the cells to differentiate and respond.

### Primary macrophages data set

**Figures 9A-9C** depict data from primary macrophages seeded on SP0.5 particles and stimulated with dexamethasone at various concentrations. **Figure 9A** shows cell viability and TNF-α release at days 1 and 5. **Figures 9B** and **9C** show TNF-α, IL-1RA and IL-10 gene expression at 1 and 5 days, respectively.

*Cell viability:* As shown in Figure 9A, the cell viability differs between days 1 and 5. At 1 day, viability of primary macrophages was up to 80% at 1 day when the cells were cultured in the presence of dexamethasone at a concentration of about ∼25 µg/ml to ∼1.6 µg/ml, whereas the cell viability remained constant at around 55% at 5 days. Comparing the 1-day cultures with THP1 macrophages and primary macrophages, 80 % cell viability was observed with dexamethasone concentrations at and below 25 µg/ml for primary macrophages and at and below 12.5 µg/ml for THP1 macrophages.

*TNF-α release:* As shown in **Figure 9A****,** at both 1 and 5 days, both controls are significantly different in TNF-α release. There was also a significant decrease in TNF-α release when the dexamethasone concentration decreased from ∼25 µg/ml to ∼1.5625 µg/ml for both time points. Further, it is noted that, compared to SP1 negative control results, the concentration of TNF-α released from the negative control on SP0.5 was reduced, *e.g.,* by half. Doubling the concentration of LPS results in a similar TNF-α release (about 0.05 pg/mL) regardless of particle size.

*Gene expression:* As shown in **Figure 9B,** at 1 day, an anti-inflammatory gene profile was observed, with low TNF-α gene expression and high gene expression of IL-10. The low TNF-α gene expression supports TNF-α release, as measured by ELISA. These results indicate that dexamethasone can activate expression of genes coding for anti-inflammatory proteins, such as IL-10. Interestingly, a high level of TNF-α gene expression in the negative control was observed with a fold-change greater than 1 (relative to the positive control) and was thus greater than the positive control. This finding corresponds more to a proinflammatory profile and was not supported by the ELISA results. As shown in **Figure 9C,** at 5 days, anti-inflammatory profile was observed with lower TNF-α gene expression and much higher IL-10 gene expression. About the same high level of TNF-α gene expression in negative control was observed at days 1 and 5, but at 5 days there was also a high gene expression of IL-1RA. These results indicate that the anti-inflammatory pathway can act to balance the proinflammatory pathway. Dexamethasone can have an antiinflammatory effect on primary macrophages, as determined by gene expression and TNF-α release. The range of effective dexamethasone concentration can range from about 25 µg/ml to about 1.6 µg/ml.

*Cell morphology :* **Figure 10** are the SEM images depicting the morphology of primary macrophages cultured with dexamethasone on SP0.5 particles at 1 day (A1-H1) and at 5 days (A5-H5), respectively. Similar to the results from THP1 macrophages in Example 2, the difference in the macrophage morphology between the negative and positive control was observed at 1 day (**Figure 10**: A1, B1) and 5 days (**Figure 10**: A5, B5). Primary macrophages in the positive control are more activated and display more fusion than in the negative control. For example, **Figure 10** (B5) shows a cell measuring around 70µm of length. At ∼50µg/ml dexamethasone (**Figure 10**: C1, C5), necrotic cells were observed. The SEM images shown at **Figure 10****:** E1, F1 and G1 were taken with a higher magnification and show that the cells are smaller, more rounded, and express low levels of ECM, indicating that they were less activated than in the media control. However, the high magnification images show some pseudopods, particularly at ∼12.5 µg/ml dexamethasone concentration (**Figure 10**: E1). At the lowest dexamethasone concentration (**Figure 10**: H1), the macrophages were more elongated and deposited more ECM. At 5 days, the same profile of activation was observed at various dexamethasone concentrations, except that there was more ECM at day 5 than at 1 day indicating the higher TNF-α release at 5 days compared to the controls at the same time point. The primary macrophages at 5 days with dexamethasone seem less activated than in the media control, and the morphology of the primary macrophages corresponds well with the ELISA and RT-PCR results. The SP0.5 particles were found to be well-distributed on the tissue culture plate and display homogeneous size of about 1 µm, as shown in **Figure 10**:C1 (scale of ∼1 µm).

The data from simvastatin stimulation of primary macrophages seeded on SP0.5 particles is given in **Figures 11A-11C****.** The cell viability and TNF-α release at 1 day and 3 days are combined in **Figure 11A. Figures 11B** and **11C** display TNF-α, IL-1RA, and IL-10 gene expression of primary macrophages after simvastatin stimulation at 1 and 5 days.

*Cell viability:* As shown in **Figure 11A,** at 5 days, the viability of primary macrophages was low, *e.g.,* less than 30 %, at various simvastatin concentrations, but at 1 day, the cell viability was over 100% (*e.g.,* at least about 120%) for simvastatin concentrations at and below 125 µg/ml. These profiles are different than the one observed on SP1 particles with THP1 macrophages. It is speculated that 5 days can be too long of a delay for primary macrophages stimulated with simvastatin.

TNF-α *release:* As shown in **Figure 11A****,** it should be noted that the positive and negative controls are significantly different at both time points. In addition, there were significant reductions in TNF-α release as the simvastatin concentrations decreased from about 250 µg/ml to about 31.25 µg/ml at 1 day. Because of the poor cell viability at day 5, while TNF-α release was relatively high for all concentrations, TNF-α release decreased at lower concentrations of simvastatin with a small increase in cell viability. The TNF-α release in the negative control is low (∼0.01 pg/ml) and is smaller than the level obtained with SP1 particles.

*Gene expression:* As shown in **Figure 11B,** at 1 day, an anti-inflammatory profile, low TNF-α gene expression that correlates with the ELISA results, was observed when simvastatin concentrations ranging from 125 µg/ml to 31.25 µg/ml were used. Unexpectedly, an increase in IL-10 gene expression was not observed. In contrast to the results obtained with dexamethasone, there was an increase in anti-inflammatory cytokine IL-1RA gene expression. TNF-α expression was high in the media control.

The gene expression data of primary macrophages after simvastatin stimulation as shown in **Figure 11C** is different from the cells after dexamethasone stimulation, partly because the cell viability of primary macrophages was low after simvastatin stimulation for 5 days. As observed with dexamethasone, the expression of IL-10 increased at 5 days while TNF-α maintains a relatively low level of expression. These findings show that 5 days of culture with simvastatin can be damaging to primary macrophages. However, simvastatin can have immediate or short-term antiinflammatory effects on cytokine production as well as gene expression, despite the fact that the level of IL-10 gene expression does not correlate with the literature (49). The range of effective concentrations for simvastatin can range from about 125 to about 31.25 µg/ml.

*Cell morphology:* **Figure 12** are the SEM images depicting the morphology of primary macrophages cultured with simvastatin on SP0.5 particles at 1 day (I1-P1) and at 5 days (I5-P5), respectively. A difference in the activation characteristics of the negative and positive controls was observed (**Figure 12****:** I1, J1 and I5, J5). The SEM images at 5 days for various simvastatin concentrations show that there are not many adherent cells, and the ones that adhered did not appear to be viable. However, at 1 day, at low simvastatin concentrations (*e.g*., from about 31.25 µg/ml to about 7.8125 µg/ml) (**Figure 12****:** N1, O1, P1), the cells were relatively small, had a rounded shape, and produced low levels of ECM. The cells that are spread on the particle surface did not have pseudopods.

**Figures 13A-13C** show experimental data obtained from primary macrophages seeded on SP0.5 particles and stimulated with nicotine. The cell viability and TNF-α release at 1 day and 5 days are combined in **Figure 13A. Figures 13B** and **13C** display TNF-α, IL-1RA and IL-10 gene expression of primary macrophages stimulated with nicotine at 1 and 5 days.

*Cell viability:* As shown in **Figure 13A****,** treatment with nicotine leads to a reduction in cell viability of primary macrophages between 1 day and 5 days in culture. In contrast to dexamethasone and simvastatin treatment, the primary macrophages remained viable at 5 days and cell viability reached about 90% when the nicotine concentration decreased from about 187.5 µg/ml to 46.87 µg/ml. Similar to cultures of THP1 cells on SP1 particles, the cell viability was over 100% at a nicotine concentration ranging from about 187.5 µg/ml to about 46.87 µg/ml at 1 day.

*TNF-α release:* As shown in **Figure 13A****,** the positive and negative controls were significantly different for both time points. The TNF-α release was lower in both controls compared to the data sets from dexamethasone or simvastatin stimulation. There is no significant decrease in TNF-α release compared to the LPS control at either time point.

*Gene expression:* As shown in **Figure 13B,** at 1 day and nicotine concentrations of 187.5 and 93.75 µg/ml, TNF-α gene expression of primary macrophages was downregulated but might not be low enough to assume an antiinflammatory profile. Nicotine concentration from 1.5 mg/ml to 375 µg/ml appears to be more cytotoxic than indicated by high level of TNF-α detected by ELISA. However, better viability was observed between about 750 µg/ml and about 275 µg/ml, and between these concentrations, the cells balanced the inflammatory response with high gene expressions of IL-1RA and IL-10. At 1 day in the negative control, there is a high level of TNF-α gene expression with low IL1-RA and IL-10 gene expression.

As shown in **Figure 13C,** at 5 days, similar gene expression profiles as 1 day were observed, except that the levels of cytokine expressions were higher. Moreover, the same profile for the negative control treated with the other drugs was observed. In some embodiments, nicotine seems to provide proliferative effects more than antiinflammatory effects on both immortalized and primary macrophages. Due to the variability of TNF release and gene expressions in the controls, the SP0.5 particles did not improve the reproducibility of these experiments compared to the SP1 particles. However, the positive and negative controls in this data set were significantly different, which indicates that doubling the concentration of LPS may have resolved the issue of their relevance.

*Cell morphology:* **Figure 14** are the SEM images depicting the morphology of primary macrophages cultured with nicotine on SP0.5 particles at 1 day (Q1-X1) and at 5 days (Q5-X5), respectively. The negative and positive controls (**Figure 14**:Q1, R1 and Q5, R5) show the same characteristics of activation as in the previous dexamethasone or simvastatin data set. For both time points, the number of cells increased in accordance with the cell viability results in **Figure 13A****.** In addition, the primary macrophages appeared more spread and produced more ECM. There is also more cell fusion as shown in **Figure 14**:V5 and W5. In contrast to the dexamethasone data set, no detectable pseudopods were observed, but large spread cells that show some characteristics of activation.

### Primary dendritic cell data set

Presented herein includes the data of primary dendritic cells seeded on SP0.5 particles stimulated with dexamethasone. The cell viability and TNF-α release at 1 day and 5 days are combined in **Figure 15A. Figures 15B** and **15C** display IL-1β, IL-1RA and IL-8 gene expression of dendritic cells at 1 and 5 days, respectively.

*Cell viability:* As shown in **Figure 15A****,** cell viability follows the same profile as the primary macrophages.

*TNF-α release:* As shown in **Figure 15A****,** at both 1 and 5 days, the two positive and negative controls are significantly different. Similar to the primary macrophage data, a significant decrease in TNF-α was measured for various dexamethasone concentrations at both time points.

*Gene expression:* As shown in **Figure 15B,** at 1 day, an anti-inflammatory profile is present for various dexamethasone concentrations. IL-1β gene expression (relative to the positive control) was low, and the fold-change ranged from 0.1 and 0.2. IL-1RA was expressed to a greater extent in primary dendritic cells than in primary macrophages, which may explain the low IL-1β levels. Interestingly, the level of IL-1RA in the primary dendritic cells was the same as or similar to the levels detected in the primary macrophages, and it was also at the same or similar level as IL-1β and acted to balance the proinflammatory pathway.

As shown in **Figure 15C,** at 5 days, the same or similar profile was observed, but the expression of IL-1β was 2.5 times higher in the media control. Dendritic cells give the same or similar TNF-α release profile as the macrophages with less variability in gene expression. In some embodiments, TNF-α and IL-10 gene expression in dendritic cells can be analyzed to correlate all the results.

*Cell morphology:* As shown in **Figure 16****,** the positive and negative controls show different cell morphology of dendritic cells, where the cells were more elongated with more ECM production in the positive control (**Figure 16**: B5) but smaller in the negative control (**Figure 16**: A5). There was also a difference in activation characteristics between the dendritic cells and macrophages. While fusion was found to be an activation marker for macrophages, elongated cells with many pseudopods are characteristic of activation for dendritic cells (**Figure 16**: B5, D5, H5). One of the characteristics of dendritic cells is that they do not generally fuse together. Moreover, considering this morphology type, the dendritic cells, at various dexamethasone concentrations, were less activated than in the positive control, and this correlates with the cytokine release and gene expression described above.

The data from primary dendritic cells seeded on SP0.5 particles and stimulated with simvastatin is given in **Figures 17A-17C** and **Figure 18****.** The cell viability and TNF-α release at 1 day and 5 days are combined in **Figure 17A. Figures 17B** and **17C** display IL1β, IL-1RA, and IL-8 gene expression of dendritic cells stimulated with simvastatin at 1 and 5 days.

*Cell viability:* As shown in **Figure 17A****,** the same or similar cell viability profile as those of the primary macrophages stimulated with simvastatin was observed for both time points.

*TNF-α release:* As shown in **Figure 17A****,** the same or similar profiles of TNF-α release as those of the primary macrophages stimulated with simvastatin were observed at both time points except that there is no significant decrease compared to LPS control. Moreover, the positive and negative controls are significantly different.

*Gene expression:* As shown in **Figure 17B,** at 1 day, interestingly, the antiinflammatory genes IL-1RA and IL-8 were increasingly expressed with decreasing simvastatin concentrations. For simvastatin concentrations that showed good cell viability (*e.g*., about 125 µg/ml to about 7.8 µg/ml), an anti-inflammatory profile was observed at ∼62.5µg/ml. The negative control has the same or similar profile as the negative control from dexamethasone data set.

As the cell viability of dendritic cells stimulated with simvastatin for 5 days was low, the gene expression data shown in **Figure 17C** varied. However, the same or similar profile was observed for the negative control as in the previous dexamethasone data. The IL-1RA fold change (relative to the positive control) was less than 1. In addition, 5 days of cell culture with simvastatin was found to be damaging to macrophages and dendritic cells. There was also some variability on gene expression between different control samples.

*Cell morphology:* In **Figure 18****,** the SEM images show that the cell death and activation morphology characteristics are similar to the experiment on primary macrophages.

The data from primary dendritic cells seeded on SP0.5 particles and stimulated with nicotine is given in **Figures 19A-19C** and **Figure 20****.** The cell viability and TNF-α release at 1 day and 5 days are combined in **Figure 19A. Figures 19B** and **19C** display IL1β, IL-1RA, and IL-8 gene expression of dendritic cells stimulated with nicotine at 1 and 5 days.

*Cell viability:* As shown in **Figure 19A****,** cell viability follows the same or similar profile as observed in the primary macrophages, where the nicotine concentrations ranging from about 187.5 µg/ml to about 46.87µg/ml show a cell viability greater than 85% to 100% at both time points.

*TNF-α release:* As shown in **Figure 19A****,** the profile observed with dendritic cells was the same as observed with macrophages, and the controls were significantly different at both time points.

*Gene expression:* As shown in **Figure 19B,** at 1 day there was more variability in gene expression as compared to the primary macrophage data set for the 3 higher nicotine concentrations (about 375 µg/ml to about 1.5 mg/ml), where the cell viability was low and varied. Moreover, there was no anti-inflammatory profile for the lower 3 nicotine concentrations (about 46.87 µg/ml to about 187.5 µg/ml).

As shown in **Figure 19C,** at 5 days, the same profile as observed in the primary macrophages was observed, indicating that there is no anti-inflammatory profile. Moreover, as described earlier, the results are the same for the negative control as in the case of the dexamethasone. There are some differences between dendritic cells and macrophages, but the data from the two cell types are similar. From these experiments, nicotine was not found to have any anti-inflammatory effects.

*Cell morphology:* **Figure 20** shows the SEM images of dendritic cells stimulated with nicotine for 1 day or 5 days, and their cell morphology was same as or similar to the ones in the primary macrophage data set. The number of dendritic cells with spread shape and production of ECM increased with decreasing nicotine concentration.

### Discussion

The findings as described in Examples 2-3 are summarized in Table 4. Specifically, Table 4 summarizes the results of TNF-α release and different gene expression with respect to the positive control (media+ LPS). Based on the findings described herein, dexamethasone was shown to reduce proinflammatory cytokine production as well as to induce an antiinflammatory gene expression profile. Simvastatin is also capable of inducing anti-inflammatory gene expression profiles, as experiments with primary macrophages or dendritic cells at certain simvastatin concentrations revealed anti-inflammatory gene expression profiles. Nicotine did not appear to have anti-inflammatory effects, but proliferative properties. Moreover, the results showed that SP0.5 particles (∼1 µm in size) induced a milder inflammatory response than SP1 particles (∼2 µm in size).

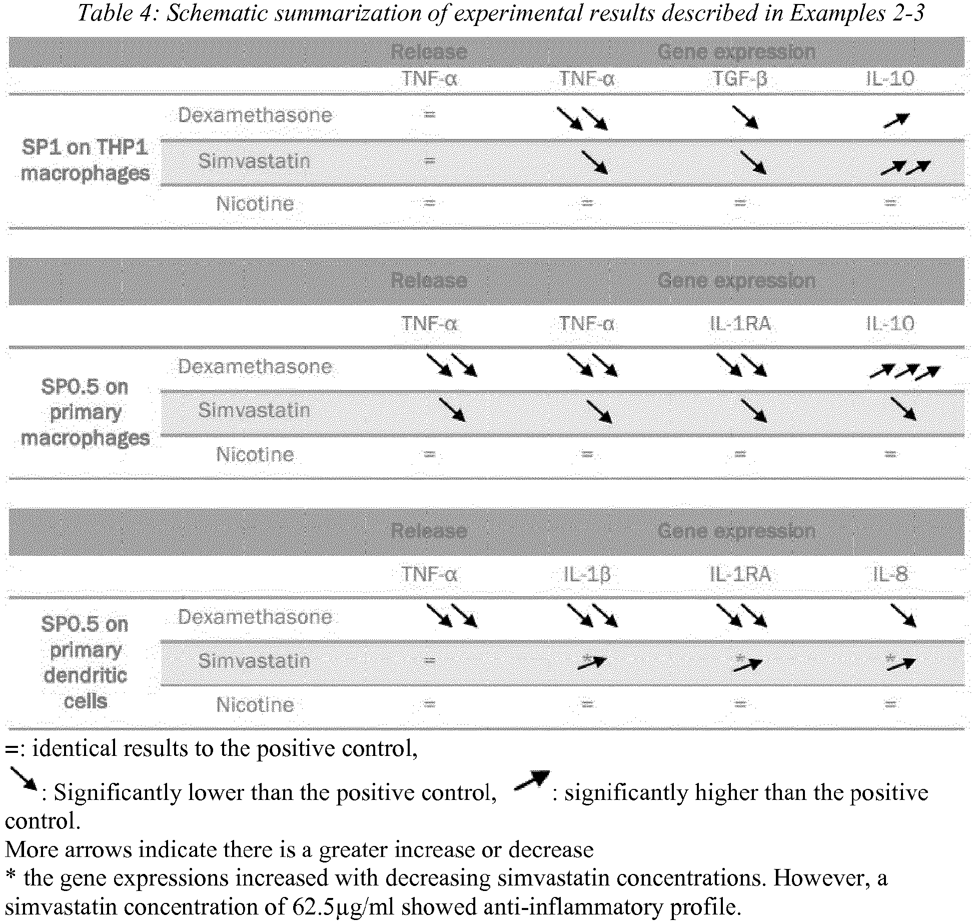

Described herein are experiments demonstrating how the inflammatory response induced by silk particles of different sizes can be optimized. Three well known drugs with different properties, mechanisms of action and side effects were evaluated. Dexamethasone, a drug known for its anti-inflammatory properties and used in this study as a reference, showed the best results in term of reproducible decreases of pro-inflammatory cytokine production as well as induction of an anti-inflammatory gene expression profile (Table 4). Simvastatin was also found to be promising because the study on primary cells revealed anti-inflammatory profiles for several concentrations. Nicotine did not show relevant anti-inflammatory effects, and instead affected proliferation to a greater extent. Moreover, considering the size effect of the particles on inflammatory response and comparing the negative controls between SP1 (∼2µm in size) and SP0.5 (∼1µm in size) data sets, SP0.5 particles were found to induce less of an inflammatory response than SP1 particles. Increasing the concentration of LPS concentration improved the relevance of the controls. However, changing the size of particles did not improve the reproducibility of the results from the controls. The challenges with reproducibility may be the particles themselves, because the films showed reproducible controls and other changes in experiment conditions (particle drying conditions, bigger batches of silk and particles, etc.) have not improved reproducibility in the particle experiments. However, approaching *in vivo* conditions with primary cells seems to improve reproducibility for the results.

In the Examples 2-3, the findings showed that the inflammatory response induced by the silk particles can be modulated. Dexamethasone, as expected, showed better effect in the reduction of the inflammatory response induced by the particles than the other two drugs tested.

### Example 4. Controlled macrophage differentiation or skewing with silk films functionalized with macrophage-skewing agent

Biomaterials can be modified, for example, chemically and/or via inclusion of appropriate compounds or molecules during processing or post processing, to regulate macrophage and/or other cellular responses *in vitro* or *in vivo.* For example, introduction of a selective compound that can promote inflammation or reduce inflammation, to a cell population in order to influence degradation of the biomaterial, *e.g*., degrading the biomaterial faster, slower, or in a more selective way, *e.g.,* to control inflammatory responses or regeneration responses for biomaterials and tissues. The control process can be mediated by the nature of the cell populations (*e.g*., macrophages) activated or promoted in the presence of the selective compounds, *e.g*., but not limited to, small molecules, peptides, and LPS.

In some embodiments, a silk matrix can comprise a macrophage-skewing agent that can selectively up-regulate at least one marker of M2 macrophage. In some embodiments, a silk matrix can comprise a macrophage-skewing agent that can selectively up-regulate at least one marker of M1 macrophage. As shown in **Figure 21****,** various macrophage-skewing candidate agents (*e.g.,* at a concentration ranging from ng/ml to µg/ml) were added to silk films, which were then cultured with THP-1 monocytes. **Figure 21** shows that addition of about 10mg/ml interleukin-4 (IL-4) to silk films was capable of up-regulating at least one markers of M2 macrophage (*e.g*., but not limited to, CCL18 and CD206) in THP-1 monocytes. Thus, in one embodiment, at least about 10 µg/ml of IL-4 can induce M2 macrophage phenotype differentiation. Without wishing to be bound by theory, M2 macrophages can typically help in regenerating damaged tissues.

However, low level of LPS that was added to the silk films was not sufficient to stimulate M1 differentiation. Macrophage differentiation can depend on concentrations and/or amounts of macrophage-skewing agents present in a silk matrix. Thus, in some embodiments, the LPS level in the silk films can be increased to a level sufficient to stimulate M1 differentiation.

**Figure 22** is a bar graph showing expression of markers of M1 macrophages and M2 macrophages when the THP-1 monocytes were in contact with a silk film comprising interferon-gamma (INF-γ). About 10 µg/ml interferon-gamma (INF-γ) was loaded or embedded in silk films. In some embodiments, it is desirable to avoid the wash steps of the silk films after the loading. The THP-1 monocytes were cultured in contact with the silk films. As shown in **Figure 22****,** at least one marker (*e.g.,* at least two or more) of M1 macrophage (*e.g.,* but not limited to, CCL3 and CCR7) were up-regulated in THP-1 monocytes. Thus, in one embodiment, at least about 10 µg/ml INF-γ can induce M1 macrophage differentiation. CCL-18, an M2 macrophage marker, was also upregulated. CD206, a common marker of M2 macrophage was strongly downregulated relative to control. Without wishing to be bound by theory, the M1 macrophage can elicit an inflammatory response to protect newly damaged tissue from infection. While M1 macrophages may not be desirable for tissue remodeling, they can be used for rapid degradation of biomaterials, *e.g*., but not limited to, silk.

**Figure 23** is a line graph showing the release rate of INF-γ from the silk films. The silk films were made from a silk solution at a concentration of about 10 µg/ml and about 0.2 ml of the silk solution was used per film (with a loading of about 2 µg INF-γ). As shown in the table of **Figure 23****,** within one hour an amount of about 400 pg/ml INF-γ was released, which is about 0.02 % of the total loading. More INF-γ was released by day 4, indicating that there is a sustained or constant release of INF-γ. While the levels of INF-γ release appeared to be low, the level is physiologically relevant.

In some embodiments, a silk matrix, *e.g*., a silk film, can comprise at least one M1 macrophage differentiation agent (*e.g*., but not limited to, INF-γ) and at least one M2 macrophage differentiation agent (*e.g.,* but not limited to, IL-4), for example, in a pre-determined ratio, *e.g.,* to control the degradation of the silk matrix (*e.g*., upon implantation to a target site in need thereof) and facilitate tissue regeneration. The M1 and M2 macrophage differentiation agents can be distributed uniformly or heterogeneously in a silk matrix. In some embodiments, the M1 and M2 macrophage differentiation agents can be distributed in different layers of a silk matrix, *e.g*., forming a multi-layered silk matrix, wherein a first layer can comprise a M1 macrophage differentiation agent and a second layer can comprise a M2 macrophage differentiation agent.

### References

*1.* Silk as a Biomaterial. L.Kaplan, Charu Vepari and David. 2007, Prog Polym Sci, Vols. 32(8-9), pp. 991-1007.
*2.* Silk-based Biomaterials. Gregory H. Altman, Frank Diaz, Caroline Jakuba, Tara Calabro, Rebecca L. Horan, Jingsong Chen, Helen Lu, John Richmond and David L. Kaplan. February 2003, Biomaterials, Vols. Volume 24, Issue 3, pp. 401-416 .
*3.* Evolution of the Insects. Engel, Grimaldi &. s.l. : Cambridge University Press, 2005.
*4.* Taken for a Spin. (2007)., Cunningham A. s.l. : Science News, Vol. 171, pp. 231-33.
*5.* Spider silks and their applications. Jonathan A. Kluge, Olena Rabotyagova, Gary G. Leisk and David L. Kaplan. s.l. : Trends in Biotechnology, Vol. 26:5.
*6.* Applications of natural silk protein sericin in biomaterials. YQ., Zhang. s.l. : Biotechnol Adv, 2002 May, Vol. 20(2), pp. 91-100.
*7.* Spider Silk Fibers Spun from Soluble Recombinant Silk Produced in Mammalian Cells. Anthoula Lazaris, Steven Arcidiacono, Yue Huang, Jiang-Feng Zhou, François Duguay, Nathalie Chretien, Elizabeth A. Welsh, Jason W. Soares and Costas N. Karatzas. s.l. : Science, 2002, Vols 295, n°5554, pp. 472-476.
*8.* Transgenic silkworms (Bombyx mori) produce recombinant spider dragline silk in cocoons. Wen, H. X. et al. s.l. : Molecular Biology Reports, 2010, Vol. 37, pp. 1815-1821.
*9.* Native-sized recombinant spider silk protein produced in metabolically engineered Escherichia coli results in a strong fiber. Xia, X. X. et al. s.l. : PNAS, 2010, Vol. 107, pp. 14059-14063.
10. Biomedical applications of chemically-modified silk fibroin. Kaplan, Amanda R. Murphya and David L. s.l. : Mater Chem., 2009 June 23, Vol. 19(36), pp. 6443-6450.
11. Silk: a potential medium for tissue engineering. Sobajo C, Behzad F, Yuan XF, Bayat A. s.l. : Eplasty, 2008, Vol. 8.
*12.* The inflammatory responses to silkfilms in vitro and in vivo. Lorenz Meinel, Sandra Hofmann, Vassilis Karageorgiou, Carl Kirker-Head, John McCool, Gloria Gronowicz, Ludwig Zichner, Robert Langer, Gordana Vunjak-Novakovic, David L. Kaplan. s.l. : Biomaterials, January 2005, Vols. 26, Issue 2, pp. Pages 147-155.
13. Sericin-producing race of Bombyx mori for skincare material. Mase K., Okada E., Lizuka T., Yamamoto T. s.l. : Natural Ingredients, 2010, Vol. 1.
14. Silk sericin and its applications: a review. Pawar, M N Padamwar and A P. s.l. : Journal of Scientific and Industrial Research, 2004, Vol. 63, pp. 323-329.
15. Applications of natural silk protein sericin in biomaterials. Zhang, Yu-Qing. s.l. : Biotechnology Advances, May 2002, Vols. 20, Issue 2, pp. 91-100.
16. Silk sericin and its applications: A review. N., Padamwar M. and P., Pawas A. s.l. : Journal of scientific & industrial research, 2004, Vols. 63, no4, pp. 323-329.
17. V. Kearns, A.C. MacIntosh, A. Crawford and P.V. Hatton. Silk-based Biomaterials for Tissue Engineering. Eds. N Ashammakhi, R Reis, & F Chiellini ©. s.l. : Tissue Engineering, 2008. Vol. 4.
18. The Williams dictionary of biomaterials. Williams, David Franklyn. 1987.
19. Miller-Keane Encyclopedia & Dictionary of Medicine, Nursing & Allied Health. Vol. 7th Edition.
*20.* Macrophage responses to silk. Bruce Panilaitis, Gregory H. Altman, Jingsong Chen, Hyoung-Joon Jin, Vassilis Karageorgiou, David L. Kaplan. s.l. : Biomaterials, 2003, Vol. 24, pp. 3079-3085.
*21.* Structural and mechanical characteristics of silk fibroin and chitosan blend scaffolds for tissue regeneration. Gobin AS, Froude VE, Mathur AB. s.l. : J Biomed Mater Res, 2005, Vols. 74, n°3, pp. 465-73.
*22.* Biodegradation of Silk Biomaterials. Wang, Yang Cao and Bochu. s.l. : Int. J. Mol. Sci., 2009, Vol. 10, pp. 1514-1524.
*23.* Silkfibroin nanoparticles for cellular uptake and control release. Joydip Kundu, Yong-Il Chung, Young Ha Kim, Giyoong Tae, S.C. Kundu. s.l. : International Journal of Pharmaceutics, 2010, Vol.388, pp. 242-250.
*24.* Silk fibroin as an organic polymer for controlled drug delivery. S. Hofmann, C.T. Wong Po Foo, F. Rossetti, M. Textor, G. Vunjak-Novakovic,D.L. Kaplan, H.P. Merkle, L. Meinel. s.l. : Journal of Controlled Release, 2006, Vol. 111, pp. 219-227.
*25.* Poly (lactic acid) Nanoparticles for Sustained Release of Budesonide. Kompella B, Bandi S, Ayalasomayajula N. 1, s.l. : Drug Development and Delivery, 2001, Vol. 1.
*26.* Nanoparticle Based Biocompatible and Targeted Drug Delivery: Characterization and In vitro Studies. Yu X, Pishko MV. s.l. : Biomacromolecules, 2011.
*27.* Controlling silk fibroin particle features for drug delivery. Lammel AS, Hu X, Park SH, Kaplan DL, Scheibel TR. s.l. : Biomaterials, 2010 Jun, Vol. 31(16), pp. 4583-91.
*28.* Inflammation in atherosclerosis. Libby, Peter. s.l. : Nature, 2002, Vol. 420, pp. 19-26.
29. Th1 and Th2 responses: what are they? Berger, Abi. s.l. : BMJ, 2000, Vol. 321, p. 424.
30. Exploring the full spectrum of macrophage activation. David M. Mosser, Justin P. Edwards, s.l. : Nature reviews: immunology, december 2008, Vol. volume 8 .
*31.* Normoxic Stabilization of Hypoxia-Inducible Factor-1αby modulation of the Labile Iron Pool in Differentiating U937 Macrophages: Effect of Natural Resistance-Associated Macrophage Protein 1. Helen J. Knowles, David R. Mole, Peter J. Ratcliffe, et al. s.l. : Cancer Res, 2006, Vol. 66, pp.2600-2607.
*32. Bacterial extract cantastim activates macrophages via TLR-2*. Cara 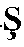 I, Tucureanu C, Pitica R, S 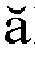 l 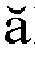 geanu A. s.l. : Roum Arch Microbiol Immunol., 2011 Jan-Mar, Vol. 70(1), pp. 28-36.
33. Effects of Titanium Dioxide Nanoparticle Aggregate Size on Gene Expression. Junko Okuda-Shimazaki, Saiko Takaku, Koki Kanehira, Shuji Sonezaki, Akiyohshi Taniguchi. s.l. : Int. J. Mol.Sci., 2010, Vol. 11, pp. 2383-2392.
34. Tumor-Associated Macrophage-Induced Invasion and Angiogenesis of Human Basal Cell Carcinoma Cells by Cyclooxygenase-2 Induction. Jeng-Wei Tjiu1, Jau-Shiuh Chen, Chia-Tung Shun, Sung-Jan Lin, Yi-Hua Liao, Chia-Yu Chu, Tsen-Fang Tsai, Hsien-Ching Chiu, Yang-Shia Dai, Hiroyasu Inoue, Pan-Chyr Yang, Min-Liang Kuo and Shiou-Hwa Jee. s.l. : Journal of Investigative Dermatology, 2009, Vol. 129, pp. 1016-1025.
35. Blood monocytes: development, heterogeneity, and relationship with dendritic cells. Auffray C, Sieweke MH, Geissmann F. s.l. : Annu Rev Immunol., 2009, Vol. 27, pp. 669-692.
36. Macrophage and dendritic cell phenotypic diversity in the context of biomaterials. Kou PM, Babensee JE. s.l. : J Biomed Mater Res A., 2010 Nov 10.
*37.* Immune inhibitory receptors: Essential regulators of phagocyte function. Tessa A. M., Steevels and Linde Meyaard. s.l. : Eur.J. immunol, 2011, Vol. 41, pp. 575-587.
38. Immunibiology of dendritic cells. al, Banchereau et. s.l. : Annu. Rev. Immunol., 2000, Vol. 18, pp. 767-811.
39. The uptake of PLGA micro or nanoparticles by macrophages provokes distinct in vitro inflammatory response. Roberto Nicolete, Daiane F. dos Santos, Lúcia H. Faccioli. s.l. : Int Immunopharmacol, 2011.
40. Size-Dependency of DL-Lactide/Glycolide Copolymer Particulates for Intra-Articular Delivery System on Phagocytosis in Rat Synovium. Eijiro Horisawa, Katsuaki Kubota, Izumi Tuboi, Keiichi Sato, Hiromitsu Yamamoto, Hirofumi Takeuchi, and Yoshiaki Kawashima. s.l. : Pharmaceutical Research, February 2002, Vols. Vol. 19, No. 2.
41. Clathrin-Coated Pit-Associated Proteins Are Required for Alveolar Macrophage Phagocytosis. Douglas G. Perry, Gena L. Daugherty and William J. Martin, s.l. : J Immunol, 1999, Vol. 162, pp. 380-386.
*42.* The mechanism of phagocytic uptake promoted by invasin-integrin interaction. Nhieu, Ralph R. Isberga and Guy Tran Van. s.l. : Trends in Cell Biology, 1995, Vol. 5:3, pp. 20-124.
43. Drug-Eluting Biodegradable Poly-D/L-Lactic Acid Vascular Stents: An Experimental pilot study. Uurto, Ilkka, et al. s.l. : J ENDOVASC THER, 2005, Vol. 12, pp. 371-379.
44. Factors influencing the anti-inflammatory effect of dexamethasone therapy in experimental pneumococcal meningitis. I. Lutsar, I. R. Friedland, H. S. Jafri, L. Wubbel, A. Ahmed, M. Trujillo, C. C. McCoig, G. H. McCracken Jr. s.l. : Journal of Antimicrobial Chemot, 2003, Vol. 52, pp. 651-655.
45. Neural regulation of innate immunity: a coordinated nonspecific host response to pathogens. Sternberg, Esther M. s.l. : Nature Reviews Immunology, April 2006, Vol. 6, pp. 318-328.
46. Differential Modulation of TLR3- and TLR4-Mediated Dendritic Cell Maturation and Function by Progesterone. Leigh A. Jones, Shrook Kreem, Muhannad Shweash, Andrew Paul, James Alexander, and Craig W. Roberts. s.l. : J Immunol 2010, 2010, Vol. 185, pp. 4525-4534.
*47.* Molecular mechanisms of glucocorticoid action: what is important? Newton, Robert, s.l. : Thorax, 2000, Vol. 55, pp. 603-613 .
48. Local Drug Delivery System: Inhibition of Inflammatory Angiogenesis in a Murine Sponge Model by Dexamethasone-Loaded Polyurethane Implants. Moura SA, Lima LD, Andrade SP, Da Silva-Cunha A Jr, Órefice RL, Ayres E, Da Silva GR. s.l. : J Pharm Sci., 2011, Vol. 100(7), pp. 2886-95.
49. Glucocorticoid Receptor Signaling. [Online] http://www.sabiosciences.com/pathway.php?sn=Glucocorticoid_Receptor_Signaling.
50. Sonya M. Abraham, Toby Lawrence, Anna Kleiman, Paul Warden, Mino Medghalchi, Jan Tuckermann, Jeremy Saklatvala, and Andrew R. Clark. 1, Antiinflammatory effects of dexamethasone are partly dependent on induction of dual specificity phosphatase. s.l. : JEM, 2006, Vol. 203 No. 8, pp. 1883-1889 .
*51.* Nicotinic Modulation of Innate Immune Pathways Via alpha 7 Nicotinic Acetylcholine Receptor. Li, Wen-Yan Cui & Ming D. s.l. : J Neuroimmune Pharmacol, 2010, Vol. 5, pp. 479-488.
*52.* Stimulation of the vagus nerve attenuates macrophage activation by activating the Jak2- STAT3 signaling pathway. s.l. : Nature Immunology, 2005, Vol. 6, pp. 844 - 851.
53. Nicotinic acetylcholine receptor α7 subunit is an essential regulator of inflammation. Hong Wang, Man Yu, Mahendar Ochani, Carol Ann Amella, Mahira Tanovic, Seenu Susarla, Jian Hua Li, Haichao Wang, Huan Yang, Luis Ulloa, Yousef Al-Abed, Christopher J. Czura & Kevin J. Tracey. s.l. : Nature, 2003, Vol. 421, pp. 384-388.
54. Inflammation: A nervous connection. Libert, Claude, s.l. : Nature, 2003, Vol. 421, pp. 328-329.
55. Reflex control of immunity. Tracey, Kevin J. s.l. : Nature Reviews Immunology, 2009, Vol. 9, pp. 418-428.
56. Are statins anti-inflammatory? Ridker, Gavin J Blake and Paul M. s.l. : Curr Control Trials Cardiovasc Med, 2000, Vol. 1, pp. 161-165.
*57.* Acute Antiinflammatory Properties of Statins Involve Peroxisome Proliferator-Activated Receptor-avia Inhibition of the Protein Kinase C Signaling Pathway. Réjane Paumelle, Christophe Blanquart, Olivier Briand, Olivier Barbier, Christian Duhem, Gaëtane Woerly, Frédéric Percevault, Jean-Charles Fruchart, David Dombrowicz, Corine Glineur, Bart Staels. s.l. : Circulation Research., 2006, Vol. 98, pp. 361-369.
58. The role of simvastatin in the osteogenesis of injectable tissue-engineered bone based on human adipose-derived stromal cells and platelet-rich plasma. Yongsheng Zhou, Yongwei Ni, Yunsong Liu, Baijin Zeng, Yongwei Xu, Wenshu Ge. s.l. : Biomaterials, 2010, Vol. 31, pp. 5325-5335.
59. The selected pathophysiological aspects of PPARs activation. Kieć-Wilk B, Dembińska-KiećA, Olszanecka A, Bodzioch M, Kawecka-Jaszcz K. s.l. : J Physiol Pharmacol. , 2005, Vol. 56/2, pp. 149-162.
60. Topographical control of human macrophages by a regularly microstructured polyvinylidene fluoride surface. Nora E. Paul Claudia Skazik, Marc Harwardt, Matthias Bartneck, Bernd Denecke, Doris Klee, Jochen Salber and Gabriele Zwadlo-Klarwasser. s.l. : Biomaterials, October 2008, Vol. 29:30, pp. 4056-4064.
61. Antimicrobial peptides. Izadpanah A, Gallo RL. s.l. : J Am Acad Dermatol., 2005, Vol. 52, pp. 381-90.
*62.* Spotlight on Human LL-37, an Immunomodulatory Peptide with Promising Cell-Penetrating Properties. Michèle Seil, Carole Nagant, Jean-Paul Dehaye, Michel Vandenbranden and Marc. s.l. :Pharmaceuticals, 2010, Vol. 3, pp. 3435-3460.
63. Antimicrobial functions of the human cathelicidin hCAP18. Alexandra, Simona. s.l. : A Journal of Clinical Medicine, 2009, Vol. 4.
64. Host Defense Peptide LL-37 Selectively Reduces Proinflammatory Macrophage Responses. Kelly L. Brown, Grace F. T. Poon, Darlene Birkenhead, Olga M. Pena,Reza Falsafi, Claes Dahlgren, Anna Karlsson, Johan Bylund, Robert E. W. Hancock, and Pauline Johnson. s.l. : The Journal of Immunology, 2011, Vol. 186, pp. 5497-5505.
65. Augmentation of the Lipopolysaccharide-Neutralizing Activities of Human Cathelicidin CAP18/LL-37-Derived Antimicrobial Peptides by Replacement with Hydrophobic and Cationic Amino Acid Residues. Isao Nagaoka, Satoko Hirota, François Niyonsaba, Michimasa Hirata, Yoshiyuki Adachi, Hiroshi Tamura, Shigenori Tanaka, and Didier Heumann. s.l. : Clin Diagn Lab Immunol. 2002 September; 9(5): ., 2002, Vol. 9(5), pp. 972-982.
66. Human Apolipoprotein E. Stanley C. Rall, Jr.,K arl H. Weisgraber, and RoberWt . Mahley. 8, 1981, Vols. 257, No. 8, Ieaue of April 25, pp. 41714178, 1982.
*67.* Apolipoprotein E Induces Antiinflammatory Phenotype in Macrophages. Daniel Baitsch, Hans H. Bock, Thomas Engel, Ralph Telgmann, Carsten Müller-Tidow, Georg Varga, Martine Bot, Joachim Herz, Horst Robenek, Arnold von Eckardstein, Jerzy-Roch Nofer. s.l. : Arterioscler Thromb Vasc Biol, 2011, Vol. 31.
68. Apolipoprotein E and Peptide Mimetics Modulate Inflammation by Binding the SET Protein and Activating Protein Phosphatase 2A. Dale J. Christensen, Nobutaka Ohkubo, Jessica Oddo, Michael J. Van Kanegan, Jessica Neil, Fengqiao Li, Carol A. Colton and Michael P. Vitek. s.l. : J Immunol, 2011, Vol. 186, pp. 2535-2542.
69. Understanding the Association of Apolipoprotein E4 with Alzheimer Disease: Clues from Its Structure. Weisgraber, Ning Zhong and Karl H. 10, s.l. : Journal of Biological Chemistry, 2009, Vol.
*70.* Functionalized silk-based biomaterials for bone formation. Sofia S, McCarthy MB, Gronowicz G, Kaplan DL. s.l. : J Biomed Mater Res, 2001, Vol. 54, pp. 139-148.

## Claims

1. A composition comprising a functionalized silk fibroin biomaterial comprising at least one immune cell-modulating agent encapsulated therein or present on a surface thereof in an effective amount sufficient to selectively alter an activation state of at least one type of immune cells upon contact with said at least one immune cell-modulating agent,
wherein said at least one immune cell-modulating agent is selected to modulate immune cell response to a target stimulus in a subject;
wherein the target stimulus is at least one of tissue regeneration, tissue reconstruction, tissue repair, wound healing or tissue augmentation; and
wherein the silk fibroin biomaterial is in a format appropriate for administration by insertion of a needle, cannula and/or tubing.

2. The composition of claim 1, wherein the silk fibroin biomaterial induces a regenerative or anti-inflammatory response.

3. The composition of claim 1, wherein the regenerative or anti-inflammatory response comprises facilitating tissue repair and/or regeneration at a target site; and/or the regenerative or anti-inflammatory response comprises reducing rejection of the silk fibroin biomaterial by a host's immune system.

4. The composition of claim 1, wherein the silk fibroin biomaterial comprises porous structures.

5. The composition of claim 4, wherein the porous silk fibroin biomaterial is adapted to have a pore size of 1 nm to 1000 um, 50 nm to 900 µm, 100 nm to 800 µm, 500 nm to 700 µm, 1 µm to 600 µm, 10 µm to 500 µm, 50 µm to 400 µm, 75 µm to 250 µm or greater than 100 µm.

6. The composition of claim 4, wherein the porous silk fibroin biomaterial has a porosity of at least 1%, at least 3%, at least 5%, at least 10%, at least 15%, at least 20%, at least 25%, at least 30%, at least 40%, at least 50%, at least 60%, at least 70%, at least 80%, at least 90% or higher.

7. The composition of claim 1, wherein the silk fibroin biomaterial is a particle.

8. The composition of claim 7, wherein the particle is of a size too large to be phagocytosed by macrophages.

9. The compostion of claim 7, wherein the particle has a diameter of at least 5 µm - 7 µm.

10. The composition of claim 1 for use in cosmetic surgery procedures selected from the group consisting of breast enhancement, mastopexy, face lift, forehead lift, upper and lower eyelid surgery (blepharoplasty), nose reshaping (rhinoplasty), nasal reconstruction, dermal tissue augmentation, filling of lines, folds, wrinkles, minor facial depressions, and cleft lips, correction of minor deformities due to aging or disease, including in the hands and feet, fingers and toes; augmentation of the vocal cords or glottis to rehabilitate speech; dermal filling of sleep lines and expression lines; replacement of dermal and subcutaneous tissue lost due to aging; lip augmentation; filling of crow's feet and the orbital groove around the eye; chin augmentation; augmentation of the cheek and/or nose; filling of indentations in the soft tissue, dermal or subcutaneous, due to, e.g., overzealous liposuction or other trauma; filling of acne or traumatic scars; filling of nasolabial lines, nasoglabellar lines and intraoral lines, and any combinations thereof.

11. The composition of claim 1, wherein:
(a) said at least one type of the immune cells is selected from the group consisting of monocytes, macrophages, dendritic cells, megakaryocytes, granulocytes, T cells, B cells, natural killer (NK) cells, and any combinations thereof; and/or
(b) said at least one type of the immune cells comprises macrophages; and/or (c) said at least one type of the immune cells comprises dendritic cells; and/or
(d) the silk fibroin biomaterial is adapted for a sustained release of said at least one immune cell-modulating agent; and/or
(e) the silk fibroin biomaterial comprises at least two immune cell-modulating agents, optionally wherein the silk fibroin biomaterial is adapted to release a first immune cell-modulating agent and a second immune cell-modulating agent at different time points, for example wherein the silk fibroin biomaterial comprises the first immune cell-modulating agent in a first layer and the second immune cell-modulating agent in a second layer; and/or
(f) said at least one immune cell-modulating agent comprises a macrophage-skewing agent.

12. The composition of claim 1, wherein the at least one immune cell-modulating agent is a macrophage-skewing agent; and
the macrophage-skewing agent is released at a pre-determined rate from the silk fibroin biomaterial, upon the placement at the target site, to alter the ratio of M1 macrophages to M2 macrophages surrounding the silk fibroin biomaterial, wherein when the ratio of M1 macrophages to M2 macrophages is above a threshold, the silk fibroin biomaterial induces an inflammatory response; and when the ratio of M1 macrophages to M2 macrophages is below a threshold, the silk fibroin biomaterial induces a regenerative or anti-inflammatory response.

13. The composition of claim 1 for use in a method for controlling macrophage response to a target stimulus in a subject comprising:
placing said composition in close proximity to or at the site of a target stimulus in an effective amount sufficient to
(a) switch at least a population of macrophages to M1 phenotypic state, thereby inducing an inflammatory response to the stimulus;
(b) switch at least a population of macrophages to M2 phenotypic state, thereby inducing a regenerative response to the stimulus; or
(c) both (a) and (b), optionally wherein:
(i) the silk fibroin biomaterial is adapted for a sustained release of said at least one macrophage-skewing agent; and/or
(ii) the silk fibroin biomaterial comprises at least two macrophage-skewing agents, optionally wherein the silk fibroin biomaterial is adapted to release a first macrophage-skewing agent and a second macrophage-skewing agent at different time points, for example wherein the silk fibroin biomaterial comprises the first macrophage-skewing agent in a first layer and the second macrophage-skewing agent in a second layer.

14. The composition of claim 11 or 12, wherein said at least one macrophage-skewing agent is selected from the group consisting of: glucocorticoid (*e.g.,* dexamethasone); nicotine; statins (*e.g.,* simvastatin); an antimicrobial peptide (*e.g.,* LL-37 peptide, apolipoprotein E); LPS; INF-γ; TNF-α; prolactin; Notch activators (*e.g.,* delta or jagged ligands); IL-4; IL-13; IL-10; insulin sensitizer and PPAR-γ inducer (*e.g.,* rosiglitazone or other thiazolidinediones); HDAC inhibitors (*e.g.,* VPA); Notch signaling inhibitors (*e.g.,* GSI or DAPT); JAK inhibitors (*e.g.*, AG490); inhibitors of 3-hydroxy-3-methylglutaryl-coenzyme A (HMG-CoA) reductase; LiCl; thymosin; PLA2, and any combinations thereof.

15. The composition of any of the preceding claims, wherein:
(a) the composition or silk fibroin biomaterial is adapted for use in reconstructive surgery, for example wherein the reconstructive surgery further comprises cosmetic surgery; and/or
(b) the composition or silk fibroin biomaterial is adapted for use in soft tissue repair and/or augmentation, for example wherein the soft tissue is selected from the group consisting of a skin, a breast tissue, tendon, a ligament, a fibrous tissue, a connective tissue, a muscle, and any combinations thereof; and/or (c) the composition or silk fibroin biomaterial is adapted for use as a wound dressing; and/or
(d) the silk fibroin biomaterial is in a form of a film, a fiber, a collection of particles, a tube, a mat, a gel, a mesh, a fabric, or any combinations thereof; and/or (e) said at least one immune cell-modulating agent or said at least one macrophage-skewing agent is encapsulated into the silk fibroin biomaterial; and/or
(f) said at least one immune cell-modulating agent or said at least one macrophage-skewing agent is present on a surface of the silk fibroin biomaterial.

16. A process for producing a biomedical material comprising coating a first material with a composition as defined in claim 1, optionally wherein the biomaterial comprises a silk fibroin-based matrix.

## Patentansprüche

1. Eine Zusammensetzung, umfassend ein funktionalisiertes Seidenfibroin-Biomaterial, das zumindest einen Immunzellen modulierenden Wirkstoff umfasst, der in einer ausreichenden wirksamen Menge in dem Seidenfibroin-Biomaterial umkapselt ist oder an einer Oberfläche von diesem vorliegt, um einen Aktivierungszustand zumindest eines Typs von Immunzellen bei Kontakt mit dem zumindest einen Immunzellen modulierenden Wirkstoff selektiv zu ändern,
wobei der zumindest eine Immunzellen modulierende Wirkstoff ausgewählt ist um eine Immunzellenreaktion auf einen Zielreiz in einem Proband zu modulieren;
wobei der Zielreiz zumindest einer aus Geweberegeneration, Geweberekonstruktion, Gewebereparatur, Wundheilung oder Gewebeaugmentation ist; und
wobei das Seidenfibroin-Biomaterial in einer Form vorliegt, die zur Verabreichung durch Einführung einer Nadel, Kanüle und/oder Rohr geeignet ist.

2. Die Zusammensetzung von Anspruch 1, wobei das Seidenfibroin-Biomaterial eine regenerative oder entzündungshemmende Reaktion hervorruft.

3. Die Zusammensetzung von Anspruch 1, wobei die regenerative oder entzündungshemmende Reaktion die Förderung von Gewebereparatur und/oder Regeneration an einem Wirkort umfasst; und/oder wobei die regenerative oder entzündungshemmende Reaktion die Reduzierung von Abstoßung des Seidenfibroin-Biomaterials durch das Immunsystem eines Wirts umfasst.

4. Die Zusammensetzung von Anspruch 1, wobei das Seidenfibroin-Biomaterial poröse Strukturen umfasst.

5. Die Zusammensetzung von Anspruch 4, wobei das poröse Seidenfibroin-Biomaterial angepasst ist, eine Porengröße von 1 nm bis 1000 µm, 50 nm bis 900 µm, 100 nm bis 800 µm, 500 nm bis 700 µm, 1 µm bis 600 µm, 10 µm bis 500 µm, 50 µm bis 400 µm, 75 µm bis 250 µm oder größer als 100 µm aufzuweisen.

6. Die Zusammensetzung von Anspruch 4, wobei das poröse Seidenfibroin-Biomaterial eine Porosität von zumindest 1%, zumindest 3%, zumindest 5%, zumindest 10%, zumindest 15%, zumindest 20%, zumindest 25%, zumindest 30%, zumindest 40%, zumindest 50%, zumindest 60%, zumindest 70%, zumindest 80%, zumindest 90% oder höher aufweist.

7. Die Zusammensetzung von Anspruch 1, wobei das Seidenfibroin-Biomaterial ein Partikel ist.

8. Die Zusammensetzung von Anspruch 7, wobei der Partikel eine Größe aufweist, die zu groß ist um durch Makrophagen phagozytiert zu werden.

9. Die Zusammensetzung von Anspruch 7, wobei der Partikel einen Durchmesser von mindestens 5 µm - 7 µm aufweist.

10. Die Zusammensetzung von Anspruch 1 zur Verwendung in Verfahren der kosmetischen Chirurgie, ausgewählt aus der Gruppe bestehend aus Brustvergrößerung, Mastopexie, Gesichtsstraffung, Stirnstraffung, Ober- und Unterlidchirurgie (Blepharoplastik), Nasenkorrektur (Rhinoplastik), Nasenrekonstruktion, Hautgewebeaugmentation, Auffüllung von Linien, Falten, Unebenheiten, kleinen Gesichtsvertiefungen, und Lippenspalten, Korrektur von kleinen Deformitäten aufgrund von Alterung oder Krankheit, einschließlich in den Händen und Füßen, Fingern und Zehen; Augmentation der Stimmbänder oder Stimmritze um die Sprache wiederherzustellen; Hautauffüllung von Schlaffalten und Ausdrucksfalten; Erneuerung von aufgrund von Alterung verlorenem Hautgewebe und Unterhautgewebe; Lippenvergrößerung; Auffüllung von Krähenfüßen und der orbitalen Furche um das Auge; Kinnvergrößerung; Vergrößerung der Wange und/oder Nase; Auffüllung von Vertiefungen im Weichgewebe, dermal oder subkutan, aufgrund von z.B. übertriebener Fettabsaugung oder anderem Trauma; Auffüllung von Aknenarben oder traumatischen Narben; Auffüllung von Nasolabialfalten, Glabellafalten und intraoralen Falten, und beliebigen Kombinationen daraus.

11. Die Zusammensetzung von Anspruch 1, wobei:
(a) der zumindest eine Typ von Immunzellen aus der Gruppe ausgewählt ist, die aus Monozyten, Makrophagen, dendritischen Zellen, Magakaryozyten, Granulozyten, T-Zellen, B-Zellen, natürlichen Killerzellen (NK Zellen) und beliebigen Kombinationen dieser besteht; und/oder
(b) der zumindest eine Typ von Immunzellen Makrophagen umfasst; und/oder (c) der zumindest eine Typ von Immunzellen dendritische Zellen umfasst; und/oder
(d) das Seidenfibroin-Biomaterial angepasst ist zur anhaltenden Freisetzung des zumindest einen Immunzellen modulierenden Wirkstoffs; und/oder
(e) das Seidenfibroin-Biomaterial zumindest zwei Immunzellen modulierende Wirkstoffe umfasst, wobei das Seidenfibroin-Biomaterial optional angepasst ist, einen ersten Immunzellen modulierenden Wirkstoff und einen zweiten Immunzellen modulierenden Wirkstoff zu unterschiedlichen Zeitpunkten freizusetzen, wobei beispielsweise das Seidenfibroin-Biomaterial den ersten Immunzellen modulierenden Wirkstoff in einer ersten Schicht und den zweiten Immunzellen modulierenden Wirkstoff in einer zweiten Schicht umfasst; und/oder
(f) der zumindest eine Immunzellen modulierende Wirkstoff einen Makrophagenpolarisationsumkehrenden Wirkstoff umfasst.

12. Die Zusammensetzung von Anspruch 1, wobei der zumindest eine Immunzellen modulierende Wirkstoff ein Makrophagenpolarisationsumkehrender Wirkstoff ist; und der Makrophagenpolarisationsumkehrende Wirkstoff bei der Platzierung an dem Zielort in einem vorbestimmten Tempo aus dem Seidenfibroin-Biomaterial freigesetzt wird, um das Verhältnis von das Seidenfibroin-Biomaterial umgebenden M1 Makrophagen zu M2 Makrophagen zu ändern, wobei das Seidenfibroin-Biomaterial eine entzündliche Reaktion hervorruft, wenn das Verhältnis von M1 Makrophagen zu M2 Makrophagen oberhalb eines Grenzwertes ist; und eine regenerative oder entzündungshemmende Reaktion hervorruft, wenn das Verhältnis von M1 Makrophagen zu M2 Makrophagen unterhalb eines Grenzwertes ist.

13. Die Zusammensetzung von Anspruch 1 zur Verwendung in einem Verfahren zum Kontrollieren der Makrophagenreaktion auf einen Zielreiz in einem Probanden, umfassend:
Platzieren der Zusammensetzung in unmittelbarer Nähe zu oder an dem Ort eines Zielreizes in einer ausreichenden wirksamen Menge um
(a) zumindest eine Population von Makrophagen in den phänotypischen Zustand M1 zu überführen, wobei eine entzündliche Reaktion auf den Reiz hervorgerufen wird;
(b) zumindest eine Population von Makrophagen in den phänotypischen Zustand M2 zu überführen, wobei eine regenerative Reaktion auf den Reiz hervorgerufen wird; oder
(c) sowohl (a) als auch (b), wobei optional:
(i) das Seidenfibroin-Biomaterial an eine anhaltende Freisetzung des zumindest einen Makrophagenpolarisationsumkehrenden Wirkstoffs angepasst ist; und/oder
(ii) das Seidenfibroin-Biomaterial zumindest zwei Makrophagenpolarisationsumkehrende Wirkstoffe umfasst, wobei das Seidenfibroin-Biomaterial optional angepasst ist, einen ersten Makrophagenpolarisationsumkehrenden Wirkstoff und einen zweiten Makrophagenpolarisationsumkehrenden Wirkstoff zu unterschiedlichen Zeitpunkten freizusetzen, wobei zum Beispiel das Seidenfibroin-Biomaterial den ersten Makrophagenpolarisationsumkehrenden Wirkstoff in einer ersten Schicht und den zweiten Makrophagenpolarisationsumkehrenden Wirkstoff in einer zweiten Schicht umfasst.

14. Die Zusammensetzung von Anspruch 11 oder 12, wobei der zumindest eine Makrophagenpolarisationsumkehrende Wirkstoff ausgewählt ist aus der Gruppe bestehend aus: Glucocorticoid (z.B. Dexamethason); Nikotin; Statine (z.B. Simvastatin); ein antimikrobielles Peptid (z.B. LL-37 Peptid, Apolipoprotein E); LPS; INF-γ; TNF-α; Prolaktin; Notch Aktivatoren (z.B. Delta oder Jagged Liganden); IL-4; IL-13; IL-10; Insulin-Sensitizer und PPAR-γ Induktoren (z.B. Rosiglitazon oder andere Thiazolidindione); HDAC Inhibitoren (z.B. VPA); Notch Signal-Inhibitoren (z.B. GSI oder DAPT); JAK Inhibitoren (z.B. AG490); Inhibitoren von 3-Hydroxy-3-methylglutaryl-coenzym A (HMG-CoA) Reduktase; LiCI; Thymosin; PLA2, und beliebigen Kombinationen dieser.

15. Die Zusammensetzung nach einem der voranstehenden Ansprüche, wobei:
(a) die Zusammensetzung oder das Seidenfibroin-Biomaterial zur Verwendung in der rekonstruktiven Chirurgie angepasst ist, wobei die rekonstruktive Chirurgie zum Beispiel weiterhin kosmetische Chirurgie umfasst; und/oder
(b) die Zusammensetzung oder das Seidenfibroin-Biomaterial zur Verwendung in der Weichgewebereparatur und/oder-augmentation angepasst ist, wobei zum Beispiel das Weichgewebe ausgewählt ist aus der Gruppe, die aus Haut, Brustgewebe, Sehne, einem Band, Fasergewebe, Bindegewebe, Muskel und beliebigen Kombinationen dieser besteht; und oder
(c) die Zusammensetzung oder das Seidenfibroin-Biomaterial zur Verwendung als eine Wundauflage angepasst ist; und/oder
(d) das Seidenfibroin-Biomaterial in einer Form eines Films, einer Faser, einer Partikelansammlung, eines Rohrs, einer Matte, eines Gels, eines Netzes, eines Gewebes oder beliebiger Kombinationen dieser ist; und/oder (e) der zumindest eine Immunzellen modulierende Wirkstoff oder der zumindest eine Makrophagenpolarisationsumkehrende Wirkstoff in dem Seidenfibroin-Biomaterial eingekapselt ist; und/oder
(f) der zumindest eine Immunzellen modulierende Wirkstoff oder der zumindest eine Makrophagenpolarisationsumkehrenden Wirkstoff auf einer Oberfläche des Seidenfibroin-Biomaterials vorhanden ist.

16. Ein Verfahren zum Herstellen eines biomedizinischen Materials umfassend das Beschichten eines ersten Materials mit einer Zusammensetzung wie in Anspruch 1 definiert, wobei das Biomaterial optional eine Seidenfibroin-basierte Matrix aufweist.

## Revendications

1. Composition comprenant un biomatériau de fibroïne de soie fonctionnalisé, comprenant au moins un agent modulant les cellules immunitaires encapsulé à l'intérieur ou présent à la surface de celui-ci en une quantité efficace suffisante pour modifier sélectivement un état d'activation d'au moins un type de cellules immunitaires lors du contact avec ledit au moins un agent modulant les cellules immunitaires,
dans laquelle ledit au moins un agent modulant les cellules immunitaires est choisi pour moduler une réponse des cellules immunitaires à un stimulus cible chez un sujet ;
dans laquelle le stimulus cible est au moins un stimulus parmi la régénération des tissus, la reconstruction des tissus, la réparation des tissus, la cicatrisation des plaies ou l'augmentation des tissus ; et
dans laquelle le biomatériau de fibroïne de soie se présente sous un format adéquat pour l'administration par insertion d'une aiguille, d'une canule et/ou d'un tuyau.

2. Composition selon la revendication 1, dans laquelle le biomatériau de fibroïne de soie induit une réponse régénérative ou anti-inflammatoire.

3. Composition selon la revendication 1, dans laquelle la réponse régénérative ou anti-inflammatoire comprend la facilitation de la réparation et/ou de la régénération des tissus sur un site cible ; et/ou la réponse régénérative ou anti-inflammatoire comprend la réduction du rejet du biomatériau de fibroïne de soie par le système immunitaire d'un hôte.

4. Composition selon la revendication 1, dans laquelle le biomatériau de fibroïne de soie comprend des structures poreuses.

5. Composition selon la revendication 4, dans laquelle le biomatériau de fibroïne de soie poreuse est adapté pour avoir une taille de pores de 1 nm à 1000 µm, de 50 nm à 900 µm, de 100 nm à 800 µm, de 500 nm à 700 µm, de 1 µm à 600 µm, de 10 µm à 500 µm, de 50 µm à 400 µm, de 75 µm à 250 µm ou de plus de 100 µm.

6. Composition selon la revendication 4, dans laquelle le biomatériau de fibroïne de soie poreuse a une porosité d'au moins 1 %, d'au moins 3 %, d'au moins 5 %, d'au moins 10 %, d'au moins 15 %, d'au moins 20 %, d'au moins 25 %, d'au moins 30 %, d'au moins 40 %, d'au moins 50 %, d'au moins 60 %, d'au moins 70 %, d'au moins 80 %, d'au moins 90 % ou plus.

7. Composition selon la revendication 1, dans laquelle le biomatériau de fibroïne de soie est une particule.

8. Composition selon la revendication 7, dans laquelle la particule est d'une taille trop importante pour être phagocytée par les macrophages.

9. Composition selon la revendication 7, dans laquelle la particule a un diamètre d'au moins 5 µm à 7 µm.

10. Composition selon la revendication 1, pour utilisation dans des interventions de chirurgie esthétique choisies dans le groupe consistant en l'augmentation mammaire, la mastopexie, le lifting du visage, le lifting du front, la chirurgie des paupières inférieures et supérieures (blépharoplastie), le remodelage du nez (rhinoplastie), la reconstruction du nez, l'augmentation du tissu dermique, le comblement des rides, des plis, des ridules, des dépressions faciales mineures, et des fentes labiales, la correction des déformations mineures dues au vieillissement ou à la maladie, y compris sur les mains et les pieds, les doigts de main et de pied ; l'augmentation des cordes vocales ou de la glotte pour une rééducation au langage ; le comblement dermique des rides de fatigue et des rides d'expression ; le remplacement du tissu dermique et sous-cutané perdu à cause du vieillissement ; l'augmentation des lèvres ; le comblement des pattes d'oies et de la cavité orbitale autour des yeux ; l'augmentation du menton ; l'augmentation des joues et/ou du nez ; le comblement des cavités dans les tissus mous, dermiques ou sous-cutanés, dues par exemple à une liposuccion excessive ou autre trauma ; le comblement des cicatrices d'acné ou traumatiques ; le comblement des plis nasolabiaux, des plis nasoglabellaires et des rides intrabuccales, et toutes combinaisons de ceux-ci.

11. Composition selon la revendication 1, dans laquelle :
(a) ledit au moins un type de cellules immunitaires est choisi dans le groupe consistant en monocytes, macrophages, cellules dendritiques, mégacaryocytes, granulocytes, lymphocytes T, lymphocytes B, cellules tueuses naturelles (NK) et toutes combinaisons de ceux-ci ; et/ou
(b) ledit au moins un type de cellules immunitaires comprend les macrophages ; et/ou
(c) ledit au moins un type de cellules immunitaires comprend les cellules dendritiques ; et/ou
(d) le biomatériau de fibroïne de soie est adapté à une libération prolongée d'au moins un agent modulant les cellules immunitaires ; et/ou
(e) le biomatériau de fibroïne de soie comprend au moins deux agents modulant les cellules immunitaires, où le biomatériau de fibroïne de soie est éventuellement adapté à libérer un premier agent modulant les cellules immunitaires et un second agent modulant les cellules immunitaires à des moments différents, par exemple où le biomatériau de fibroïne de soie comprenant le premier agent modulant les cellules immunitaires dans une première couche et le second agent modulant les cellules immunitaires dans une seconde couche ; et/ou
(f) ledit au moins un agent modulant les cellules immunitaires comprend un agent polarisant les macrophages.

12. Composition selon la revendication 1, dans laquelle ledit au moins un agent modulant les cellules immunitaires est un agent polarisant les macrophages ; et
l'agent polarisant les macrophages est libéré à un taux prédéterminé à partir du biomatériau de fibroïne de soie lors du placement sur le site cible, afin de modifier le rapport entre les macrophages M1 et les macrophages M2 aux environs du biomatériau de fibroïne de soie, où, quand le rapport entre les macrophages M1 et les macrophages M2 est supérieur à un seuil, le biomatériau de fibroïne de soie induit une réponse inflammatoire ; et quand le rapport entre les macrophages M1 et les macrophages M2 est inférieur à un seuil, le biomatériau de fibroïne de soie induit une réponse régénérative ou anti-inflammatoire.

13. Composition selon la revendication 1, pour utilisation dans une méthode permettant de contrôler la réponse des macrophages à un stimulus cible chez un sujet, comprenant les étapes consistant à :
placer ladite composition très près ou sur le site d'un stimulus cible en une quantité efficace suffisante pour
(a) faire passer au moins une population de macrophages au stade phénotypique M1, ce qui permet d'induire une réponse inflammatoire au stimulus ;
(b) faire passer au moins une population de macrophages au stade phénotypique M2, ce qui permet d'induire une réponse régénérative au stimulus ;
(c) à la fois (a) et (b), où éventuellement
(i) le biomatériau de fibroïne de soie est adapté à une libération prolongée d'au moins deux agents polarisant les macrophages ; et/ou
(ii) le biomatériau de fibroïne de soie comprend au moins deux agents polarisant les macrophages, où le biomatériau de fibroïne de soie est éventuellement adapté à libérer un premier agent polarisant les macrophages et un second agent polarisant les macrophages à des moments différents, par exemple où le biomatériau de fibroïne de soie comprend le premier agent polarisant les macrophages dans une première couche et le second agent polarisant les macrophages dans une seconde couche.

14. Composition selon la revendication 11 ou 12, dans laquelle ledit au moins un agent polarisant les macrophages est choisi dans le groupe consistant en : glucocorticoïde (par exemple dexaméthasone) ; nicotine ; statines (par exemple simvastatine) ; un peptide antimicrobien (par exemple peptide LL-37, apolipoprotéine E) ; LPS ; INF-γ ; TNF-α ; prolactine ; activateurs de Notch (par exemple ligands delta ou jagged) ; IL-4 ; IL-13 ; IL-10 ; sensibilisant à l'insuline et inducteur PPAR-γ (par exemple rosiglitazone ou autres thiazolidinediones) ; inhibiteurs HDAC (par exemple VPA) ; inhibiteurs de signalisation de Nocth (par exemple GSI ou DAPT) ; inhibiteurs JAK (par exemple AG490) ; inhibiteurs de la 3-hydroxy-3-méthyl-glutaryl-coenzyme A (HMG-CoA) réductase ; LiCl ; thymosine ; PLA2 et toutes combinaisons de ceux-ci.

15. Composition selon l'une quelconque des revendications précédentes, dans laquelle :
(a) la composition ou le biomatériau de fibroïne de soie est adapté pour l'utilisation dans la chirurgie reconstructive, par exemple où la chirurgie reconstructive comprend en outre la chirurgie esthétique ; et/ou
(b) la composition ou le biomatériau de fibroïne de soie est adapté pour l'utilisation dans la réparation et/ou l'augmentation des tissus mous, par exemple où les tissus mous sont choisis dans le groupe consistant en une peau, un tissu mammaire, un tendon, un ligament, un tissu fibreux, un tissu conjonctif, un muscle et toutes combinaisons de ceux-ci ; et/ou
(c) la composition ou le biomatériau de fibroïne de soie est adapté pour l'utilisation comme pansement ; et/ou
(d) le biomatériau de fibroïne de soie se présente sous la forme d'un film, d'une fibre, d'un ensemble de particules, d'un tube, d'un mat, d'un gel, d'un maillage, d'un tissu ou de toutes combinaisons de ceux-ci ; et/ou
(e) ledit au moins un agent modulant les cellules immunitaires ou ledit au moins un agent polarisant les macrophages est encapsulé dans le biomatériau de fibroïne de soie ; et/ou
(f) ledit au moins un agent modulant les cellules immunitaires ou ledit au moins un agent polarisant les macrophages est présent sur une surface du biomatériau de fibroïne de soie.

16. Procédé de production d'un matériau biomédical comprenant le revêtement d'un premier matériau avec une composition selon la revendication 1, où éventuellement le biomatériau comprend une matrice à base de fibroïne de soie.
